(19) Europäisches Patentamt / European Patent Office / Office européen des brevets

(11) EP 3 880 672 B1

(12) **EUROPÄISCHE PATENTSCHRIFT**

(45) Veröffentlichungstag und Bekanntmachung des Hinweises auf die Patenterteilung:
**23.10.2024 Patentblatt 2024/43**

(21) Anmeldenummer: **19813240.9**

(22) Anmeldetag: **08.11.2019**

(51) Internationale Patentklassifikation (IPC):
***C07D 403/14*** *(2006.01)* ***H10K 50/11*** *(2023.01)*
***H10K 85/60*** *(2023.01)*

(52) Gemeinsame Patentklassifikation (CPC):
**C07D 403/14; H10K 85/40; H10K 85/654; H10K 85/6572;** C07D 209/86; C07D 401/14; H10K 50/11; H10K 2101/20; H10K 2101/90; Y02E 10/549; Y02P 70/50

(86) Internationale Anmeldenummer:
**PCT/EP2019/080654**

(87) Internationale Veröffentlichungsnummer:
**WO 2020/099259 (22.05.2020 Gazette 2020/21)**

(54) **CARBAZOLDERIVATE ZUR VERWENDUNG IN OPTOELEKTRONISCHEN VORRICHTUNGEN**

CARBAZOLE DERIVATIVES FOR USE IN OPTOELECTRONIC DEVICES

DERIVES DE CARBAZOLE DESTINÉES À L'UTILISATION DANS DES DISPOSITIFS OPTOÉLECTRONIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.11.2018 EP 18206790**
**28.12.2018 EP 18001020**
**28.12.2018 EP 18001022**

(43) Veröffentlichungstag der Anmeldung:
**22.09.2021 Patentblatt 2021/38**

(73) Patentinhaber: **Samsung Display Co., Ltd.**
**Gyeonggi-do 17113 (KR)**

(72) Erfinder:
- **DANZ, Michael**
  **76344 Eggenstein-Leopoldshafen (DE)**
- **THIRION, Damien**
  **76689 Karlsdorf-Neuthard (DE)**
- **DIGENNARO, Angela**
  **69120 Heidelberg (DE)**
- **SEIFERMANN, Stefan**
  **77815 Bühl (DE)**
- **JOLY, Damien**
  **67930 Beinheim (FR)**

(74) Vertreter: **Dr. Weitzel & Partner**
**Patent- und Rechtsanwälte mbB**
**Friedenstrasse 10**
**89522 Heidenheim (DE)**

(56) Entgegenhaltungen:
**EP-A1- 3 109 247**
**WO-A1-2013/100540**
**WO-A1-2013/165192**
**WO-A1-2015/142036**
**WO-A1-2016/013875**

- **PALOMA L DOS SANTOS: "Triazatruxene: A Rigid Central Donor Unit for a D-A3 Thermally Activated Delayed Fluorescence Material Exhibiting Sub-Microsecond Reverse Intersystem Crossing and Unity Quantum Yield via Multiple Singlet-Triplet State Pairs", ADVANCED SCIENCE, 16 April 2018 (2018-04-16), pages 1 - 9, XP093152628, Retrieved from the Internet <URL:https://onlinelibrary.wiley.com/doi/epdf/10.1002/advs.201700989>**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

## Beschreibung

Die Erfindung betrifft organische Moleküle und ihre Verwendung in organischen lichtemittierenden Dioden (OLEDs) und in anderen optoelektronischen Vorrichtungen.

Stand der Technik

Die WO 2013/100540 A1 bezieht sich auf eine Verbindung für eine organische optoelektronische Vorrichtung, dargestellt durch die chemische Formel 1:

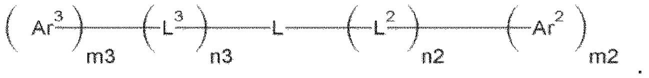

$Ar^3$, $L^3$, L, $L^2$; $Ar^2$, m3, m2, n3 und n2 sind so wie in der WO 2013/100540 A1 definiert. L wird durch die folgenden chemischen Formeln 2-4 dargestellt:

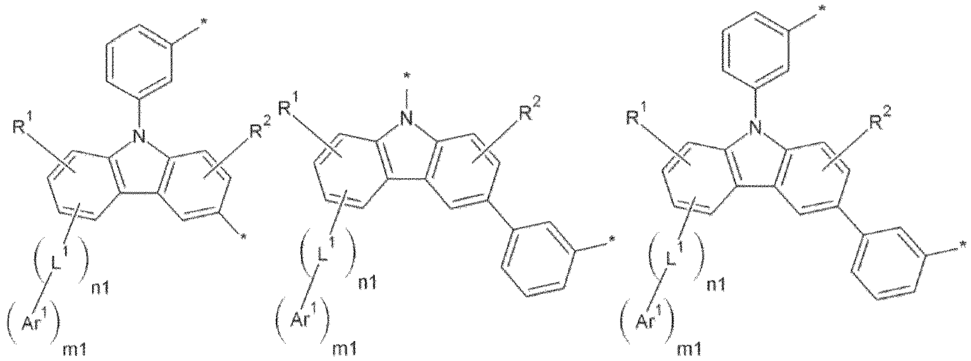

wobei $Ar^1$, $L^1$, $R^1$, $R^2$, n1, m1 und * so sind wie in der WO 2013/100540 A1 definiert.

Die EP 3 109 247 A1 bezieht sich auf eine kondensierte cyclische Verbindung und eine organische lichtemittierende Vorrichtung, die diese Verbindung enthält. Die cyclische Verbindung wird durch die Formel 1 repräsentiert:

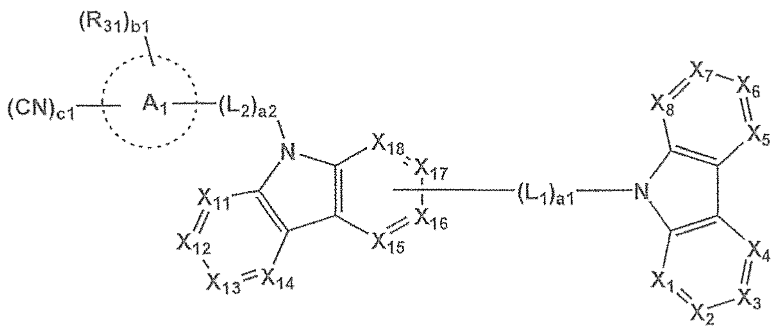

wobei $A_1$, $R_{31}$, $X_1$-$X_{18}$, $L^1$, $L_2$, a1, a2, b1, c1 so sind wie in der EP 3 109 247 A1 definiert.

Die WO 2016/013875 A1 offenbart eine organische Elektrolumineszenzvorrichtung, die eine Anode, eine Kathode und eine organische Schicht zwischen der Anode und der Kathode umfasst, wobei die organische Schicht eine oder mehrere lichtemittierende Schichten und eine oder mehrere Lochtransportschichten umfasst; mindestens eine lichtemittierende Schicht eine oder mehrere Dotierstoffverbindungen und zwei oder mehrere Wirtsverbindungen umfasst; eine erste Wirtsverbindung durch die folgende Formel 1 dargestellt ist; eine zweite Wirtsverbindung durch die folgende Formel 2 dargestellt ist; und mindestens eine Lochtransportschicht, welche die durch die folgende Formel 3 dargestellte Verbindung umfasst:

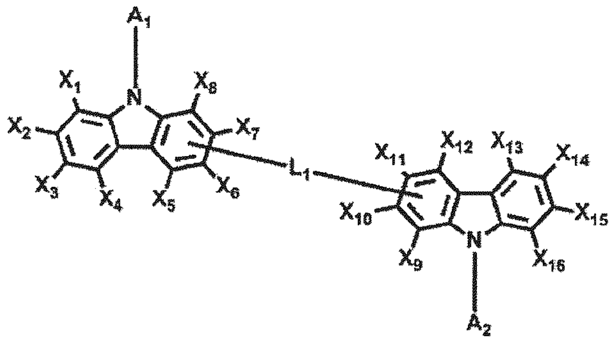

(1)

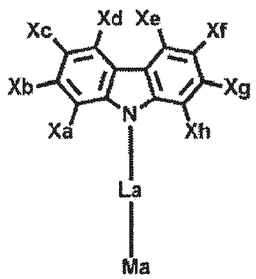

(2)

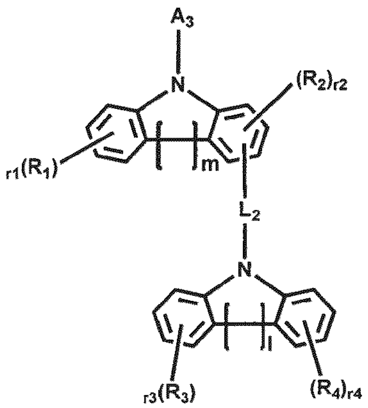

------------(3)

wobei die $A_1$-$A_3$, $X_1$-$X_{18}$, Xa-Xh, La, Ma, $L^1$, $L_2$, $R_1$-$R_4$, r1-r4, m und l wie in der WO 2016/013875 A1 definiert sind.

Die WO 2015/142036 A1 beschreibt ein Elektronenpuffermaterial, das eine Verbindung umfasst, die ein stickstoffhaltiges Heteroaryl enthält, das aus den durch die folgenden Formeln 1 bis 3 dargestellten Verbindungen ausgewählt ist:

$H-(Cz-L_1)_a-M$ ------------ (1)

$H-(Cz)_b-L_1-M$ ------------ (2)

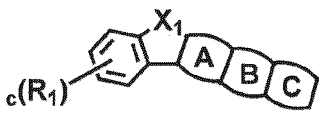

------------(3)

wobei Cz die folgende Struktur darstellt:

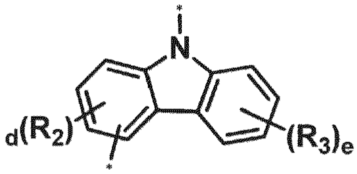

A stellt dar:

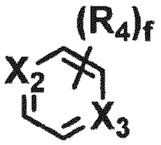

B stellt dar:

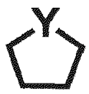

C stellt dar:

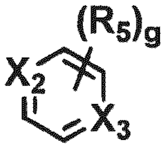

,

wobei $X_1$-$X_3$, $R_1$-$R_5$, a-g, $L^1$, Y und M wie in der WO 2015/142036 A1 definiert sind.

Die WO 2013/165192 A1 bezieht sich auf eine organische elektrolumineszente Verbindung, dargestellt durch die folgende Formel 1

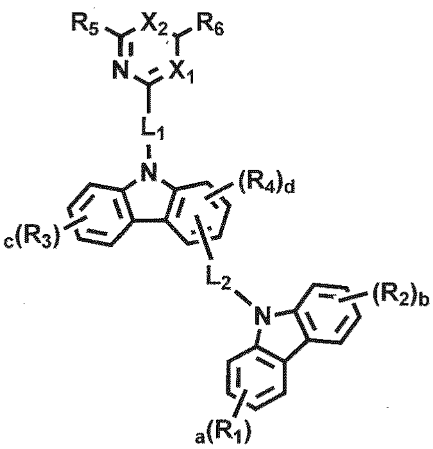

-------- (1) ,

wobei $L^1$, $L_2$, $X_1$, $X_2$, $R_1$-$R_6$, a-d wie in der WO 2013/165192 A1 definiert sind.

**Beschreibung**

Die Aufgabe der vorliegenden Erfindung besteht darin, Moleküle bereitzustellen, die zur Verwendung in optoelektronischen Vorrichtungen geeignet sind.

Diese Aufgabe wird durch die Erfindung gelöst, die eine neue Klasse von organischen Molekülen bereitstellt.

Erfindungsgemäß sind die organischen Moleküle rein organische Moleküle, d. h., sie enthalten im Gegensatz zu Metallkomplexen, die für ihre Verwendung in optoelektronischen Vorrichtungen bekannt sind, keine Metallionen.

Gemäß der vorliegenden Erfindung weisen die organischen Moleküle Emissionsmaxima im blauen, himmelblauen oder grünen Spektralbereich auf. Die organischen Moleküle weisen insbesondere Emissionsmaxima zwischen 420 nm und 520 nm auf, vorzugsweise zwischen 440 nm und 495 nm, weiter bevorzugt zwischen 450 nm und 470 nm. Die Photolumineszenzquantenausbeuten der erfindungsgemäßen organischen Moleküle betragen insbesondere 70 % und mehr. Die erfindungsgemäßen Moleküle weisen insbesondere eine thermisch aktivierte verzögerte Fluoreszenz (TADF) auf. Die Verwendung der erfindungsgemäßen Moleküle in einer optoelektronischen Vorrichtung, zum Beispiel eine organische lichtemittierende Diode (OLED), führt zu höheren Effizienzen der Vorrichtung. Entsprechende OLEDs weisen eine höhere Stabilität als OLEDs mit bekannten Emittermaterialien und vergleichbarer Farbe auf.

Das erfindungsgemäße organische Molekül ist ausgewählt aus der Gruppe bestehend aus Formel IIa-1, Formel IIa-2 und Formel IIa-3:

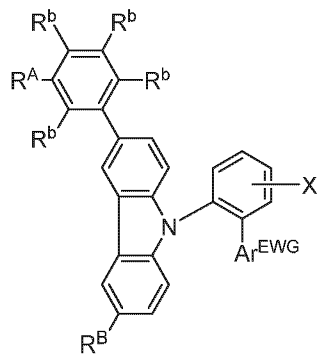
Formel IIa-1

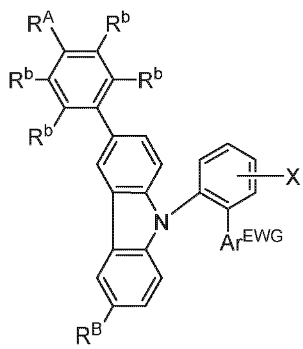
Formel IIa-2

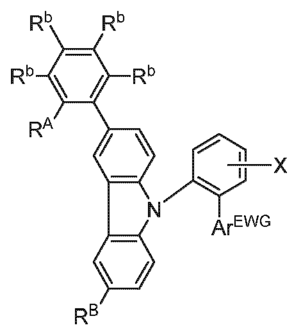
Formel IIa-3

wobei

X ausgewählt ist aus der Gruppe bestehend aus H, $SiMe_3$, $SiPh_3$, CN und $CF_3$;
$Ar^{EWG}$ ausgewählt ist aus der Gruppe bestehend aus den Formeln IIa bis IIc und IIi

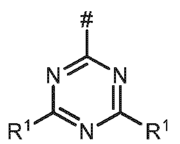
Formel IIa

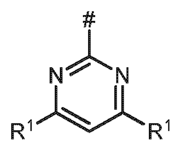
Formel IIb

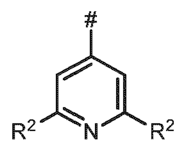
Formel IIc

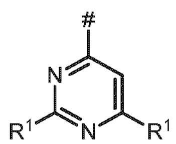
Formel IIi

wobei # die Bindungsstelle einer Einfachbindung darstellt, die $Ar^{EWG}$ mit dem Phenylring verbindet;
$R^1$ bei jedem Auftreten unabhängig voneinander ausgewählt ist aus der Gruppe bestehend aus Wasserstoff, Deuterium,
$C_1$-$C_5$-Alkyl,

wobei ein oder mehrere Wasserstoffatome optional substituiert sind durch Deuterium; $C_2$-$C_8$-Alkenyl, wobei ein oder mehrere Wasserstoffatome optional substituiert sind durch Deuterium; $C_2$-$C_8$-Alkinyl, wobei ein oder mehrere Wasserstoffatome optional substituiert sind durch Deuterium; und $C_6$-$C_{18}$-Aryl, das optional substituiert ist mit einem oder mehreren Substituenten $R^6$;

$R^2$ bei jedem Auftreten unabhängig voneinander ausgewählt ist aus der Gruppe bestehend aus Wasserstoff, Deuterium,

$C_1$-$C_5$-Alkyl,

wobei ein oder mehrere Wasserstoffatome optional substituiert sind durch Deuterium; $C_2$-$C_8$-Alkenyl, wobei ein oder mehrere Wasserstoffatome optional substituiert sind durch Deuterium; $C_2$-$C_8$-Alkinyl, wobei ein oder mehrere Wasserstoffatome optional substituiert sind durch Deuterium; und $C_6$-$C_{18}$-Aryl, das optional substituiert ist mit einem oder mehreren Substituenten $R^6$;

$R^d$ bei jedem Auftreten unabhängig voneinander ausgewählt ist aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I,
$C_1$-$C_{40}$-Alkyl,

das optional substituiert ist mit einem oder mehreren Substituenten $R^6$ und wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_1$-$C_{40}$-Alkoxy,

das optional substituiert ist mit einem oder mehreren Substituenten $R^6$ und wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_1$-$C_{40}$-Thioalkoxy,

das optional substituiert ist mit einem oder mehreren Substituenten $R^6$ und wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_2$-$C_{40}$-Alkenyl,

das optional substituiert ist mit einem oder mehreren Substituenten $R^6$ und wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_2$-$C_{40}$-Alkinyl,

das optional substituiert ist mit einem oder mehreren Substituenten $R^6$ und wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_6$-$C_{60}$-Aryl,

das optional substituiert ist mit einem oder mehreren Substituenten $R^6$; und $C_3$-$C_{57}$-Heteroaryl, das optional substituiert ist mit einem oder mehreren Substituenten $R^6$;

$R^6$ bei jedem Auftreten unabhängig voneinander ausgewählt ist aus der Gruppe bestehend aus Wasserstoff, Deuterium, OPh, $CF_3$, CN, F,
$C_1$-$C_5$-Alkyl,
wobei optional ein oder mehrere Wasserstoffatome unabhängig voneinander substituiert sind durch Deuterium, CN, $CF_3$ oder F;
$C_1$-$C_5$-Alkoxy,
wobei optional ein oder mehrere Wasserstoffatome unabhängig voneinander substituiert sind durch Deuterium, CN, $CF_3$ oder F;
$C_1$-$C_5$-Thioalkoxy,
wobei optional ein oder mehrere Wasserstoffatome unabhängig voneinander substituiert sind durch Deuterium, CN, $CF_3$ oder F;
$C_2$-$C_5$-Alkenyl,
wobei optional ein oder mehrere Wasserstoffatome unabhängig voneinander substituiert sind durch Deuterium, CN,

$CF_3$ oder F;
$C_2$-$C_5$-Alkinyl,
wobei optional ein oder mehrere Wasserstoffatome unabhängig voneinander substituiert sind durch Deuterium, CN, $CF_3$ oder F;
$C_6$-$C_{18}$-Aryl,
das optional substituiert ist mit einem oder mehrere $C_1$-$C_5$-Alkyl-Substituenten;
$C_3$-$C_{17}$-Heteroaryl,
das optional substituiert ist mit einem oder mehrere $C_1$-$C_5$-Alkyl-Substituenten;
N($C_6$-$C_{18}$-Aryl)$_2$;
N($C_3$-$C_{17}$-Heteroaryl)$_2$,
und N($C_3$-$C_{17}$-Heteroaryl)($C_6$-$C_{18}$-aryl);
$R^A$ bei jedem Auftreten, unabhängig voneinander ausgewählt ist aus einer chemischen Struktur der Formel IIA

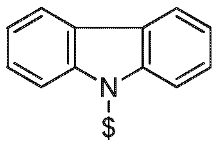

Formel IIA

oder einer chemischen Struktur der Formel IIB

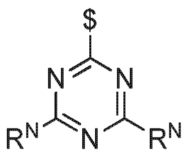

Formel IIB,

wobei $R^N$ ausgewählt ist aus der Gruppe bestehend aus Me, $^i$Pr, $^t$Bu, CN, $CF_3$ und Ph;
wobei $ die Bindungsstelle einer Einfachbindung darstellt, mittels derer $R^A$ angebunden ist.
wobei $R^B$ ausgewählt ist aus der Gruppe bestehend aus
H,
Ph,
CN,
$CF_3$,
aus einer chemischen Struktur der Formel IIA-2

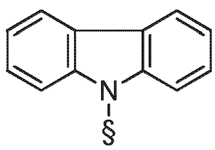

Formel IIA-2,

oder einer chemischen Struktur der Formel IIB-2

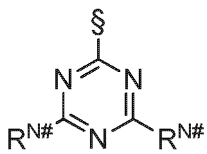

Formel IIB-2;

wobei $R^{N\#}$ ausgewählt ist aus der Gruppe bestehend aus Me, $^{i}Pr$, $^{t}Bu$, CN, $CF_3$ und Ph;
wobei S die Bindungsstelle einer Einfachbindung darstellt und.
wobei $R^b$ bei jedem Auftreten unabhängig voneinander ausgewählt ist aus der Gruppe bestehend aus:

H,
Me,
$^{i}Pr$,
$^{t}Bu$,
CN,
$CF_3$,
Ph, das optional substituiert ist mit einem oder mehreren Substituenten unabhängig ausgewählt aus der Gruppe bestehend aus Me, $^{i}Pr$, $^{t}Bu$, CN, $CF_3$ und Ph,
Pyridinyl, das optional substituiert ist mit einem oder mehreren Substituenten unabhängig ausgewählt aus der Gruppe bestehend aus Me, $^{i}Pr$, $^{t}Bu$, CN, $CF_3$ und Ph,
Pyrimidinyl, das optional substituiert ist mit einem oder mehreren Substituenten unabhängig ausgewählt aus der Gruppe bestehend aus Me, $^{i}Pr$, $^{t}Bu$, CN, $CF_3$ und Ph,
Carbazolyl, das optional substituiert ist mit einem oder mehreren Substituenten unabhängig ausgewählt aus der Gruppe bestehend aus Me, $^{i}Pr$, $^{t}Bu$, CN, $CF_3$ und Ph,
Triazinyl, das optional substituiert ist mit einem oder mehreren Substituenten unabhängig ausgewählt aus der Gruppe bestehend aus Me, $^{i}Pr$, $^{t}Bu$, CN, $CF_3$ und Ph,
und $N(Ph)_2$.

In einer Ausführungsform der Erfindung ist $R^1$ bei jedem Auftreten unabhängig voneinander $C_6$-$C_{18}$-Aryl, das optional substituiert ist mit einem oder mehreren Substituenten $R^6$.

In einer Ausführungsform der Erfindung ist $R^1$ bei jedem Auftreten unabhängig voneinander ausgewählt aus der Gruppe bestehend aus
Phenyl (Ph), das optional substituiert ist mit einem oder mehreren Substituenten $R^6$.

In einer Ausführungsform der Erfindung ist $R^1$ gleich Phenyl.

In einer Ausführungsform der Erfindung ist $R^N$ gleich Phenyl.

In einer Ausführungsform der Erfindung ist $R^{N\#}$ gleich Phenyl.

In einer Ausführungsform der Erfindung ist $R^2$ bei jedem Auftreten unabhängig voneinander ausgewählt aus der Gruppe bestehend aus

Wasserstoff,
CN,
$CF_3$ und
$C_6$-$C_{18}$-Aryl, das optional substituiert ist mit einem oder mehreren Substituenten $R^6$.

In einer Ausführungsform der Erfindung ist $R^2$ bei jedem Auftreten unabhängig voneinander ausgewählt aus der Gruppe bestehend aus

Wasserstoff,
CN,
$CF_3$ und
Phenyl, das optional substituiert ist mit einem oder mehreren Substituenten $R^6$.

In einer Ausführungsform der Erfindung ist $R^2$ bei jedem Auftreten unabhängig voneinander ausgewählt aus der Gruppe bestehend aus
Wasserstoff, CN, $CF_3$ und Phenyl.

In einer Ausführungsform der Erfindung ist $R^2$ bei jedem Auftreten unabhängig voneinander ausgewählt aus der Gruppe bestehend aus
Wasserstoff, CN und $CF_3$.

In einer Ausführungsform der Erfindung ist $R^2$ bei jedem Auftreten unabhängig voneinander ausgewählt aus der Gruppe bestehend aus
Wasserstoff und CN.

In einer Ausführungsform der Erfindung ist $R^2$ bei jedem Auftreten gleich Wasserstoff.

In einer Ausführungsform der organischen Moleküle ist $R^1$ bei jedem Auftreten unabhängig voneinander

Phenyl, das optional substituiert ist mit einem oder mehreren Substituenten $R^6$, und

$R^2$, das bei jedem Auftreten unabhängig voneinander ausgewählt ist aus der Gruppe bestehend aus Wasserstoff, CN, $CF_3$ und Phenyl, das optional substituiert ist mit einem oder mehreren Substituenten $R^6$.

In einer Ausführungsform der organischen Moleküle ist $R^1$ bei jedem Auftreten Phenyl,

$R^2$ ist bei jedem Auftreten unabhängig voneinander ausgewählt aus der Gruppe bestehend aus Wasserstoff, CN, $CF_3$ und Phenyl;

$R^N$ ist bei jedem Auftreten gleich Phenyl, und

$R^{N\#}$ ist bei jedem Auftreten ist gleich Phenyl.

In einer Ausführungsform der organischen Moleküle ist $R^1$ bei jedem Auftreten Phenyl,

$R^2$ ist bei jedem Auftreten gleich Wasserstoff,

$R^N$ ist bei jedem Auftreten gleich Phenyl; und

$R^{N\#}$ ist bei jedem Auftreten ist gleich Phenyl.

In einer Ausführungsform ist $R^6$ ausgewählt aus der Gruppe bestehend aus Me, $^i$Pr, $^t$Bu, CN, $CF_3$ und Ph.

In einer Ausführungsform ist X ausgewählt aus der Gruppe bestehend aus $SiMe_3$, $SiPh_3$, CN und $CF_3$.

In einer bevorzugten Ausführungsform ist X ausgewählt aus der Gruppe bestehend aus CN und $CF_3$.

In einer bevorzugten Ausführungsform ist X gleich CN.

In einer Ausführungsform ist $R^B$ ausgewählt aus der Gruppe bestehend aus

H,

Ph,

CN,

$CF_3$,

oder einer chemischen Struktur der Formel IIB-2

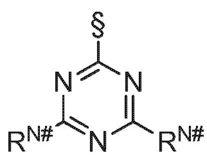

Formel IIB-2;

wobei $R^{N\#}$ ausgewählt ist aus der Gruppe bestehend aus Me, $^i$Pr, $^t$Bu, CN, $CF_3$ und Ph.

In einer Ausführungsform ist $R^B$ ausgewählt aus der Gruppe bestehend aus

H,

Ph,

CN,

$CF_3$,

oder einer chemischen Struktur der Formel IIB-2

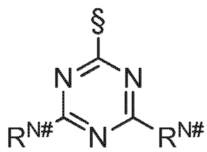

Formel IIB-2;

wobei $R^{N\#}$ gleich Ph ist.

In einer Ausführungsform ist $R^B$ ausgewählt aus der Gruppe bestehend aus

H,
Ph,
CN,
oder einer chemischen Struktur der Formel IIB-2

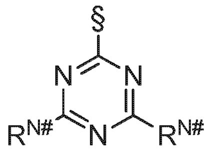

Formel IIB-2;

wobei $R^{N\#}$ gleich Ph ist.

In einer Ausführungsform ist $R^B$ ausgewählt aus der Gruppe bestehend aus

H,
oder einer chemischen Struktur der Formel IIB-2

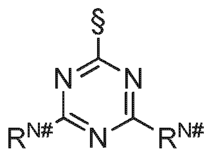

Formel IIB-2;

wobei $R^{N\#}$ gleich Ph ist.

In einer Ausführungsform ist $R^B$ gleich H.

In einer Ausführungsform ist $R^B$ bestehend aus einer chemischen Struktur der Formel IIB-2

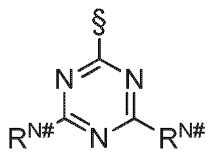

Formel IIB-2;

wobei $R^{N\#}$ gleich Ph ist.

In einer weiteren bevorzugten Ausführungsform ist $R^B$ ausgewählt aus der Gruppe bestehend aus

H,
oder einer chemischen Struktur der Formel IIB-2

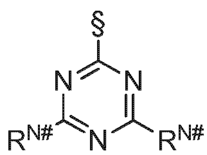

Formel IIB-2;

wobei $R^{N\#}$ gleich Ph ist.

Die erfindungsgemäßen organischen Moleküle umfassen oder bestehen aus einer Struktur, die ausgewählt ist

aus der Gruppe bestehend aus Formel IIa-1, Formel IIa-2, und Formel IIa-3:

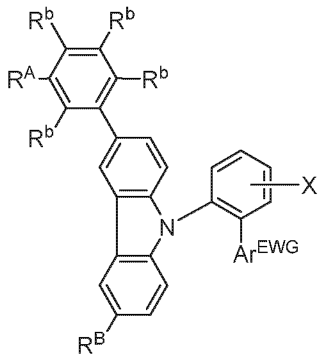

Formel IIa-1

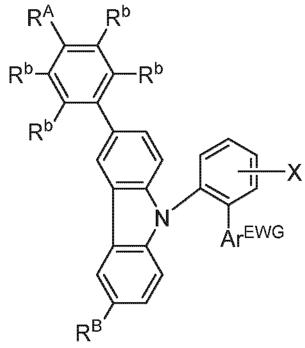

Formel IIa-2

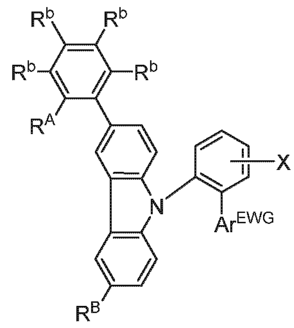

Formel IIa-3

wobei $R^b$ bei jedem Auftreten unabhängig voneinander ausgewählt aus der Gruppe bestehend aus

H,
Me,
$^iPr$,
$^tBu$,
CN,
$CF_3$,
Ph, das optional substituiert ist mit einem oder mehreren Substituenten unabhängig ausgewählt aus der Gruppe bestehend aus Me, $^iPr$, $^tBu$, CN, $CF_3$ und Ph,
Pyridinyl, das optional substituiert ist mit einem oder mehreren Substituenten unabhängig ausgewählt aus der Gruppe bestehend aus Me, $^iPr$, $^tBu$, CN, $CF_3$ und Ph,
Pyrimidinyl, das optional substituiert ist mit einem oder mehreren Substituenten unabhängig ausgewählt aus der Gruppe bestehend aus Me, $^iPr$, $^tBu$, CN, $CF_3$ und Ph,
Carbazolyl, das optional substituiert ist mit einem oder mehreren Substituenten unabhängig ausgewählt aus der Gruppe bestehend aus Me, $^iPr$, $^tBu$, CN, $CF_3$ und Ph,
Triazinyl, das optional substituiert ist mit einem oder mehreren Substituenten unabhängig ausgewählt aus der Gruppe bestehend aus Me, $^iPr$, $^tBu$, CN, $CF_3$ und Ph,
und $N(Ph)_2$.

In einer weiteren Ausführungsform der Erfindung ist $R^b$ bei jedem Auftreten unabhängig voneinander ausgewählt ist aus der Gruppe bestehend aus

H,
Me,
$^iPr$,
$^tBu$,
CN,
$CF_3$,
Ph, das optional substituiert ist mit einem oder mehreren Substituenten unabhängig ausgewählt aus der Gruppe bestehend aus Me, $^iPr$, $^tBu$, CN, $CF_3$ und Ph,
Pyridinyl, das optional substituiert ist mit einem oder mehreren Substituenten unabhängig ausgewählt aus der Gruppe bestehend aus Me, $^iPr$, $^tBu$, CN, $CF_3$ und Ph,
Pyrimidinyl, das optional substituiert ist mit einem oder mehreren Substituenten unabhängig ausgewählt aus der Gruppe bestehend aus Me, $^iPr$, $^tBu$, CN, $CF_3$ und Ph und
Triazinyl, optional substituiert ist mit einem oder mehreren Substituenten unabhängig ausgewählt aus der Gruppe bestehend aus Me, $^iPr$, $^tBu$, CN, $CF_3$ und Ph.

In einer weiteren Ausführungsform der Erfindung ist $R^b$ bei jedem Auftreten unabhängig voneinander ausgewählt ist aus der Gruppe bestehend aus

H,

CN,
$CF_3$.

In einer weiteren Ausführungsform der Erfindung ist $R^b$ bei jedem Auftreten unabhängig voneinander ausgewählt ist aus der Gruppe bestehend aus
H und CN.

In einer zusätzlichen Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2, Formel IIc-3, Formel IIc-4 und Formel IIc-5:

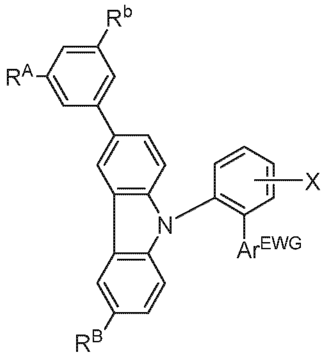
Formel IIc-1

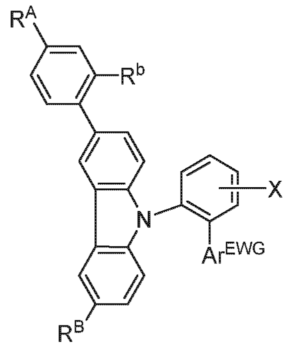
Formel IIc-2

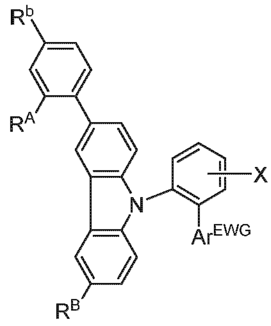
Formel IIc-3.

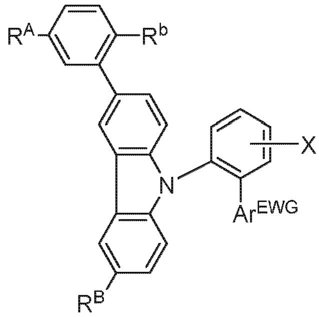
Formel IIc-4

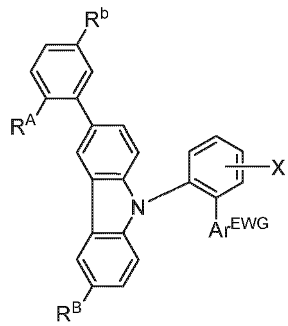
Formel IIc-5.

wobei die vorstehend erwähnten Definitionen gelten.

In einer zusätzlichen Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2, und Formel IIc-3:

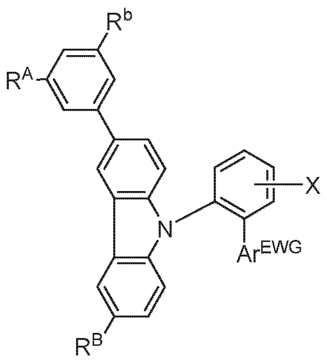
Formel IIc-1

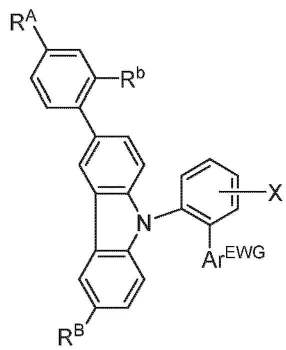
Formel IIc-2

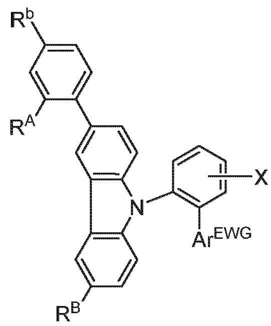
Formel IIc-3.

In einer weiteren Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2, Formel IIc-3,

Formel IIc-4 und Formel IIc-5, wobei
$R^b$ bei jedem Auftreten ausgewählt aus der Gruppe bestehend aus H und CN.

In einer weiteren Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2 und Formel IIc-3, wobei
$R^b$ bei jedem Auftreten ausgewählt aus der Gruppe bestehend aus H und CN.

In einer weiteren Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2, Formel IIc-3, Formel IIc-4 und Formel IIc-5, wobei
$R^b$ gleich H ist.

In einer weiteren Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2 und Formel IIc-3, wobei
$R^b$ gleich H ist.

In einer weiteren Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2, Formel IIc-3, Formel IIc-4 und Formel IIc-5, wobei
$R^b$ gleich CN ist.

In einer weiteren Ausführungsform der Erfindung umfassen oder bestehen die erfindungsgemäßen organischen Moleküle aus einer Struktur, die ausgewählt ist aus der Gruppe bestehend aus Formel IIc-1, Formel IIc-2 und Formel IIc-3, wobei
$R^b$ gleich CN ist.

In einer bevorzugten Ausführungsform der Erfindung umfassen oder bestehen die erfindungsgemäßen organischen Moleküle aus einer Struktur gemäß Formel IIc-1:

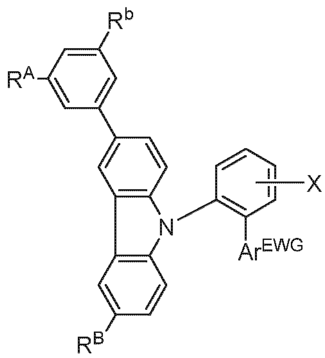

Formel IIc-1

wobei $R^b$ bei jedem Auftreten ausgewählt aus der Gruppe bestehend aus H und CN ist.

In einer bevorzugten Ausführungsform der Erfindung umfassen oder bestehen die erfindungsgemäßen organischen Moleküle aus der nachfolgend dargestellten Gruppe:

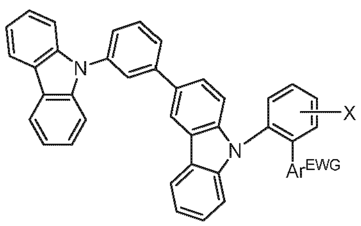
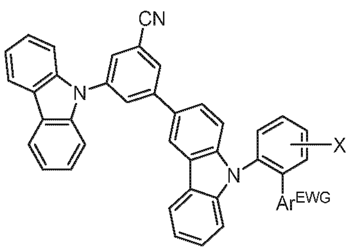
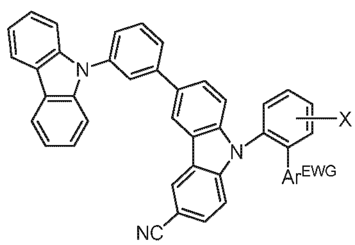

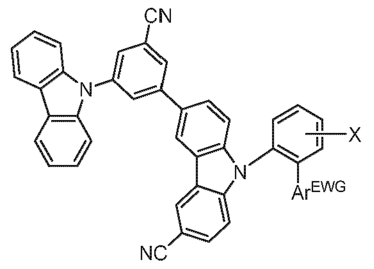

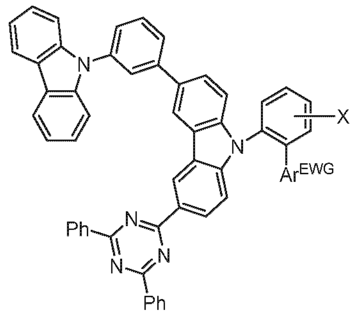

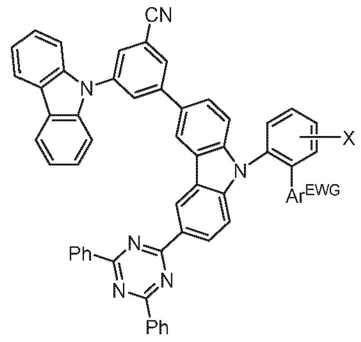

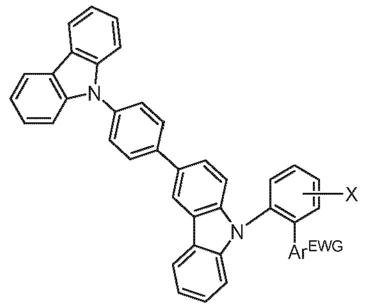

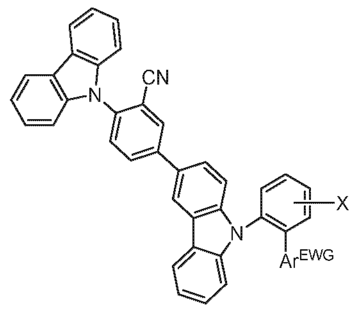

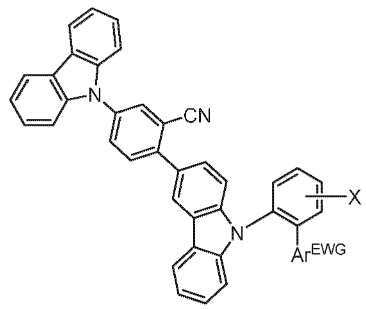

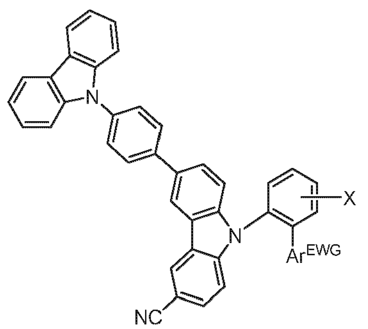

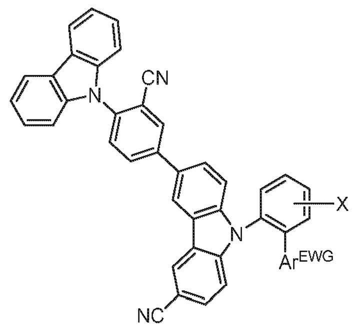

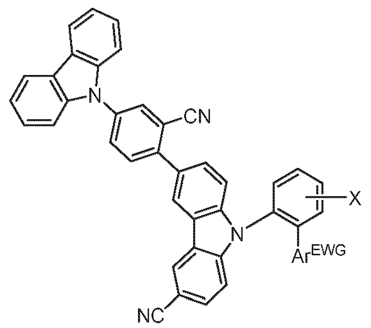

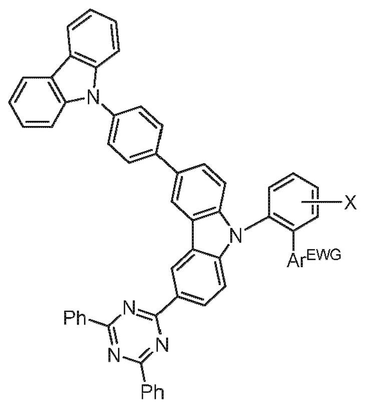

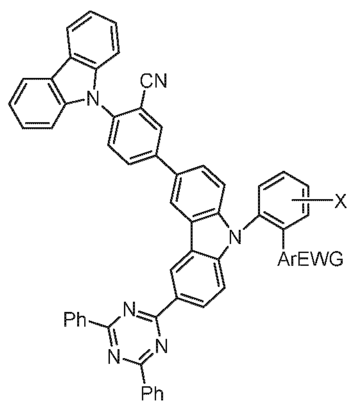

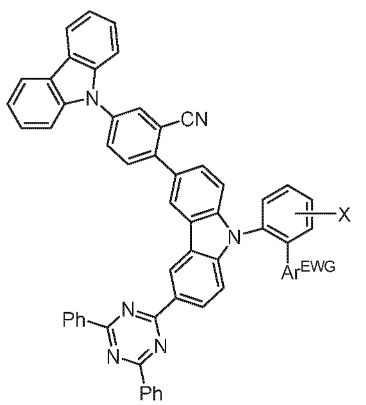

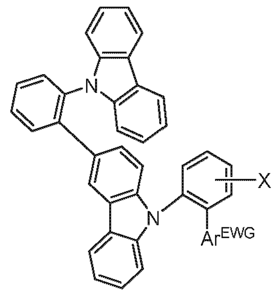
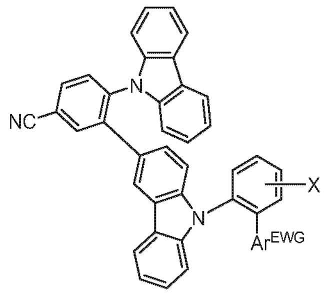
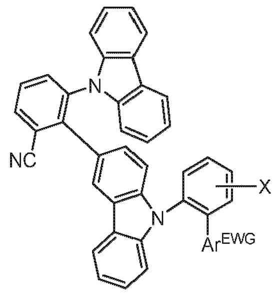

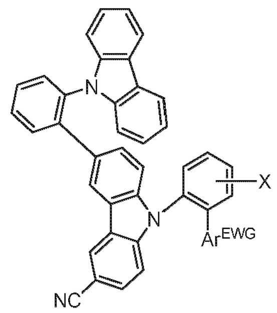
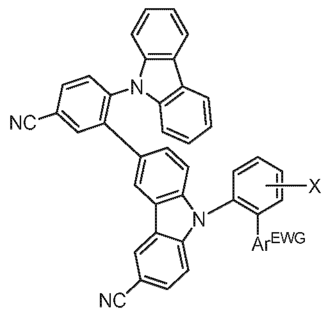
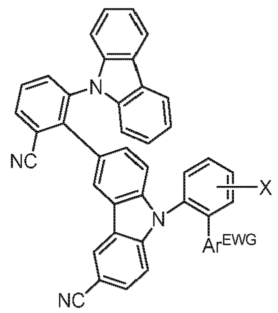

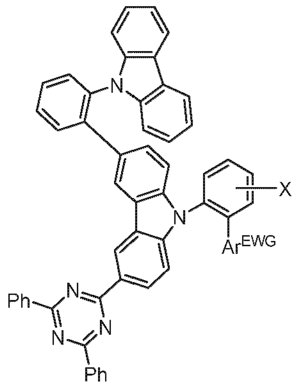
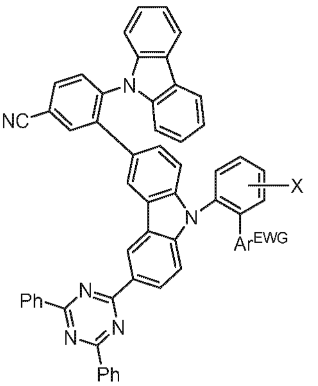
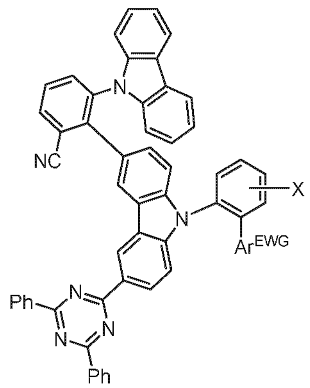

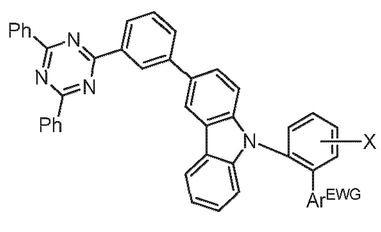
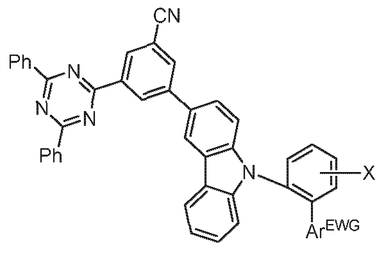
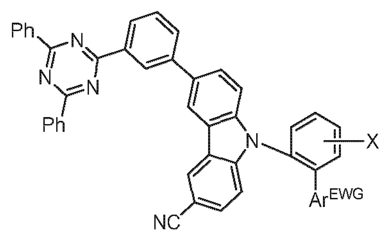

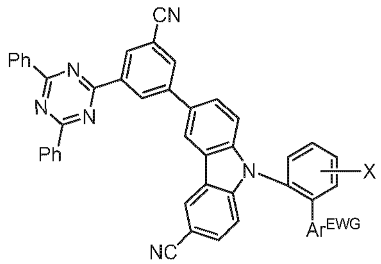
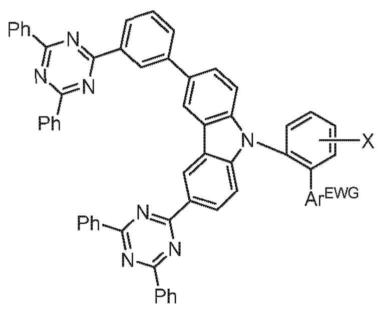
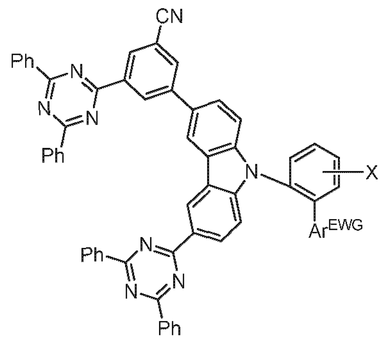

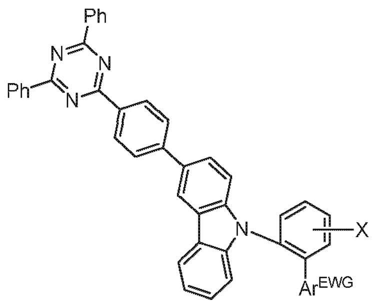
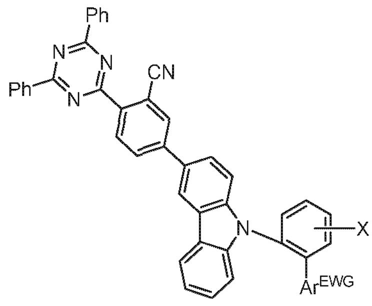
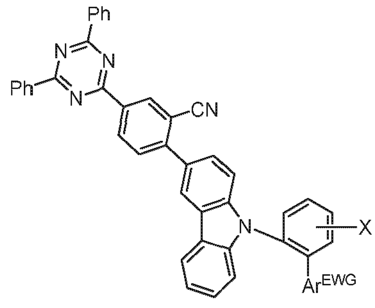
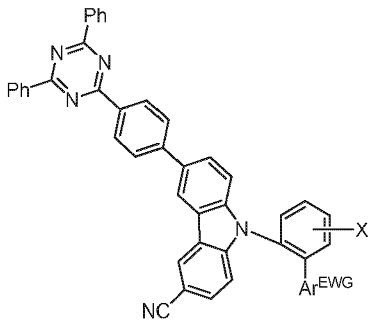
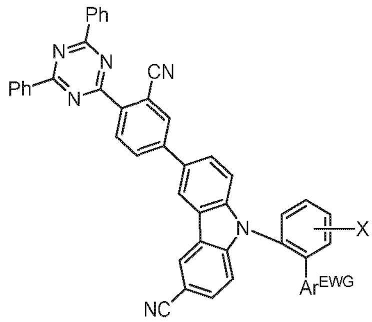
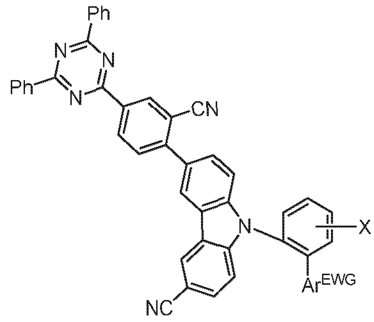
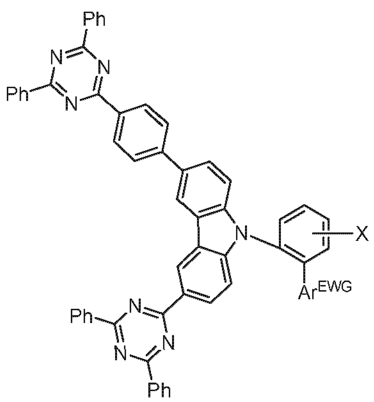
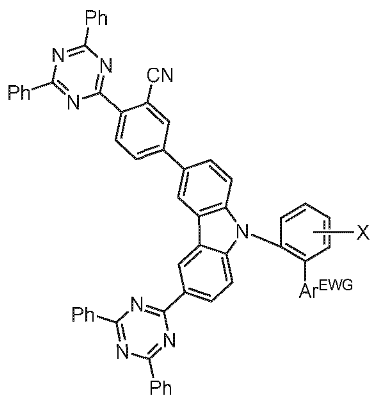
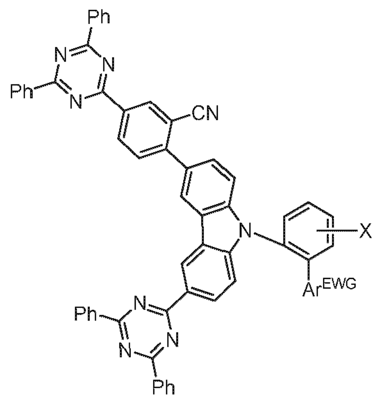
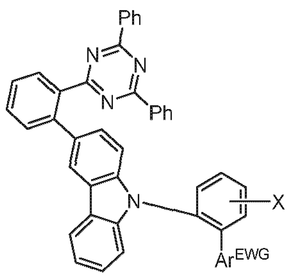
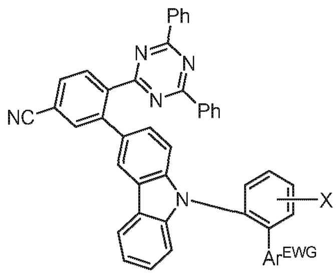
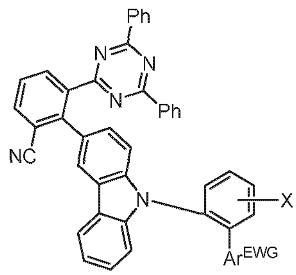
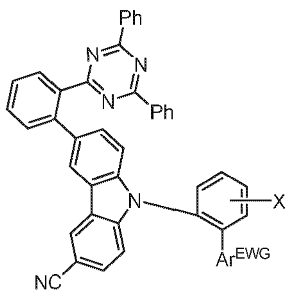
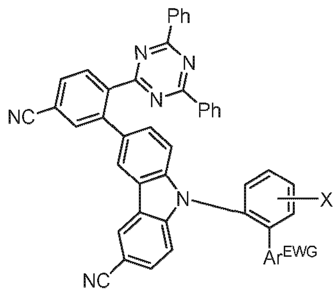
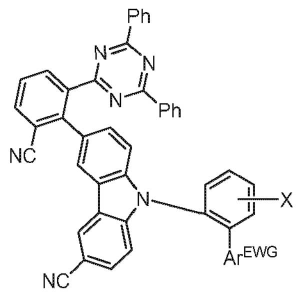

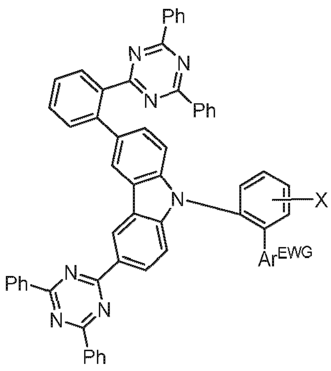

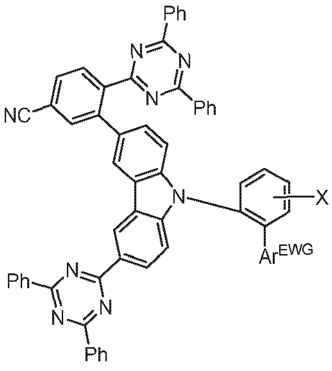

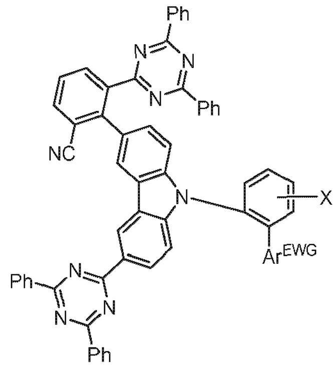

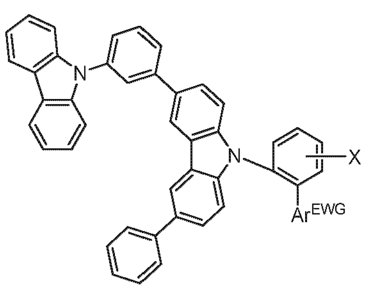

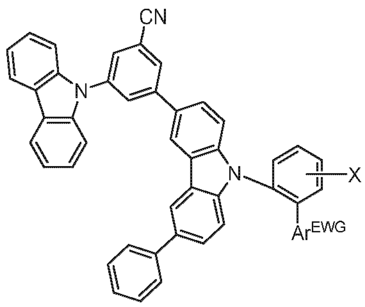

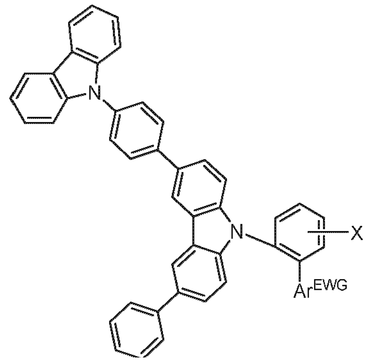

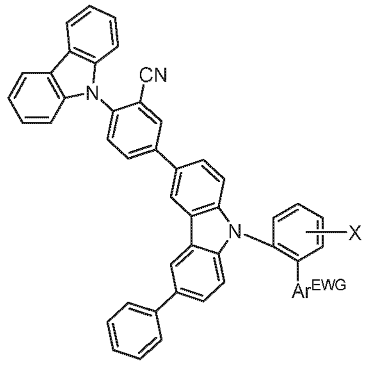

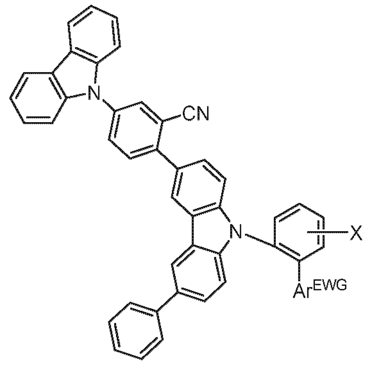

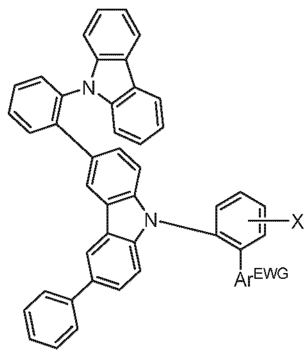

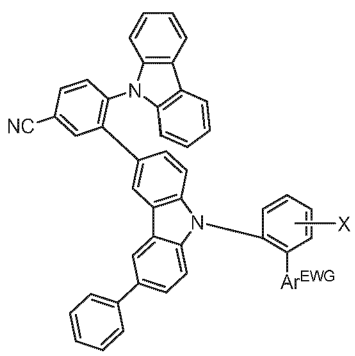

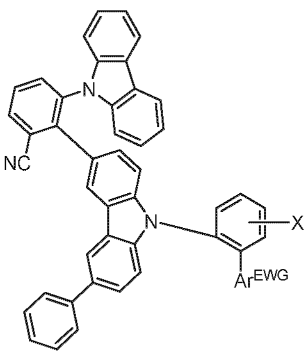

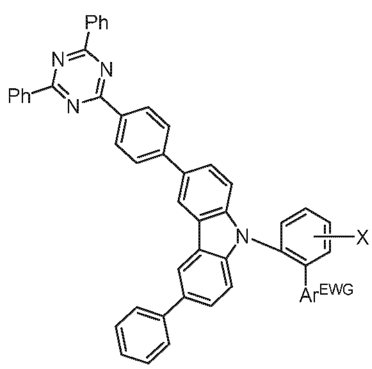

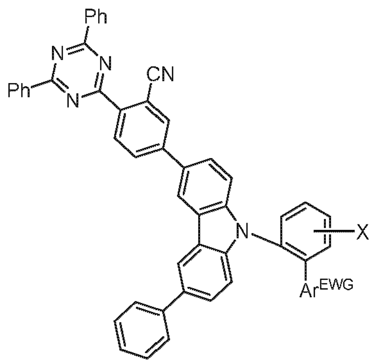
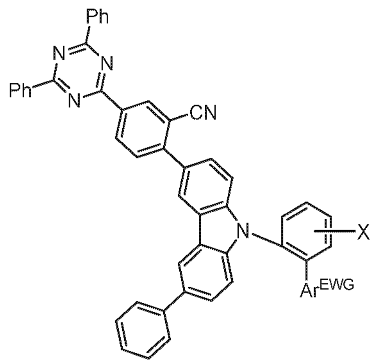
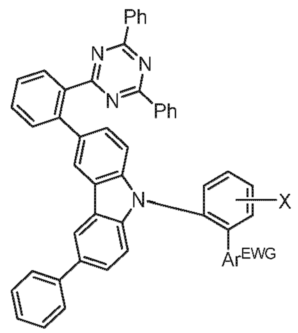

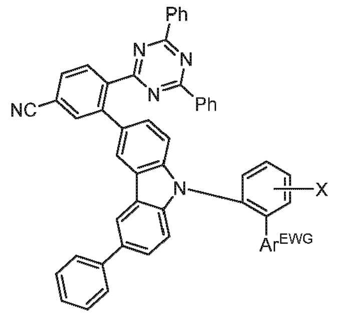
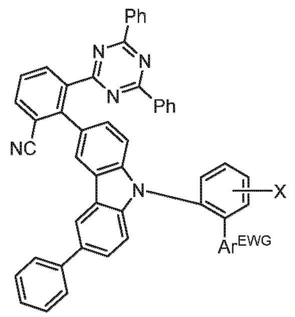
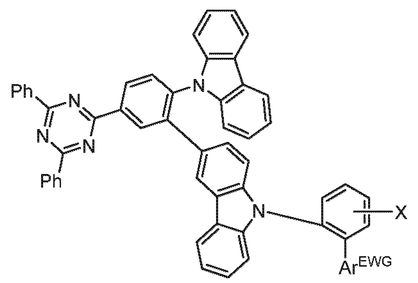

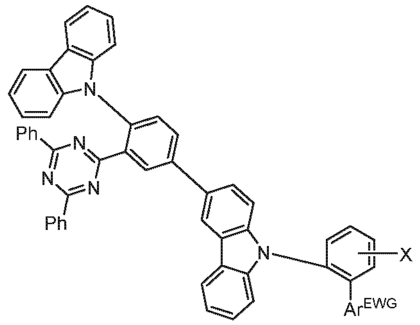
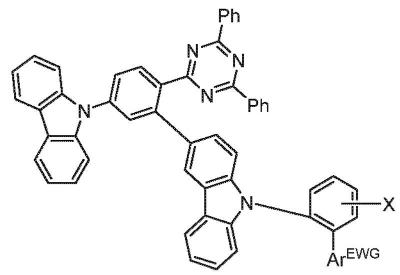
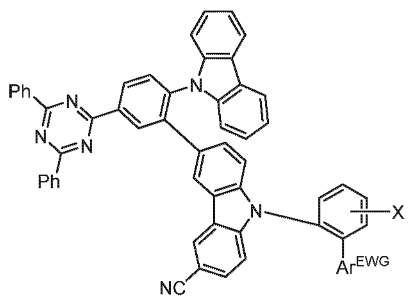

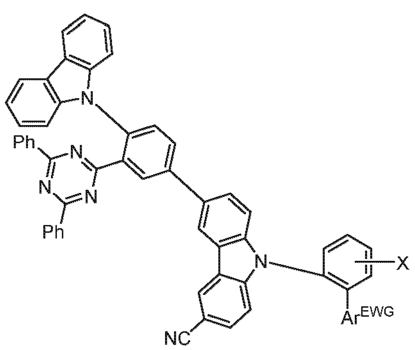
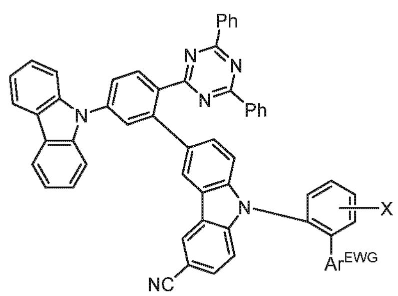
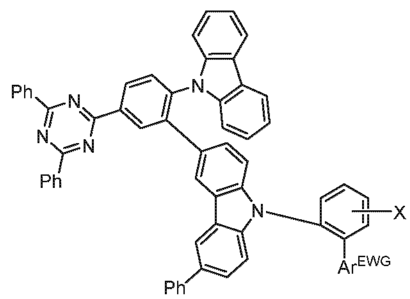

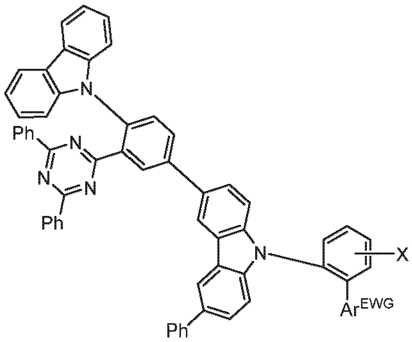
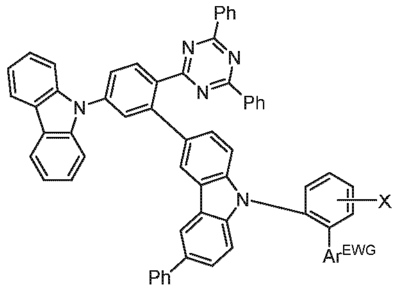

Gemäß der Erfindung ist $Ar^{EWG}$ ausgewählt ist aus der Gruppe bestehend aus

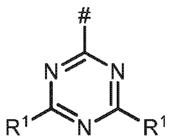
Formula IIa

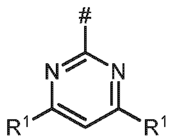
Formula IIb

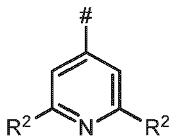
Formula IIc

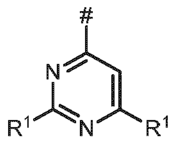
Formula IIi

In einer bevorzugten Ausführungsform der Erfindung ist $Ar^{EWG}$ ausgewählt ist aus der Gruppe bestehend aus

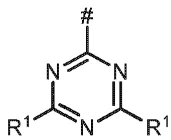
Formula IIa

Wie in der gesamten Beschreibung verwendet können die Begriffe "Aryl" und "aromatisch" im weitesten Sinne als jede beliebige mono-, bi- oder polycyclische aromatische Komponente verstanden werden. Dementsprechend enthält eine Arylgruppe 6 bis 60 aromatische Ringatome und eine Heteroarylgruppe enthält 5 bis 60 aromatische Ringatome, von denen mindestens eines ein Heteroatom ist. Nichtsdestotrotz kann die Anzahl von aromatischen Ringatomen in der gesamten Beschreibung in der Definition bestimmter Substituenten als tiefgestellte Anzahl gegeben werden. Insbesondere beinhaltet der heteroaromatische Ring ein bis drei Heteroatome. Die Begriffe "Heteroaryl" und "heteroaromatisch" können im weitesten Sinne als jede beliebige mono-, bi- oder polycyclische heteroaromatische Komponente, die mindestens ein Heteroatom beinhaltet, verstanden werden. Die Heteroatome können bei jedem Auftreten gleich oder unterschiedlich sein und können individuell ausgewählt sein aus der Gruppe bestehend aus N, O und S. Dementsprechend bezieht sich der Begriff "Arylen" auf einen zweiwertigen Substituenten, der zwei Bindungsstellen an andere molekulare Strukturen trägt und somit als eine Verbindungsgliedstruktur dient. Falls eine Gruppe in den beispielhaften Ausführungsformen anders als die hier gegebenen Definitionen definiert ist, zum Beispiel, wenn die Anzahl der aromatischen Ringatome oder die Anzahl von Heteroatomen von der gegebenen Definition abweicht, ist die Definition in den beispielhaften Ausführungsformen anzuwenden. Erfindungsgemäß besteht ein kondensierter (annulierter) aromatischer oder heteroaromatischer Polycyclus aus zwei oder mehreren einzelnen aromatischen oder heteroaromatischen Cyclen, die den Polycyclus durch eine Kondensationsreaktion gebildet haben.

Insbesondere, wie in der gesamten Beschreibung verwendet, umfasst der Begriff Arylgruppe oder Heteroarylgruppe Gruppen, die über eine beliebige Position der aromatischen oder heteroaromatischen Gruppe gebunden werden können, abgeleitet von Benzol, Naphthalin, Anthracen, Phenanthren, Pyren, Dihydropyren, Chrysen, Perylen, Fluoranthen, Benzanthracen, Benzphenanthren, Tetracen, Pentacen, Benzpyren, Furan, Benzofuran, Isobenzofuran, Dibenzofuran, Thiophen, Benzothiophen, Isobenzothiophen, Dibenzothiophen; Pyrrol, Indol, Isoindol, Carbazol, Pyridin, Chinolin,

Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthoimidazol, Phenanthroimidazol, Pyridoimidazol, Pyrazinoimidazol, Chinoxalinoimidazol, Oxazol, Benzoxazol, Napthooxazol, Anthroxazol, Phenanthroxazol, Isoxazol, 1,2-Thiazol, 1,3-Thiazol, Benzothiazol, Pyridazin, Benzopyridazin, Pyrimidin, Benzopyrimidin, 1,3,5-Triazin, Chinoxalin, Pyrazin, Phenazin, Naphthyridin, Carbolin, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,2,3,4-Tetrazin, Purin, Pteridin, Indolizin und Benzothiadiazol oder Kombinationen aus den vorstehend erwähnten Gruppen.

Wie in der gesamten vorliegenden Beschreibung verwendet, kann der Begriff cyclische Gruppe im weitesten Sinne als mono-, bi- oder polycyclische Komponenten verstanden werden.

Wie in der gesamten vorliegenden Beschreibung verwendet, kann der Begriff Alkylgruppe im weitesten Sinne als ein beliebiger linearer, verzweigter oder cyclischer Alkylsubstituent verstanden werden. Insbesondere umfasst der Begriff Alkyl die Substituenten Methyl (Me), Ethyl (Et), n-Propyl ("Pr), i-Propyl ($^i$Pr), Cyclopropyl, n-Butyl ("Bu), i-Butyl ('Bu), s-Butyl ($^s$Bu), t-Butyl ($^t$Bu), Cyclobutyl, 2-Methylbutyl, n-Pentyl, s-Pentyl, t-Pentyl, 2-Pentyl, Neopentyl, Cyclopentyl, n-Hexyl, s-Hexyl, t-Hexyl, 2-Hexyl, 3-Hexyl, Neohexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, 2,2,2-Trifluorethyl, 1,1-Dimethyl-n-hex-1-yl, 1,1-Dimethyl-n-hept-1-yl, 1,1-Dimethyl-n-oct-1-yl, 1,1-Dimethyl-n-dec-1-yl, 1,1-Dimethyl-n-dodec-1-yl, 1,1-Dimethyl-n-tetradec-1-yl, 1,1-Dimethyl-n-hexadec-1-yl, 1,1-Dimethyl-n-octadec-1-yl, 1,1-Diethyl-n-hex-1-yl, 1,1-Diethyl-n-hept-1-yl, 1,1-Diethyl-n-oct-1-yl, 1,1-Diethyl-n-dec-1-yl, 1,1-Diethyl-n-dodec-1-yl, 1,1-Diethyl-n-tetradec-1-yl, 1,1-Diethyln-n-hexadec-1-yl, 1,1-Diethyl-n-octadec-1-yl, 1-(n-Propyl)-cyclohex-1-yl, 1-(n-Butyl)-cyclohex-1-yl, 1-(n-Hexyl)-cyclohex-1-yl, 1-(n-Octyl)-cyclohex-1-yl und 1-(n-Decyl)-cyclohex-1-yl.

Wie in der gesamten vorliegenden Beschreibung verwendet, umfasst der Begriff Alkenyl lineare, verzweigte und cyclische Alkenylsubstituenten. Der Begriff Alkenlygruppe umfasst beispielhaft die Substituenten Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl.

Wie in der gesamten Beschreibung verwendet, umfasst der Begriff Alkinyl lineare, verzweigte und cyclische Alkinylsubstituenten. Der Begriff Alkinylgruppe umfasst beispielhaft Ethinyl, Propinyl, Butinyl, Pentinyl, Hexinyl, Heptinyl oder Octinyl.

Wie in der gesamten Beschreibung verwendet, umfasst der Begriff Alkoxy lineare, verzweigte und cyclische Alkoxysubstituenten. Der Begriff Alkoxygruppe umfasst beispielhaft Methoxy, Ethoxy, n-Propoxy, i-Propoxy, n-Butoxy, i-Butoxy, s-Butoxy, t-Butoxy und 2-Methylbutoxy.

Wie in der gesamten Beschreibung verwendet, umfasst der Begriff Thioalkoxy lineare, verzweigte und cyclische Thioalkoxysubstituenten, in denen das O der beispielhaften Alkoxygruppen durch S ersetzt wird.

Wie in der gesamten Beschreibung verwendet, können die Begriffe "Halogen" und "Halo" im weitesten Sinne derart verstanden werden, dass sie vorzugsweise Fluor, Chlor, Brom oder Iod sind.

Wenn Wasserstoff hierin erwähnt wird, kann er bei jedem Auftreten auch durch Deuterium ersetzt werden.

Es versteht sich, dass, wenn ein molekulares Fragment derart beschrieben ist, dass es ein Substituent ist oder anderweitig an eine andere Komponente befestigt ist, kann dessen Name so geschrieben werden, als wenn es ein Fragment (z. B. Naphtyl, Dibenzofuryl) wäre oder als wenn es das gesamte Molekül (z. B. Naphthalin, Dibenzofuran) wäre. Wie hierin verwendet, werden diese unterschiedlichen Möglichkeiten, einen Substituenten oder ein befestigtes Fragment zu bezeichnen, als äquivalent betrachtet.

In einer Ausführungsform weisen die erfindungsgemäßen organischen Moleküle eine Lebensdauer im angeregten Zustand von nicht mehr als 150 μs, von nicht mehr als 100 μs, insbesondere von nicht mehr als 50 μs, weiter bevorzugt von nicht mehr als 10 μs oder nicht mehr als 7 μs in einem Film aus Poly(methylmethacrylat) (PMMA) mit 10 Gew.-% des organischen Moleküls bei Raumtemperatur auf.

In einer Ausführungsform der Erfindung stellen die erfindungsgemäßen organischen Moleküle thermisch aktivierte verzögerte Fluoreszenz(TADF)-Emitter dar, die einen • $E_{ST}$-Wert, der der Energiedifferenz zwischen dem ersten angeregten Singulettzustand (S1) und dem ersten angeregten Triplettzustand (T1) entspricht, von weniger als 5000 $cm^{-1}$, vorzugsweise weniger als 3000 $cm^{-1}$, weiter bevorzugt weniger als 1500 $cm^{-1}$, noch weiter bevorzugt weniger als 1000 $cm^{-1}$ oder sogar weniger als 500 $cm^{-1}$ aufweisen.

In einer weiteren Ausführungsform der Erfindung weisen die erfindungsgemäßen organischen Moleküle eine Emissionsspitze im sichtbaren oder nahen Ultraviolettbereich, d. h. im Bereich einer Wellenlänge von 380 bis 800 nm, mit einer Halbwertsbreite von weniger als 0,50 eV, vorzugsweise weniger als 0,48 eV, weiter bevorzugt weniger als 0,45 eV, noch weiter bevorzugt weniger als 0,43 eV oder sogar weniger als 0,40 eV in einem Film aus Poly(methylmethacrylat) (PMMA) mit 10 Gew.-% des organischen Moleküls bei Raumtemperatur auf.

In einer weiteren Ausführungsform der Erfindung weisen die erfindungsgemäßen organischen Moleküle einen "blauen Materialindex" ("blue material index") (BMI), der durch Dividieren der Photolumineszenzquantenausbeute (PLQY) in % durch die CIEy-Farbkoordinate des emittierten Lichts berechnet wird, von mehr als 150, insbesondere mehr als

200, vorzugsweise mehr als 250, weiter bevorzugt mehr als 300 oder sogar mehr als 500 auf.

Orbitalenergien und angeregte Zustandsenergien können entweder mithilfe experimenteller Verfahren oder durch Berechnungen, die quantenchemische Verfahren nutzen, insbesondere Berechnungen auf Grundlage der Dichtefunktionaltheorie, bestimmt werden. Die Energie des höchsten besetzen Molekülorbitals $E^{HOMO}$ wird durch Verfahren, die dem Fachmann bekannt sind, anhand cyclischer Voltametriemessungen mit einer Genauigkeit von 0,1 eV bestimmt. Die Energie des niedrigsten unbesetzten Molekülorbitals $E^{LUMO}$ wird als $E^{HOMO}$ + $E^{gap}$ berechnet, wobei $E^{gap}$ folgendermaßen bestimmt wird: Für Hostverbindungen wird der Versatz des Emissionsspektrums eines Films mit 10 Gew.-% des Hosts in Poly(methylmethacrylat) (PMMA) als $E^{gap}$verwendet, sofern nicht anderweitig angegeben. Für Emittermoleküle wird $E^{gap}$als die Energie bestimmt, bei der die Anregungs- und Emissionsspektren eines Films mit 10 Gew.-% des Emitters in PMMA durchqueren.

Die Energie des ersten angeregten Triplettzustands T1 wird anhand des Versatzes des Emissionsspektrums bei geringer Temperatur, typischerweise bei 77 K, bestimmt. Bei Hostverbindungen, bei denen der erste angeregte Singulettzustand und der geringste Triplettzustand energetisch durch >0,4 eV getrennt sind, ist die Phosphoreszenz üblicherweise in einem stationären Spektrum in 2-Me-THF sichtbar. Die Triplettenergie kann somit als der Versatz des Phosphoreszenzspektrums bestimmt werden. Für TADF-Emittermoleküle wird die Energie des ersten angeregten Triplettzustands T1 anhand des Versatzes des verzögerten Emissionsspektrums bei 77 K bestimmt, sofern nicht anderweitig in einem Film aus PMMA mit 10 Gew.-% des Emitters gemessen. Sowohl für die Host- und Emitterverbindungen wird die Energie des ersten angeregten Singulettzustands S1 anhand des Versatzes des Emissionsspektrums bestimmt, sofern nicht anderweitig in einem Film aus PMMA mit 10 Gew.-% der Host- oder Emitterverbindung gemessen. Der Versatz eines Emissionsspektrums wird durch Berechnen des Schnittpunkts der Tangente zum Emissionsspektrum mit der x-Achse bestimmt. Die Tangente zum Emissionsspektrum wird an der Seite des Emissionsbereichs mit hoher Energie, d. h., wo der Emissionsbereich ansteigt, indem er sich von Werten mit hoher Energie zu Werten mit niedriger Energie bewegt, und am Punkt bei halbem Maximum der maximalen Intensität des Emissionsspektrums festgelegt.

Ein weiterer Aspekt der Erfindung betrifft einen Prozess zum Herstellen erfindungsgemäßer organischer Moleküle (mit optionaler anschließender Reaktion), wobei eine Palladium-katalysierte Kreuzkupplungreaktion verwendet wird:

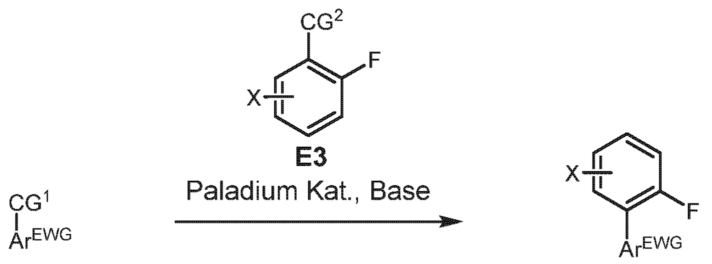


E2 E1

E1

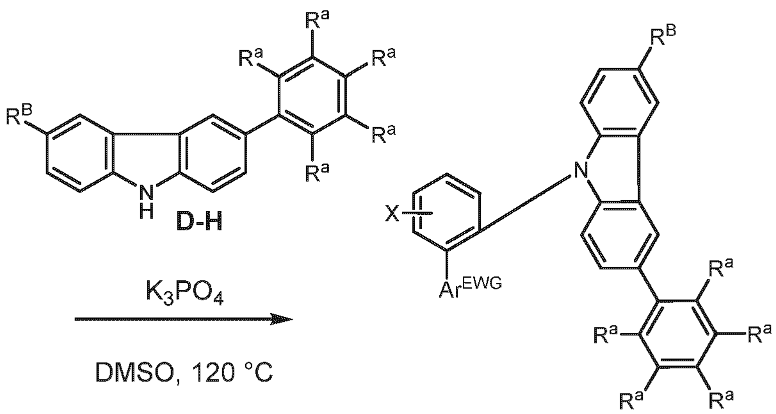


$R^a$ ist bei jedem Auftreten unabhängig voneinander ausgewählt aus der Gruppe bestehend aus

$R^A$

Wasserstoff, Deuterium, $N(R^6)_2$, $OR^6$, $Si(R^6)_3$, $B(OR^6)_2$, $OSO_2R^6$, $CF_3$, CN, F, Br, I, $C_1$-$C_{40}$-Alkyl,

das optional substituiert ist mit einem oder mehreren Substituenten $R^6$ und

wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_1$-$C_{40}$-Alkoxy,

das optional substituiert ist mit einem oder mehreren Substituenten $R^6$ und
wobei eine oder mehrere nicht benachbarte $CH_2$-Gruppen optional substituiert sind durch $R^6C=CR^6$, C•C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_1$-$C_{40}$-Thioalkoxy,

das optional substituiert ist mit einem oder mehreren Substituenten $R^6$ und
wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_2$-$C_{40}$-Alkenyl,

das optional substituiert ist mit einem oder mehreren Substituenten $R^6$ und
wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_2$-$C_{40}$-Alkinyl,

das optional substituiert ist mit einem oder mehreren Substituenten $R^6$ und
wobei eine oder mehrere nicht benachbarte $CH_2$-Gruppen optional substituiert sind durch $R^6C=CR^6$, C. C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_6$-$C_{60}$-Aryl,
das optional substituiert ist mit einem oder mehreren Substituenten $R^6$; und
$C_3$-$C_{57}$-Heteroaryl,
das optional substituiert ist mit einem oder mehreren Substituenten $R^6$.

Erfindungsgemäß reagiert E2, eine $Ar^{EWG}$-Gruppe, die mit der Kupplungsgruppe $CG^1$ substituiert ist, mit E3, einem Phenyl, welches mit X, der Kupplungsgruppe $CG^2$ und einmal F substituiert ist. Die Kupplungsgruppen $CG^1$ und $CG^2$ sind so als Reaktionspaar gewählt, dass die $Ar^{EWG}$-Gruppe von E2 an der Substitutionsposition $CG^2$ von E3 eingeführt wird. Bevorzugt wird hierbei eine sogenannte Suzuki-Kupplung. Hierbei wird entweder $CG^1$ aus Cl, Br oder I gewählt und $CG^2$ aus einer Boronsäure oder einem Boronsäureester, insbesondere einem Boronsäurepinakolester, gewählt, oder analog $CG^2$ aus Cl, Br oder I gewählt und $CG^1$ aus einer Boronsäure oder einem Boronsäureester, insbesondere einem Boronsäurepinakolester, gewählt.

D-H ist gemäß folgender Synthesewege herstellbar

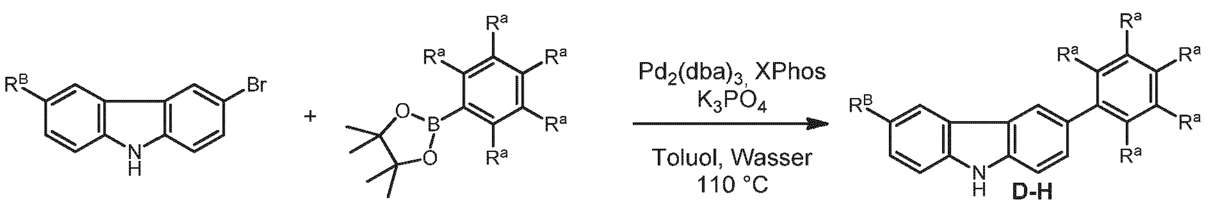


und

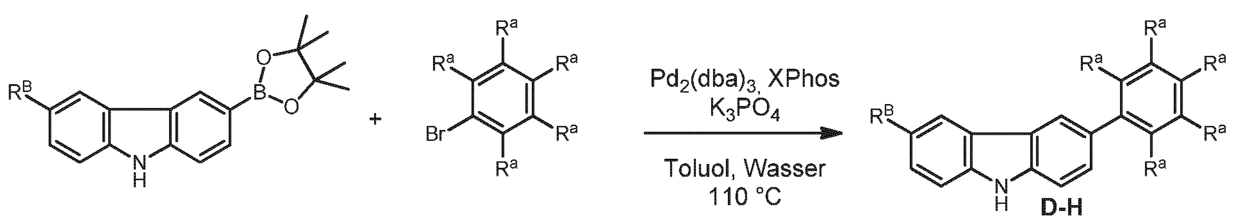

wobei der Boronsäureester auch durch eine Boronsäure ersetzt werden kann.

Typischerweise wird $Pd_2(dba)_3$ (Tris(dibenzylidenaceton)dipalladium(0)) als Palladium-Katalysator verwendet, aber Alternativen sind dem Fachmann bekannt. Beispielhaft wird der Ligand ausgewählt aus S-Phos ([2-Dicyclohexylphosphino-2',6'-dimethoxy-1,T'-biphenyl]; oder SPhos), XPhos (2-(dicyclohexylphosphino)-2",4",6"-triisopropylbiphenyl oder XPhos) und P(Cy)s (Tricyclohexylphosphin). Das Salz ist beispielhaft ausgewählt aus Kaliumphosphat, Kaliumcarbonat und Kaliumacetat und das Lösungsmittel kann entweder ein reines Lösungsmittel wie Toluol oder Dioxan oder ein Gemisch wie beispielsweise Toluol/Dioxan/Wasser sein. Dem Fachmann ist bekannt, welche Pd-Katalysator-, Ligand-, Salz- und Lösungsmittelkombination am wahrscheinlichsten zu hohen Ausbeuten führt.

Für die Reaktion eines Stickstoff-Heterocyclus in einer nukleophilen aromatischen Substitution mit einem Arylhalogenid, vorzugsweise einem Arylfluorid, beinhalten typische Bedingungen die Verwendung einer Base, wie etwa tribasisches Kaliumphosphat oder Natriumhydrid, zum Beispiel in einem aprotischen polaren Lösungsmittel, wie etwa beispielsweise Dimethylsulfoxid (DMSO) oder N,N-Dimethylformamid (DMF).

Ein alternativer Syntheseweg umfasst die Einführung eines Stickstoff-Heterocyclus über Kupfer- oder Palladium-katalysierte Kopplung an ein Arylhalogenid oder Arylpseudohalogenid, vorzugsweise ein Arylbromid, ein Aryliodid, Aryltriflat oder ein Aryltosylat.

Ein weiterer Aspekt der Erfindung betrifft die Verwendung eines erfindungsgemäßen organischen Moleküls als Lumineszenzemitter oder als Absorber und/oder als Hostmaterial und/oder als Elektronentransportmaterial und/oder als Lochinjektionsmaterial und/oder als Lochblockiermaterial in einer optoelektronischen Vorrichtung.

Die organische elektrolumineszierende Vorrichtung kann im weitesten Sinne als eine beliebige Vorrichtung auf Grundlage organischer Materialien verstanden werden, die zum Emittieren von Licht im sichtbaren oder nahen ultravioletten (UV) Bereich, d. h. im Bereich einer Wellenlänge von 380 bis 800 nm, geeignet ist. Weiter bevorzugt kann die organische elektrolumineszierende Vorrichtung in der Lage sein, Licht im sichtbaren Bereich, d. h. von 400 bis 800 nm, zu emittieren.

Im Zusammenhang mit solch einer Verwendung ist die organische optoelektronische Vorrichtung insbesondere ausgewählt aus der Gruppe bestehend aus:

- organischen lichtemittierenden Dioden (OLEDs),
- lichtemittierenden elektrochemischen Zellen,
- OLED-Sensoren, insbesondere in Gas- und Dampfsensoren nach außen nicht hermetisch abgeschirmt,
- organischen Dioden,
- organischen Solarzellen,
- organischen Transistoren,
- organischen Feldeffekttransistoren,
- organischen Lasern und
- Abwärtswandlungselementen (Down-Konversionselement, englisch: Down Conversion Element).

In einer bevorzugten Ausführungsform im Zusammenhang mit solch einer Verwendung ist die organische elektrolumineszierende Vorrichtung eine Vorrichtung ausgewählt aus der Gruppe bestehend aus einer organischen lichtemittierenden Diode (OLED), einer lichtemittierenden elektrochemischen Zelle (LEC) und eines lichtemittierenden Transistors.

Im Falle der Verwendung beträgt der Anteil des erfindungsgemäßen organischen Moleküls in der Emissionsschicht in einer optoelektronischen Vorrichtung, insbesondere in OLEDs, 1 Gew.-% bis 99 Gew.-%, insbesondere 5 Gew.-% bis 80 Gew.-%. In einer alternativen Ausführungsform beträgt die Menge des organischen Moleküls in der Emissionsschicht 100 Gew.-%.

In einer Ausführungsform umfasst die lichtemittierende Schicht nicht nur die erfindungsgemäßen organischen Moleküle, sondern auch ein Hostmaterial, dessen Triplett(T1)- und Singulett(S1)-Energieniveaus energetisch höher sind als die Triplett(T1)- und Singulett(S1)-Energieniveaus des organischen Moleküls.

Ein weiterer Aspekt der Erfindung betrifft eine Zusammensetzung, umfassend oder bestehend aus:

(a) mindestens einem erfindungsgemäßen organischen Molekül, insbesondere in der Form eines Emitters und/oder eines Hosts, und

(b) einem oder mehreren Emitter- und/oder Hostmaterialien, die sich von dem organischen Molekül gemäß der Erfindung unterscheiden, und

(c) optional einem oder mehreren Farbstoffen und/oder einem oder mehreren Lösungsmitteln.

In einer Ausführungsform umfasst die lichtemittierende Schicht eine Zusammensetzung (oder besteht (im Wesentlichen) daraus), umfassend oder bestehend aus:

(a) mindestens einem erfindungsgemäßen organischen Molekül, insbesondere in der Form eines Emitters und/oder eines Hosts, und
(b) einem oder mehreren Emitter- und/oder Hostmaterialien, die sich von dem organischen Molekül gemäß der Erfindung unterscheiden, und
(c) optional einem oder mehreren Farbstoffen und/oder einem oder mehreren Lösungsmitteln.

Besonders bevorzugt umfasst die lichtemittierende Schicht EML eine Zusammensetzung (oder besteht (im Wesentlichen) daraus), umfassend oder bestehend aus:

(i) 1-50 Gew.-%, vorzugsweise 5-40 Gew.-%, insbesondere 10-30 Gew.-% eines oder mehrerer erfindungsgemäßer organischer Moleküle;
(ii) 5-99 Gew.-%, vorzugsweise 30-94,9 Gew.-%, insbesondere 40-89 Gew.-% der mindestens einen Hostverbindung H; und
(iii) optional 0-94 Gew.-%, vorzugsweise 0,1-65 Gew.-%, insbesondere 1-50 Gew.-% der mindestens einen weiteren Hostverbindung D mit einer Struktur, die von der Struktur der erfindungsgemäßen Moleküle abweicht; und
(iv) optional 0-94 Gew.-%, vorzugsweise 0-65 Gew.-%, insbesondere 0-50 Gew.-% eines Lösungsmittels; und
(v) optional 0-30 Gew.-%, insbesondere 0-20 Gew.-%, vorzugsweise 0-5 Gew.-% von mindestens einem weiteren Emittermolekül F mit einer Struktur, die von der Struktur der erfindungsgemäßen Moleküle abweicht.

Vorzugsweise kann Energie von der Hostverbindung H zu einem oder mehreren organischen Molekülen gemäß der Erfindung übertragen werden, insbesondere vom ersten angeregten Triplettzustand T1(H) der Hostverbindung H zum ersten angeregten Triplettzustand T1(E) des einen oder der mehreren erfindungsgemäßen organischen Moleküle und/oder vom ersten angeregten Singulettzustand S1(H) der Hostverbindung H zum ersten angeregten Singulettzustand S1(E) des einen oder der mehreren erfindungsgemäßen organischen Moleküle übertragen.

In einer weiteren Ausführungsform umfasst die lichtemittierende Schicht EML eine Zusammensetzung (oder besteht (im Wesentlichen) daraus), umfassend oder bestehend aus:

(i) 1-50 Gew.-%, vorzugsweise 5-40 Gew.-%, insbesondere 10-30 Gew.-% eines erfindungsgemäßen organischen Moleküls;
(ii) 5-99 Gew.-%, vorzugsweise 30-94,9 Gew.-%, insbesondere 40-89 Gew.-% einer Hostverbindung H; und
(iii) optional 0-94 Gew.-%, vorzugsweise 0,1-65 Gew.-%, insbesondere 1-50 Gew.-% der mindestens einen weiteren Hostverbindung D mit einer Struktur, die von der Struktur der erfindungsgemäßen Moleküle abweicht; und
(iv) optional 0-94 Gew.-%, vorzugsweise 0-65 Gew.-%, insbesondere 0-50 Gew.-% eines Lösungsmittels; und
(v) optional 0-30 Gew.-%, insbesondere 0-20 Gew.-%, vorzugsweise 0-5 Gew.-% von mindestens einem weiteren Emittermolekül F mit einer Struktur, die von der Struktur der erfindungsgemäßen Moleküle abweicht.

In einer Ausführungsform weist die Hostverbindung H ein höchstes besetztes Molekülorbital HOMO(H) mit einer Energie $E^{HOMO}(H)$ im Bereich von -5 bis -6,5 eV auf, und die mindestens eine weitere Hostverbindung D weist ein höchstes besetztes Molekülorbital HOMO(D) mit einer Energie $E^{HOMO}(D)$ auf, wobei $E^{HOMO}(H) > E^{HOMO}(D)$.

In einer weiteren Ausführungsform weist die Hostverbindung H ein niedrigstes unbesetztes Molekülorbital LUMO(H) mit einer Energie $E^{LUMO}(H)$ auf, und die mindestens eine weitere Hostverbindung D weist ein niedrigstes unbesetztes Molekülorbital LUMO(D) mit einer Energie $E^{LUMO}(D)$ auf, wobei $E^{LUMO}(H) > E^{LUMO}(D)$.

In einer Ausführungsform weist die Hostverbindung H ein höchstes besetztes Molekülorbital HOMO(H) mit einer Energie $E^{HOMO}(H)$ und ein niedrigstes unbesetztes Molekülorbital LUMO(H) mit einer Energie $E^{LUMO}(H)$ auf, und

die mindestens eine weitere Hostverbindung D weist ein höchstes besetztes Molekülorbital HOMO(D) mit einer Energie $E^{HOMO}(D)$ und ein niedrgistes unbesetztes Molekülorbital LUMO(D) mit einer Energie $E^{LUMO}(D)$ auf,
das organische Molekül gemäß der Erfindung weist ein höchstes besetztes Molekülorbital HOMO(E) mit einer Energie $E^{HOMO}(E)$ und ein niedrgistes unbesetztes Molekülorbital LUMO(E) mit einer Energie $E^{LUMO}(E)$ auf,

wobei

$E^{HOMO}(H) > E^{HOMO}(D)$ und die Differenz zwischen dem Energieniveau des höchsten besetzten Molekülorbitals HOMO(E) des organischen Moleküls gemäß der Erfindung ($E^{HOMO}(E)$) und dem Energieniveau des höchsten besetzten Molekülorbitals HOMO(H) der Hostverbindung H ($E^{HOMO}(H)$) zwischen -0,5 eV und 0,5 eV, weiter bevorzugt zwischen -0,3 eV und 0,3 eV, noch weiter bevorzugt zwischen -0,2 eV und 0,2 eV oder sogar zwischen -0,1 eV und 0,1 eV beträgt; und
$E^{LUMO}(H) > E^{LUMO}(D)$ und die Differenz zwischen dem Energieniveau des niedrigsten unbesetzten Molekülorbitals

LUMO(E) des organischen Moleküls gemäß der Erfindung ($E^{LUMO}$(E)) und dem niedrigsten unbesetzten Molekülorbital LUMO(D) der mindestens einen weiteren Hostverbindung D ($E^{LUMO}$(D)) zwischen -0,5 eV und 0,5 eV, weiter bevorzugt zwischen -0,3 eV und 0,3 eV, noch weiter bevorzugt zwischen -0,2 eV und 0,2 eV oder sogar zwischen -0,1 eV und 0,1 eV beträgt.

In einem weiteren Aspekt betrifft die Erfindung eine organische optoelektronische Vorrichtung, die ein organisches Molekül oder eine Zusammensetzung des hier beschriebenen Typs umfasst, insbesondere in der Form einer Vorrichtung ausgewählt aus der Gruppe bestehend aus organischer lichtemittierender Diode (OLED), lichtemittierender elektrochemischer Zelle, OLED-Sensor, insbesondere Gas- und Dampfsensoren, die nach außen nicht hermetisch abgeschirmt sind, organischer Diode, organischer Solarzelle, organischem Transistor, organischem Feldeffekttransistor, organischem Laser und Down-Konversionselement (Abwärtswandlungselement).

In einer bevorzugten Ausführungsform ist die organische elektrolumineszierende Vorrichtung eine Vorrichtung ausgewählt aus der Gruppe bestehend aus einer organischen lichtemittierenden Diode (OLED), einer lichtemittierenden elektrochemischen Zelle (LEC) und eines lichtemittierenden Transistors.

In einer Ausführungsform der optoelektronischen Vorrichtung gemäß der Erfindung wird das organische Molekül gemäß der Erfindung als Emissionsmaterial in einer lichtemittierenden Schicht EML verwendet.

In einer Ausführungsform der optoelektronischen Vorrichtung gemäß der Erfindung besteht die lichtemittierende Schicht EML aus der hier beschriebenen Zusammensetzung gemäß der Erfindung.

Wenn die organische elektrolumineszierende Vorrichtung eine OLED ist, kann sie exemplarisch die folgende Schichtstruktur aufweisen:

1. Substrat
2. Anodenschicht A
3. Lochinjektionsschicht, HIL
4. Lochtransportschicht, HTL
5. Elektronenblockierschicht, EBL
6. emittierende Schicht, EML
7. Lochblockierschicht, HBL
8. Elektronentransportschicht, ETL
9. Elektroneninjektionsschicht, EIL
10. Kathodenschicht,

wobei die OLED jede Schicht nur optional umfasst, verschiedene Schichten zusammengeführt werden können und die OLED mehr als eine Schicht von jedem vorstehend definierten Schichttyp umfassen kann.

Außerdem kann die organische elektrolumineszierende Vorrichtung optional eine oder mehrere schützende Schichten umfassen, die die Vorrichtung vor schädigender Exposition gegenüber schädlichen Faktoren in der Umgebung schützt, einschließlich zum Beispiel Feuchtigkeit, Dampf und/oder Gasen.

In einer Ausführungsform der Erfindung ist die organische elektrolumineszierende Vorrichtung eine OLED, die die folgende umgekehrte Schichtstruktur aufweist:

1. Substrat
2. Kathodenschicht
3. Elektroneninjektionsschicht, EIL
4. Elektronentransportschicht, ETL
5. Lochblockierschicht, HBL
6. emittierende Schicht, B
7. Elektronenblockierschicht, EBL
8. Lochtransportschicht, HTL
9. Lochinjektionsschicht, HIL
10. Anodenschicht A

wobei die OLED mit einer umgekehrten Schichtstruktur jede Schicht nur optional umfasst, verschiedene Schichten zusammengeführt werden können und die OLED mehr als eine Schicht von jedem vorstehend definierten Schichttyp umfassen kann.

In einer Ausführungsform der Erfindung ist die organische elektrolumineszierende Vorrichtung eine OLED, die eine gestapelte Architektur aufweisen kann. In dieser Architektur sind die einzelnen Einheiten, im Gegensatz zur typischen Anordnung, wo die OLEDs Seite an Seite positioniert sind, aufeinander gestapelt. Gemischtes Licht kann mit OLEDs, die eine gestapelte Architektur aufweisen, generiert werden, insbesondere kann weißes Licht durch gestapelte

blaue, grüne und rote OLEDs generiert werden. Außerdem kann die OLED, die eine gestapelte Architektur aufweist, optional eine Ladungserzeugungsschicht (CGL) umfassen, die typischerweise zwischen zwei OLED-Untereinheiten positioniert ist und typischerweise aus einer n-dotierten und p-dotierten Schicht besteht, wobei die n-dotierte Schicht einer CGL typischerweise näher an der Anodenschicht positioniert ist.

In einer Ausführungsform der Erfindung ist die organische elektrolumineszierende Vorrichtung eine OLED, die zwei oder mehrere Emissionsschichten zwischen Anode und Kathode umfasst. Insbesondere umfasst diese sogenannte Tandem-OLED drei Emissionsschichten, wobei eine Emissionsschicht rotes Licht emittiert, eine Emissionsschicht grünes Licht emittiert und eine Emissionsschicht blaues Licht emittiert, und kann optional weitere Schichten umfassen, wie etwa Ladeungserzeugungsschichten, Blockier- oder Transportschichten zwischen den einzelnen Emissionsschichten. In einer weiteren Ausführungsform sind die Emissionsschichten benachbart gestapelt. In einer weiteren Ausführungsform umfasst die Tandem-OLED eine Ladungserzeugungsschichtung zwischen jeweils zwei Emissionsschichten. Zusätzlich können benachbarte Emissionsschichten oder Emissionsschichten, die durch eine Ladungserzeugungsschicht getrennt sind, zusammengeführt werden.

Das Substrat kann durch ein beliebiges Material oder eine beliebige Zusammensetzung von Materialien gebildet werden. Am häufigsten werden Glasobjektträger als Substrate verwendet. Alternativ können dünne Metallschichten (z. B. Kupfer, Gold, Silber oder Aluminiumfolien) oder Kunststofffolien oder -objektträger verwendet werden. Dies kann ein höheres Maß an Flexibilität ermöglichen. Die Anodenschicht A besteht meistens aus Materialien, die es ermöglichen, einen (im Wesentlichen) transparenten Film zu erhalten. Da mindestens eine von beiden Elektroden (im Wesentlichen) transparent sein sollte, um eine Lichtemission von der OLED zu ermöglichen, ist entweder die Anodenschicht A oder die Kathodenschicht C transparent. Vorzugsweise umfasst die Anodenschicht A einen großen Gehalt an oder besteht sogar aus transparenten leitenden Oxiden (TCOs). Solch eine Anodenschicht A kann beispielhaft Indiumzinnoxid, Aluminumzinkoxid, fluordotiertes Zinnoxid, Indiumzinkoxid, PbO, SnO, Zirkoniumoxide, Molybdänoxid, Vanadiumoxid, Wolframoxid, Graphit, dotiertes Si, dotiertes Ge, dotiertes GaAs, dotiertes Polyanilin, dotiertes Polypyrrol und/oder dotiertes Polythiophen umfassen.

Besonders bevorzugt besteht die Anodenschicht A (im Wesentlichen) aus Indiumzinnoxid (ITO) (z. B. (InO3)0,9(SnO2)0,1). Die Rauheit der Anodenschicht A, die von den transparenten leitenden Oxiden (TCOs) verursacht wird, kann durch Verwenden einer Lochinjektionsschicht (HIL) ausgeglichen werden. Außerdem kann die HIL die Injektion von Quasiladungsträgern (z. B. Löchern) erleichtern, in die der Transport von Quasiladungsträgern vom TCO zur Lochtransportschicht (HTL) erleichtert wird. Die Lochinjektionsschicht (HIL) kann Poly-3,4-ethylendioxythiophen (PEDOT), Polystyrolsulfonat (PSS), MoOz, VzOs, CuPC oder CuI, insbesondere ein Gemisch aus PEDOT und PSS umfassen. Die Lochinjektionsschicht (HIL) kann außerdem die Diffusion von Metall aus der Anodenschicht A in die Lochtransportschicht (HTL) verhindern. Die HIL kann beispielhaft PEDOT:PSS (Poly-3,4-ethylendioxythiophen: Polystyrolsulfonat), PEDOT (Poly-3,4-ethylendioxythiophen), mMTDATA (4,4;4'-Tris[phenyl(m-tolyl)amino]triphenylamin), Spiro-TAD (2,2;7,7'Tetrakis(n,n-diphenylamino)-9,9'-spirobifluoren), DNTPD (N1,N1'-(Biphenyl-4,4'-diyl)bis(N1-phenyl-N4,N4-di-m-tolylbenzol-1,4-diamin), NPB (N,N'-Nis-(1-naphthalenyl)-N,N'-bis-phenyl-(1,1'-biphenyl)-4,4'-diamin), NPNPB (N,N'-Diphenyl-N,N'-di-[4-(N,N-diphenyl-amino)phenyl]benzidin), MeO-TPD (N,N,N•,N•-Tetrakis(4-methoxyphenyl)benzidin), HAT-CN (1,4,5,8,9,11-Hexaazatriphenylen-hexacarbonitril) und/oder Spiro-NPD (N,N'Diphenyl-N,N'bis-(1-naphthyl)-9,9'spirobifluoren-2,7-diamin) umfassen.

Benachbart zur Anodenschicht A oder zur Lochinjektionsschicht (HIL) befindet sich typischerweise eine Lochtransportschicht (HTL). Hierin kann jede beliebige Lochtransportverbindung verwendet werden. Beispielhaft können elektronenreiche heteroaromatische Verbindungen, wie etwa Triarylamine und/oder Carbazole, als Lochtransportverbindung verwendet werden. Die HTL kann die Energiebarriere zwischen der Anodenschicht A und der lichtemittierenden Schicht EML reduzieren. Die Lochtransportschicht (HTL) kann außerdem eine Elektronenblockierschicht (EBL) sein. Vorzugsweise trägt die Lochtransportverbindung hohe Energieniveaus ihrer Triplettzustände T1. Beispielhaft kann die Lochtransportschicht (HTL) einen sternförmigen Heterocyclus umfassen, wie etwa Tris(4-carbazoyl-9-ylphenyl)amin (TCTA), Poly-TPD (Poly(4-butylphenyldiphenylamin)), [alpha]-NPD (Poly(4-butylphenyldiphenylamin)), TAPC (4,4'Cyclohexyliden-bis[N,N-bis(4-methylphenyl)benzenamin]), 2-TNATA (4,4;4'Tris[2-naphthyl(phenyl)amino]triphenylamin), Spiro-TAD, DNTPD, NPB, NPNPB, MeO-TPD, HAT-CN und/oder TrisPcz (9,9'-Diphenyl-6-(9-phenyl-9H-carbazol-3-yl)-9H,9'H-3,3'-bicarbazol). Zusätzlich kann die HTL eine p-dotierte Schicht umfassen, die aus einem anorganischen oder organischen Dotierstoff in einer organischen Lochtransportmatrix besteht. Übergangsmetalloxide, wie etwa Vanadiumoxid, Molybdänoxid oder Wolframoxid können beispielhaft als anorganischer Dotierstoff verwendet werden. Tetrafluortetracyanochinodimethan (F4-TCNQ), Kupferpentafluorbenzoat (Cu(I)pFBz) oder Übergangsmetallkomplexe können beispielhaft als organischer Dotierstoff verwendet werden.

Die EBL kann beispielhaft mCP (1,3-Bis(carbazol-9-yl)benzol), TCTA, 2-TNATA, mCBP (3,3-Di(9H-carbazol-9-yl)biphenyl), Tris-Pcz, CzSi (9-(4-Tert-butylphenyl)-3,6-bis(triphenylsilyl)-9H-carbazol) und/oder DCB (N,N-Dicarbazolyl-1,4-dimethylbenzol) umfassen.

Benachbart zur Lochtransportschicht (HTL) befindet sich typischerweise die lichtemittierende Schicht EML. Die lichtemittierende Schicht EML umfasst mindestens ein lichtemittierendes Molekül. Insbesondere umfasst die EML

mindestens ein lichtemittierendes Molekül gemäß der Erfindung. In einer Ausführungsform umfasst die lichtemittierende Schicht nur die erfindungsgemäßen organischen Moleküle. Typischerweise umfasst die EML zusätzlich ein oder mehrere Hostmaterialien. Beispielhaft ist das Hostmaterial ausgewählt aus CBP (4,4'-Bis-(N-carbazolyl)-biphenyl), mCP, mCBP Sif87 (Dibenzo[b,d]thiophen-2-yltriphenylsilan), CzSi, Sif88 (Dibenzo[b,d]thiophen-2-yl)diphenylsilan), DPEPO (Bis[2-(diphenylphosphino)phenyl]etheroxid), 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol, 9-[3,5-Bis(2-dibenzothiophenyl)phenyl]-9H-carbazol, T2T (2,4,6-Tris(biphenyl-3-yl)-1,3,5-triazin), T3T (2,4,6-Tris(triphenyl-3-yl)-1,3,5-triazin) und/oder TST (2,4,6-Tris(9,9-spirobifluoren-2-yl)-1,3,5-triazin). Das Hostmaterial sollte typischerweise so ausgewählt sein, dass es erste Triplett(T1)- und erste Singulett(S1)-Energieniveaus aufweist, die energetisch höher sind als die ersten Triplett(T1)- und ersten Singulett(S1)-Energieniveaus des organischen Moleküls.

In einer Ausführungsform der Erfindung umfasst die EML ein sogenanntes gemischtes Hostsystem mit mindestens einem lochdominanten Host und einem elektronendominanten Host. In einer besonderen Ausführungsform umfasst die EML genau ein erfindungsgemäßes lichtemittierendes Molekül und ein gemischtes Hostsystem, umfassend T2T als Elektronen-dominanter Host und einen Host ausgewählt aus CBP, mCP, mCBP, 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol und 9-[3,5-Bis(2-dibenzothiophenyl)phenyl]-9H-carbazol als Loch-dominanter Host. In einer weiteren Ausführungsform umfasst die EML 50-80 Gew.-%, vorzugsweise 60-75 Gew.-% eines Hosts ausgewählt aus CBP, mCP, mCBP, 9-[3-(Dibenzofuran-2-yl)phenyl]-9H-carbazol, 9-[3-(Dibenzofuran-2-yl)phenyl]-9H-carbazol, 9-[3-(Dibenzothiophen-2-yl)phenyl]-9H-carbazol, 9-[3,5-Bis(2-dibenzofuranyl)phenyl]-9H-carbazol und 9-[3,5-Bis(2-dibenzothiophenyl)phenyl]-9H-carbazol; 10-45 Gew.-%, vorzugsweise 15-30 Gew.-% von T2T und 5-40 Gew.-%, vorzugsweise 10-30 Gew.-% des erfindungsgemäßen lichtemittierenden Moleküls.

Benachbart zur lichtemittierenden Schicht EML kann sich eine Elektronentransportschicht (ETL) befinden. Hierin kann jeder beliebige Elektronenüberträger verwendet werden. Beispielhaft können Verbindungen mit wenigen Elektronen, wie etwa beispielsweise Benzimidazole, Pyridine, Triazole, Oxadiazole (z. B. 1,3,4-Oxadiazol), Phosphinoxide und Sulfon verwendet werden. Ein Elektronentransporter kann außerdem ein sternenförmiger Heterocyclus sein, wie etwa 1,3,5-Tri(1-phenyl-1H-benzo[d]imidazol-2-yl)phenyl (TPBi). Die ETL kann NBphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid), BPyTP2 (2,7 -Di(2,2--bipyridin-5-yl)triphenyl), Sif87 (Dibenzo[b,d]thiophen-2-yltriphenylsilan), Sif88 (Dibenzo[b,d]thiophen-2-yl)diphenylsilan), BmPyPhB (1,3-Bis[3,5-di(pyridin-3-yl)phenyl]benzol) und/oder BTB (4,4'Bis-[2-(4,6-diphenyl-1,3,5-triazinyl)]-1,1-biphenyl) umfassen. Optional kann die ETL mit Materialien, wie etwa Liq, dotiert sein. Die Elektronentransportschicht (ETL) kann außerdem Löcher blockieren oder eine Lochblockierschicht (HBL) wird eingeführt.

Die HBL kann beispielhaft BCP (2,9-Dimethyl-4,7-diphenyl-1,10-phenanthrolin = Bathocuproin), BAlq (Bis(8-hydroxy-2-methylchinolin)-(4-phenylphenoxy)aluminium), NBphen (2,9-Bis(naphthalen-2-yl)-4,7-diphenyl-1,10-phenanthrolin), Alq3 (Aluminium-tris(8-hydroxychinolin)), TSPO1 (Diphenyl-4-triphenylsilylphenyl-phosphinoxid), T2T (2,4,6-Tris(biphenyl-3-yl)-1,3,5-triazin), T3T (2,4,6-Tris(triphenyl-3-yl)-1,3,5-triazin), TST (2,4,6-Tris(9,9'spirobifluoren-2-yl)-1 ,3,5-triazin) und/oder TCB/TCP (1,3,5-Tris(N-carbazolyl)benzol/ 1,3,5-Tris(carbazol)-9-yl) benzol) umfassen.

Benachbart zur Elektronentransportschicht (ETL) kann sich eine Kathodenschicht C befinden. Beispielhaft kann die Kathodenschicht C ein Metall (z. B. Al, Au, Ag, Pt, Cu, Zn, Ni, Fe, Pb, LiF, Ca, Ba, Mg, In, W oder Pd) oder eine Metalllegierung umfassen oder daraus bestehen. Aus praktischen Gründen kann die Kathodenschicht außerdem aus (im Wesentlichen) intransparenten Metallen, wie etwa Mg, Ca oder Al, bestehen. Alternativ oder zusätzlich kann die Kathodenschicht C außerdem Graphit und/oder Kohlenstoffnanoröhren (CNTs) umfassen. Alternativ kann die Kathodenschicht C außerdem aus nanoskaligen Silberdrähten bestehen.

Eine OLED kann ferner optional eine Schutzschicht zwischen der Elektronentransportschicht (ETL) und der Kathodenschicht (die als Elektroneninjektionsschicht (EIL) bezeichnet werden kann) umfassen. Diese Schicht kann Lithiumfluorid, Cäsiumfluorid, Silber, Liq (8-Hydroxychinolinolatolithium), LizO, $BaF_2$, MgO und/oder NaF umfassen.

Optional können auch die Elektronentransportschicht (ETL) und/oder eine Lochblockierschicht (HBL) eine oder mehrere Hostverbindungen umfassen.

Um das Emissionsspektrum und/oder das Absorptionsspektrum der lichtemittierenden Schicht EML weiter zu modifizieren, kann die lichtemittierende Schicht EML ferner ein oder mehrere weitere Emittermoleküle F umfassen. Solch ein Emittermolekül F kann ein beliebiges fachbekanntes Emittermolekül sein. Vorzugsweise ist solch ein Emittermolekül F ein Molekül mit einer Struktur, die von der Struktur der erfindungsgemäßen Moleküle abweicht. Das Emittermolekül F kann optional ein TADF-Emitter sein. Alternativ kann das Emittermolekül F optional ein fluoreszierendes und/oder phosphoreszierendes Emittermolekül sein, das in der Lage ist, das Emissionsspektrum und/oder das Absorptionsspektrum der lichtemittierenden Schicht EML zu wechseln. Beispielhaft können Triplett- und/oder Singulettexzitonen von dem Emittermolekül gemäß der Erfindung zum Emittermolekül F übertragen werden, bevor es sich zum Ausgangszustand S0 entspannt, indem Licht typischerweise rotverschoben wird, und zwar im Vergleich zu dem Licht, das von dem Emittermolekül E emittiert wird. Optional kann das Emittermolekül F außerdem Zwei-Photonen-Wirkungen hervorrufen (d. h. die Absorption von zwei Photonen mit der halben Energie des Absorptionsmaximums).

Optional kann eine organische elektrolumineszierende Vorrichtung (z. B. eine OLED) beispielhaft eine im Wesentlichen weiße organische elektrolumineszierende Vorrichtung sein. Beispielhaft kann solch eine weiße organische elektrolumineszierende Vorrichtung mindestens ein (dunkel-) blaues Emittermolekül und ein oder mehrere Emittermoleküle, die grünes und/oder rotes Licht emittieren, umfassen. Dann kann außerdem optional wie vorstehend beschrieben eine Energieübertragung zwischen zwei oder mehreren Molekülen, erfolgen.

Wie hierin verwendet, wenn im bestimmten Zusammenhang nicht konkreter definiert, ist die Bezeichnung der Farben von emittiertem und/oder absorbiertem Licht wie folgt:

| | |
|---|---|
| violett: | Wellenlängenbereich von >380-420 nm; |
| dunkelblau: | Wellenlängenbereich von >420-480 nm; |
| himmelblau: | Wellenlängenbereich von >480-500 nm; |
| grün: | Wellenlängenbereich von >500-560 nm; |
| gelb: | Wellenlängenbereich von >560-580 nm; |
| orange: | Wellenlängenbereich von >580-620 nm; |
| rot: | Wellenlängenbereich von >620-800 nm. |

In Bezug auf Emittermoleküle beziehen sich solche Farben auf das Emissionsmaximum. Aus diesem Grund weist beispielhaft ein dunkelblauer Emitter ein Emissionsmaximum im Bereich von >420 bis 480 nm auf, ein himmelblauer Emitter weist ein Emissionsmaximum im Bereich von >480 bis 500 nm auf, ein grüner Emitter weist ein Emissionsmaximum in einem Bereich von >500 bis 560 nm auf, ein roter Emitter weist ein Emissionsmaximum in einem Bereich von >620 bis 800 nm auf.

Ein dunkelblauer Emitter kann vorzugsweise ein Emissionsmaximum von unter 480 nm, weiter bevorzugt unter 470 nm, noch weiter bevorzugt unter 465 nm oder sogar unter 460 nm aufweisen. Es beträgt typischerweise mehr als 420 nm, vorzugsweise mehr als 430 nm, weiter bevorzugt mehr als 440 nm oder sogar mehr als 450 nm.

Dementsprechend betrifft ein weiterer Aspekt der vorliegenden Erfindung eine OLED, die eine externe Quanteneffizienz bei 1000 cd/$m^2$ von mehr als 8 %, weiter bevorzugt von mehr als 10 %, weiter bevorzugt von mehr als 13 %, noch weiter bevorzugt von mehr als 15 % oder sogar mehr als 20 % aufweist und/oder ein Emissionsmaximum zwischen 420 nm und 500 nm, vorzugsweise zwischen 430 nm und 490 nm, weiter bevorzugt zwischen 440 nm und 480 nm, noch weiter bevorzugt zwischen 450 nm und 470 nm aufweist und/oder einen LT80-Wert bei 500 cd/$m^2$ von mehr als 100 h, vorzugsweise mehr als 200 h, weiter bevorzugt mehr als 400 h, noch weiter bevorzugt mehr als 750 h oder sogar mehr als 1000 h aufweist. Dementsprechend betrifft ein weiterer Aspekt der vorliegenden Erfindung eine OLED, deren Emission eine CIEy-Farbkoordinate von weniger als 0,45, vorzugsweise weniger als 0,30, weiter bevorzugt weniger als 0,20 oder noch weiter bevorzugt weniger als 0,15 oder sogar weniger als 0,10 aufweist.

Ein weiterer Aspekt der vorliegenden Erfindung betrifft eine OLED; die Licht bei einem unterschiedlichen Farbpunkt emittiert. Gemäß der vorliegenden Erfindung emittiert die OLED Licht mit einem schmalen Emissionsbereich (kleine Halbwertsbreite (FWHM)). In einem Aspekt emittiert die OLED gemäß der Erfindung Licht mit einer FWHM der Hauptemissionsspitze von weniger als 0,50 eV, vorzugsweise weniger als 0,48 eV, weiter bevorzugt weniger als 0,45 eV, noch weiter bevorzugt weniger als 0,43 eV oder weniger als 0,40 eV.

Ein weiterer Aspekt der vorliegenden Erfindung betrifft eine OLED, die Licht mit CIEx- und CIEy-Farbkoordinaten nahe den CIEx- (= 0,131) und CIEy- (= 0,046) -Farbkoordinaten der primären Farbe blau (CIEx = 0,131 und CIEy = 0,046) emittiert, wie durch ITU-R Recommendation BT.2020 (Rec. 2020) definiert, und ist somit zur Verwendung bei Ultra-High-Definition(UHD)-Displays, z. B. UHD-TVs, geeignet. In herkömmlichen Anwendungen werden typischerweise nach oben emittierende (obere Elektrode ist transparent) Vorrichtungen verwendet, wobei Testvorrichtung, wie sie in der gesamten Beschreibung verwendet werden, nach unten emittierende Vorrichtungen darstellen (untere Elektrode und Substrat sind transparent). Die CIEy-Farbkoordinate einer blauen Vorrichtung kann durch bis zu einem Faktor von zwei reduziert werden, wenn der Wechsel von einer nach unten zu einer nach oben emittierenden Vorrichtung erfolgt, während die CIEx nahezu unverändert bleibt (Okinaka et al. (2015), 22.1: Invited Paper: New Fluorescent Blue Host Materials for Achieving Low Voltage in OLEDs, SID Symposium Digest of Technical Papers, 46; doi:10.1002/sdtp.10480). Dementsprechend betrifft ein weiterer Aspekt der vorliegenden Erfindung eine OLED, deren Emission eine CIEx-Farbkoordinate von zwischen 0,02 und 0,30, vorzugsweise zwischen 0,03 und 0,25, weiter bevorzugt zwischen 0,05 und 0,20 oder weiter bevorzugt zwischen 0,08 und 0,18 oder sogar zwischen 0,10 und 0,15 und/oder eine CIEy-Farbkoordinate von zwischen 0,00 und 0,45, vorzugsweise zwischen 0,01 und 0,30, weiter bevorzugt zwischen 0,02 und 0,20 oder noch weiter bevorzugt zwischen 0,03 und 0,15 oder sogar zwischen 0,04 und 0,10 aufweist.

In einem weiteren Aspekt betrifft die Erfindung ein Verfahren zum Herstellen einer optoelektronischen Komponente. In diesem Fall wird ein organisches Molekül der Erfindung verwendet.

Die organische elektrolumineszierende Vorrichtung, insbesondere die OLED gemäß der vorliegenden Erfindung, kann mithilfe von Dampfabscheidung und/oder Flüssigkeitsbehandlung hergestellt werden. Dementsprechend wird mindestens eine Schicht

- mithilfe eines Sublimationsprozesses hergestellt,
- mithilfe eines organischen Dampfphasenabscheidungsprozesses hergestellt,
- mithilfe eines Trägergassublimationsprozesses hergestellt,
- mit Lösung verarbeitet oder gedruckt.

Die Verfahren, die zur Herstellung der organischen elektrolumineszierenden Vorrichtung, insbesondere der OLED gemäß der vorliegenden Erfindung, verwendet werden, sind im Fach bekannt. Die verschiedenen Schichten werden mithilfe eines nachfolgenden Abscheidungsprozesses einzeln und aufeinanderfolgend auf einem geeigneten Substrat abgeschieden. Die einzelnen Schichten können mithilfe der gleichen oder unterschiedlicher Abscheidungsverfahren abgeschieden werden.

Dampfabscheidungsprozesse umfassen beispielhaft thermische (Co-)Verdampfung, chemische Dampfabscheidung und physikalische Dampfabscheidung. Für eine aktive Matrix-OLED-Anzeige wird eine AMOLED-Rückwand als Substrat verwendet. Die einzelne Schicht kann aus Lösungen oder Dispersionen, die adäquate Lösungsmittel nutzen, verarbeitet werden. Der Lösungsabscheidungsprozess umfasst beispielhaft Rotationsbeschichten, Tauchbeschichten und Spritzdrucken. Flüssigkeitsbehandlung kann optional in einer inerten Atmosphäre (z. B. in einer Stickstoffatmosphäre) durchgeführt werden und das Lösungsmittel kann optional vollständig oder teilweise unter Verwendung von im Stand der Technik bekannten Mitteln entfernt werden.

**Beispiele**

Allgemeines Syntheseschema I

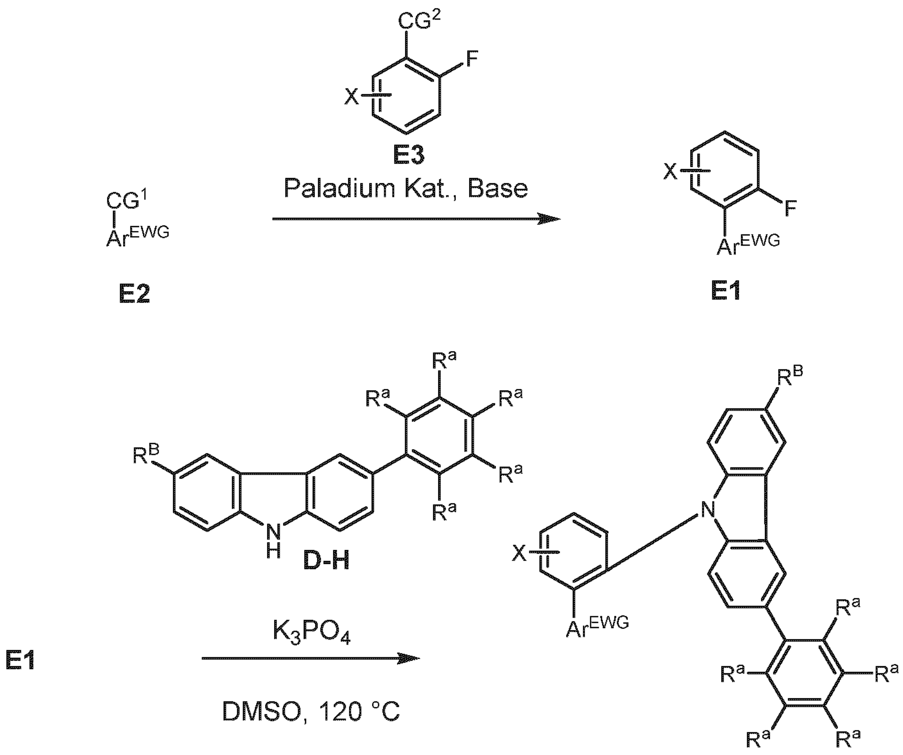


*Allgemeiner Vorgang für die Synthese AA V0:*

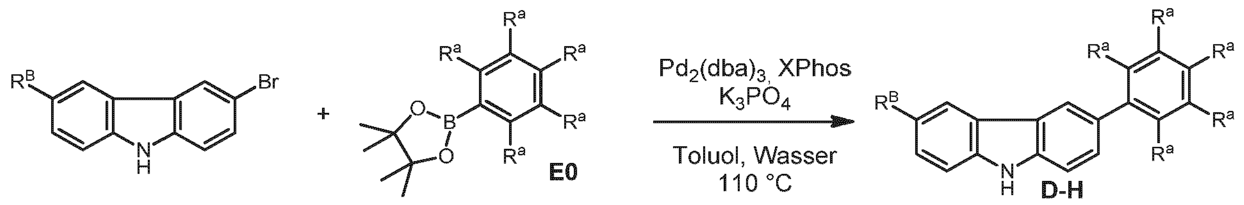


E0 (1,20 Äquivalente), 6-$R^B$-substituiertes 3-Bromcarbazol (1,00 Äquivalente), $Pd_2(dba)_3$ (0,03 Äquivalente), 2-Dicyclohexylphosphino-2;4',6triisopropylbiphenyl (XPhos) (0,12 Äquivalente) und tribasisches Kaliumphosphat (1,50 Äquivalente) werden unter Stickstoffatmosphäre in Toluol-/Wassergemisch (Verhältnis von 10:1, 3 ml Toluol pro mmol Arylbromid) bei 110 °C 16 Stunden lang gerührt. Anschließend wird das Reaktionsgemisch filtriert und der Rückstand mit Dichlormethan gewaschen. Das Lösungsmittel wird entfernt. Das erhaltene Rohprodukt wird durch Umkristallisation oder Säulenchromatographie aufgereinigt und als Feststoff erhalten. Anstelle eines Boronsäureesters kann eine entsprechende Boronsäure verwendet werden.

Allgemeines Syntheseschema II

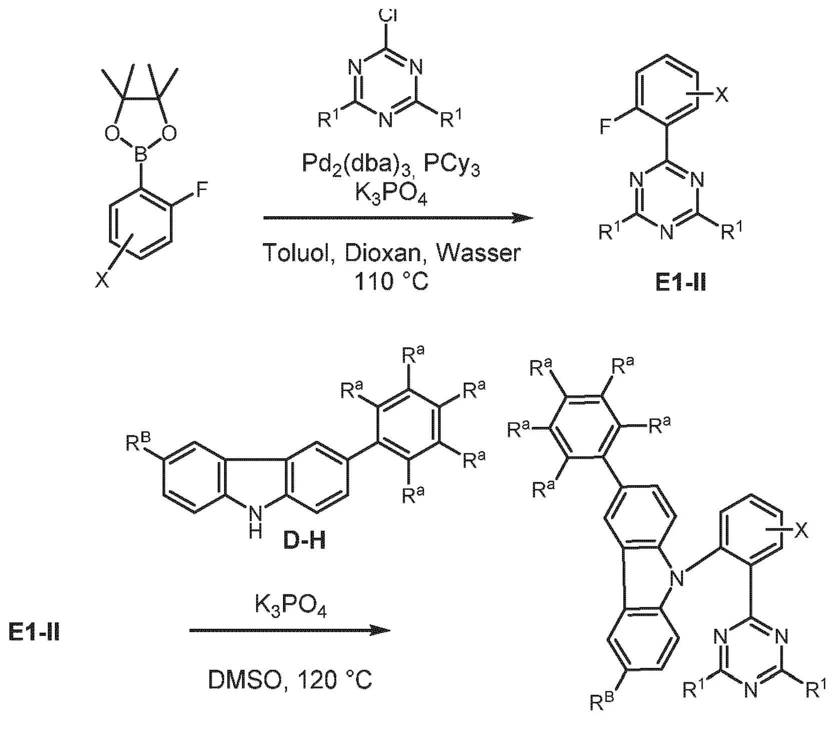


*Allgemeiner Vorgang für die Synthese* ***AA V1:***

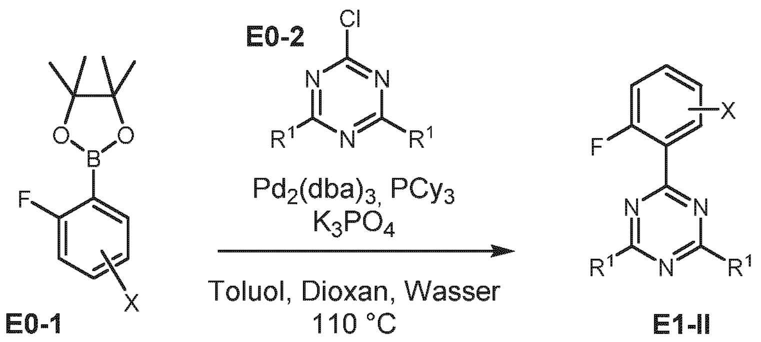


X-substituiertes Fluorophenylboronsäurepinakolester **E0-1** (1,20 Äquivalente), 2-,4-di-$R^1$-substituierts-6-Chloro-1,3,5-Triazin **E0-2** (1,00 Äquivalente), $Pd_2(dba)_3$ (0,03 Äquivalente), Tricyclohexylphosphin (PCys) (0,07 Äquivalente) und tribasisches Kaliumphosphat (2,50 Äquivalente) werden unter Stickstoffatmosphäre in Dioxan-/Toluol-/Wassergemisch (Verhältnis von 4:1:1, 4 ml Dioxan pro mmol Triazin) bei 110 °C 16 Stunden lang gerührt. Anschließend wird das Reaktionsgemisch filtriert und der Rückstand mit Dichlormethan gewaschen. Das Lösungsmittel wird entfernt. Das erhaltene Rohprodukt wird durch Umkristallisation oder Säulenchromatographie gereinigt und das Produkt **E1-II** wird als Feststoff erhalten.

Anstelle eines Boronsäureesters kann eine entsprechende Boronsäure verwendet werden.

*Allgemeiner Vorgang für die Synthese **AA V2:***

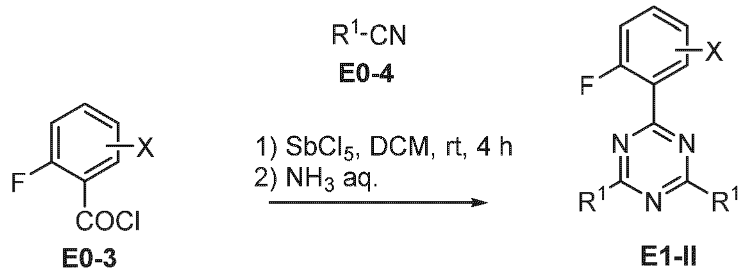


E0-3 (1,00 Äquivalente) und **E0-4** (2,00 Äquivalente) werden in Dichlormethan gelöst und in einem Eisbad gekühlt. Antimon(V)-chloride (1,00 Äquivalente) wird tropfenweise zur Lösung hinzugegeben, die Reaktionsmischung für eine Stunde bei Raumtemperatur gerührt und anschließend bei 45 °C für 6 Stunden gerührt. Das Zwischenprodukt wird gefiltert und mit Dichlormethan gewaschen.

Der getrocknete Feststoff wird zu einer gekühlten 25%-igen Ammoniaklösung (0 - 5 °C) gegeben und über Nacht bei Raumtemperatur gerührt. Die Reaktionsmischung filtriert und der gewonnene Feststoff mit Wasser gewaschen. Der Feststoff wird zu DMF gegeben und bei 155 °C für 30 Minuten gerührt. Der ausgefallene Feststoff wird heiß Filtriert. Wasser wird zur heißen DMF-Lösung gegeben um das Produkt auszufällen. Das Produkt **E1-II** wird durch Filtration erhalten.

*Allgemeiner Vorgang für die Synthese **AA V3:***

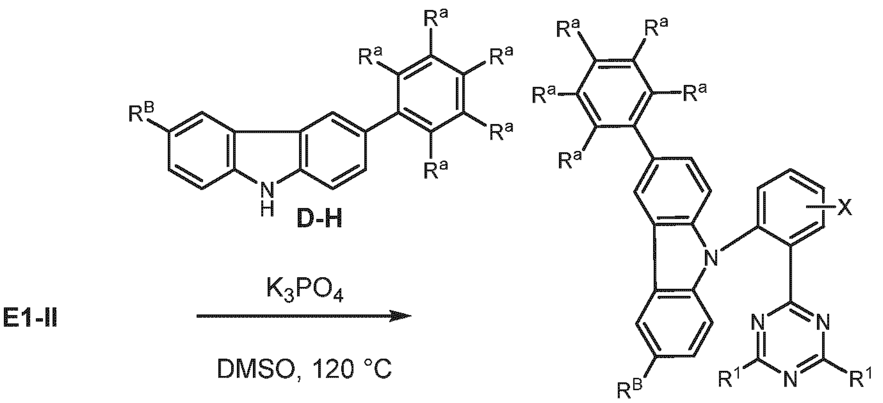


**E1-II** (1,00 Äquivalente), das entsprechende Donormolekül **D-H** (1,00 Äquivalente) und tribasisches Kaliumphosphat (4,00 Äquivalente) werden unter Stickstoffatmosphäre in DMSO suspendiert und bei 120 °C (16 h) gerührt. Anschließend wird das Reaktionsgemisch in eine gesättigte Natriumchloridlösung gegeben und drei Mal mit Dichlormethan extrahiert. Die kombinierten organischen Phasen werden zwei Mal mit gesättigter Natriumchloridlösung gewaschen, über $MgSO_4$ getrocknet und das Lösungsmittel wird entfernt. Das Rohprodukt wird durch Umkristallisation oder durch Flash-Chromatographie gereinigt. Das Produkt wird als Feststoff erhalten.

Alternativ kann in ***AA V3*** beispielsweise halogensubstituiertes Carbazol D-H-Hal, insbesondere ein 3-Brom substituiertes Carbazol, zum Beispiel 3-Bromcarbazol, anstelle von D-H verwendet werden.

In einer Folgereaktion kann eine Boronsäureester-Gruppe oder eine Boronsäure-Gruppe beispielhaft an der Position der einen oder mehreren Halogensubstituenten, die via D-H-Hal eingeführt wurden, eingeführt werden und so den entsprechenden Carbazol-3-ylboronsäureester oder die entsprechende Carbazol-3-boronsäure ergeben, beispielsweise durch die Reaktion mit Bis(pinacol)diboronsäureester (CAS-Nr. 73183-34-3). Anschließend kann ein Substituent

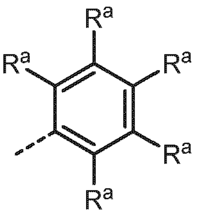
,

wobei die gestrichelte Linie die Bindungsstelle darstellt, anstelle der Boronsäureester-Gruppe oder der Boronsäure-Gruppe über eine Kupplungsrektion mit dem entsprechenden halogenierten Reaktant, vorzugsweise

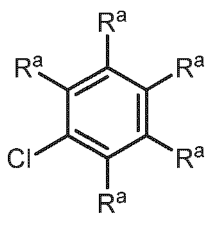
und
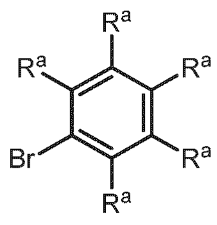
,

eingeführt werden. Alternativ kann ein Substituent

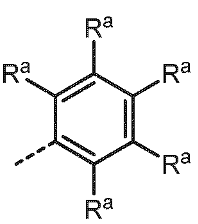

an der Position des einen Halogensubstituenten, der über **D-H-Hal** eingeführt wurde, über die Reaktion mit einer Boronsäure des Substituenten

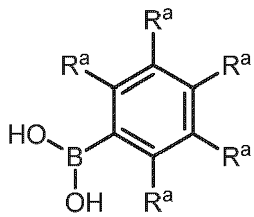

oder einen entsprechenden Boronsäureester eingeführt werden.

Alternativ lässt sich die Bromierung eines Carbozolderivates in einer Reaktion mit N-Bromosuccinimid herstellen wie beispielsweise im folgenden Reaktionsschema dargestellt ist:

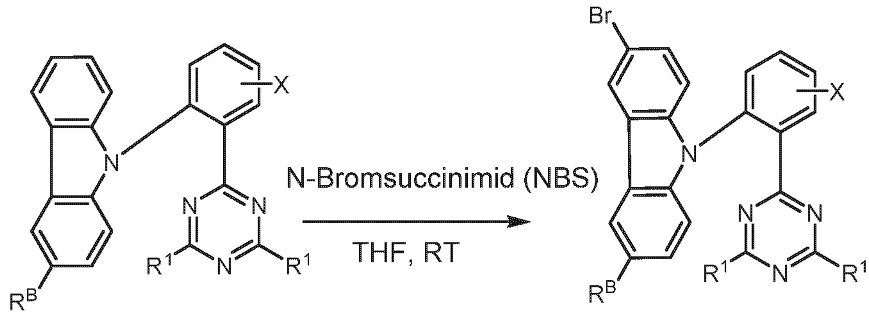


**HPLC-MS:**

HPLC-MS-Spektroskopie wird auf einem HPLC durch Agilent (1100er Serie) mit MS-Detektor (Thermo LTQ XL) durchgeführt.

Beispielsweise wird eine Umkehrphasensäule 4,6 mm x 150 mm, Partikelgröße 3,5 μm von Agilent (ZORBAX Eclipse Plus 95Ä C18, 4,6 x 150 mm, 3.5 μm HPLC column) im HPLC verwendet. Die HPLC-MS-Messungen werden bei Raumtemperatur (RT) mit folgenden Gradienten durchgeführt:

**Durchflussrate**

| [ml/min] | Zeit [min] | A[%] | B[%] | C[%] |
|---|---|---|---|---|
| 2,5 | 0 | 40 | 50 | 10 |
| 2,5 | 5 | 40 | 50 | 10 |
| 2,5 | 25 | 10 | 20 | 70 |
| 2,5 | 35 | 10 | 20 | 70 |

wobei folgende Lösungsmittelgemische verwendet werden:

| | | |
|---|---|---|
| Lösungsmittel A: | $H_2O$ (90 %) | MeCN (10 %) |
| Lösungsmittel B: | $H_2O$ (10 %) | MeCN (90 %) |
| Lösungsmittel C: | THF (50 %) | MeCN (50 %) |

Für die Messungen wird ein Injektionsvolumen von 5 μl aus einer Lösung mit einer Konzentration von 0,5 mg/ml entnommen.

Die Ionisierung der Probe wird durch APCI (chemische Ionisation bei Atmosphärendruck) entweder im positiven (APCI +) oder negativen (APCI -) Ionisierungsmodus durchgeführt.

*Cyclische Voltammetrie*

Cyclische Voltammogramme werden anhand von Lösungen mit einer Konzentration von $10^{-3}$ mol/l der organischen Moleküle in Dichlormethan oder eines geeigneten Lösungsmittels und eines geeigneten Trägerelektrolyts (z. B. 0,1 mmol/l von Tetrabutylammoniumhexafluorphosphat) gemessen. Die Messungen werden bei Raumtemperatur unter Stickstoffatmosphäre mit einer Drei-Elektroden-Baugruppe durchgeführt (Arbeits- und Gegenelektroden: Pt-Draht, Referenzelektrode: Pt-Draht) und mithilfe von $FeCp_2/FeCp_2^+$ als interner Standard kalibriert. Die HOMO-Daten wurden mithilfe von Ferrocen als interner Standard gegenüber einer gesättigten Kalomel-Elektrode (SCE) korrigiert.

*Berechnung auf Grundlage der Dichtefunktionaltheorie*

Molekulare Strukturen werden unter Verwendung der BP68-Funktion und des Identitätsauflösungsansatzes (RI) optimiert. Anregungsenergien werden mithilfe der (BP68-) optimierten Strukturen unter Verwendung von zeitabhängigen DFT- (TD-DFT-) Strukturen berechnet. Orbitale und angeregte Zustandsenergien werden mit der B2LYP-Funktion berechnet. Def2-SVP-Ausgangssätze und ein m4-Raster zur numerischen Integration werden verwendet. Das Turbomole-Programmpaket wird für alle Berechnungen verwendet.

*Photophysikalische Messungen*

Probenvorbehandlung: Rotationsbeschichtung
Vorrichtung: Spin150, SPS euro.

Die Probenkonzentration beträgt 1 mg/ml, angesetzt in Chloroform oder Dichlormethan. Zu 1 ml der entsprechenden Lösung werden 9 mg PMMA hinzugefügt. Mit 50 µl der resultierenden Lösung wird der entsprechende Dünnfilm hergestellt.

Programm: 1) 3 s bei 400 U/min; 2) 20 s bei 1000 U/min bei 1000 Upm/ s. 3) 10 s bei 4000 U/min bei 1000 Upm/s. Die Filme wurden nach dem Beschichten für 1 min bei 70 °C an Luft auf einer Präzisionsheizplatte von LHG getrocknet.

Photolumineszenz-Spektroskopie und TCSPC (*Zeitkorrelierte Einzelphotonenzählung*) Stationäre Emissionsspektroskope wird mit einem Fluoreszenzspektrometer der Horiba Scientific, Modell FluoroMax-4 durchgeführt, ausgestattet mit einer 150 W Xenon-Arc Lampe, Anregungs- und Emissionsmonochromatoren und einer Hamamatsu R928 Photomultiplier-Röhre, sowie einer "zeit-korrelierten Einphotonzähl" (*Time-correlated single-photon counting,* TCSPC)-Option. Hierbei wurde die Probenkammer kontinuierlich mit Stickstoff gespült (Durchflussrate > 800 ml/ min) Emissions- und Anregungsspektren wurden korrigiert durch Standardkorrekturkurven.

Emissionsabklingzeiten werden unter Verwendung des gleichen Systems mithilfe des TCSPC-Verfahrens mit FM-2013-Ausrüstung und einem Horiba Yvon TCSPC Hub bestimmt.

Anregungsquellen:

NanoLED 370 (Wellenlänge: 371 nm, Impulsdauer: 1,1 ns)
NanoLED 290 (Wellenlänge: 294 nm, Impulsdauer: <1 ns)
SpectraLED 310 (Wellenlänge: 314 nm)
SpectraLED 355 (Wellenlänge: 355 nm).

Die Auswertung (exponentielles Fitten) erfolgte mit dem Softwarepaket DataStation und der DAS 6 Auswertungssoftware. Der Fit wurde über die Chi-Quadrat-Methode angegeben

$$c^2 = \sum_{k=1}^{i} \frac{(e_i - o_i)^2}{e_i}$$

mit $e_i$: Durch den Fit vorhergesagte Größe und $o_i$: gemessenen Größe.

Zeitaufgelöste PL Spektroskopie im µs-Bereich

Zeitaufgelöste Transienten werden darüber hinaus an einem FS5 Fluoreszenzspektrometer von Edinburgh Instruments vermessen. im Vergleich zu Messungen an dem vergleichbaren HORIBA System, ermöglicht das FS5 eine

erhöhte Lichtausbeute und somit ein verbessertes Verhältnis zwischen Probenemission und Hintergrundrauschen, welches besonders Messungen verzögert fluoreszierender Emitter verbessert. Hierbei wird die zu untersuchende Probe über eine breitbandige Xenon-Lampe angeregt (150 W Xenon arc lamp). Das FS5 nutzt Czerny-Turner Monochromatoren sowohl für selektive Anregungs- als auch Emissionswellenlängen. Die Photolumineszenz der Probe wird über einen R928P Photomultiplier-Röhre detektiert, wobei die Photokathode des Detektors die zeitaufgelöste Messung im spektralen Bereich von 200 nm bis 870 nm ermöglicht. Die temperaturstabilisierte Detektoreinheit gewährleistet darüber hinaus eine Dunkelzählrate unter 300 Ereignissen pro Sekunde. Zur Bestimmung der Abklingzeit des PL Transienten wird anschließend der μs- Bereich mit Hilfe von drei Exponentialfunktionen angenähert. Über Amplitudengewichtung ergibt sich eine gemittelte Lebensdauer $\tau_{DF}$ für die verzögerte Fluoreszenz von

$$\tau_{\mathrm{DF}} = \sum_{i=1}^{3} \frac{A_i \tau_i}{A_i},$$

mit den jeweiligen monoexponentiellen Abklingzeiten $\tau_i$ und zugehörigen Amplituden $A_i$.

Messungen der Photolumineszenzquantenausbeute

Die Messung der Photolumineszenzquantenausbeute (PLQY) erfolgte mittels eines Absolute PL Quantum Yield Measurement C9920-03G-Systems der Hamamatsu Photonics. Dieses besteht aus einer 150 W Xenon-Gasentladungslampe, automatisch justierbaren Czerny-Turner Monochromatoren (250 - 950 nm) und einer Ulbricht-Kugel mit hochreflektierender Spektralon-Beschichtung (einem Teflon-Derivat), die über ein Glasfaserkabel mit einem PMA-12 Vielkanaldetektor mit BT- (back thinned-) CCD-Chip mit 1024 x 122 Pixeln (Größe 24 x 24 μm) verbunden ist. Die Auswertung der Quanteneffizienz und der CIE-Koordinaten erfolgte mit Hilfe der Software U6039-05 Version 3.6.0.Emissionsmaxima werden in nm angegeben, Quantenausbeuten Φ in % und CIE-Koordinaten als x-, y-Werte.

PLQY wird mithilfe des folgenden Protokolls bestimmt:

1) Durchführung der Qualitätssicherung: Als Referenzmaterial dient Anthracene in Ethanol mit bekannter Konzentration.
2) Ermitteln der Anregungswellenlänge: Es wurde zuerst das Absorbtionsmaximum des organischen Moleküls bestimmt und mit diesem angeregt.
3) Durchführung der Probenmessung:
Es wurde von entgasten Lösungen und Filmen unter Stickstoff-Atmosphäre die absolute Quantenausbeute bestimmt.

Die Berechnung erfolgte systemintern nach folgender Gleichung:

$$\Phi_{PL} = \frac{n_{photon}, emittiert}{n_{photon}, absorbed} = \frac{\int \frac{\lambda}{hc}\left[Int_{emittiert}^{Probe}(\lambda) - Int_{emittiert}^{Referenz}(\lambda)\right]d\lambda}{\int \frac{\lambda}{hc}\left[Int_{emittiert}^{Referenz}(\lambda) - Int_{absorbed}^{Probe}(\lambda)\right]d\lambda}$$

wobei $n_{photon}$ die Photonenzählung und Int. die Intensität bezeichnet.

**Herstellung und Charakterisierung von organischen elektrolumineszierenden Vorrichtungen**

OLED-Vorrichtung, die erfindungsgemäße organische Moleküle umfassen, können unter Verwendung von Vakuumabscheidungsverfahren hergestellt werden. Wenn eine Schicht mehr als eine Verbindung enthält, wird der Gewichtsprozentsatz von einer oder mehreren Verbindungen in % angegeben. Die Gesamtgewichtsprozentsatzwerte betragen 100 %, wenn also ein Wert nicht angegeben ist, gleicht der Anteil dieser Verbindung der Differenz zwischen den angegebenen Werten und 100 %.

Die nicht vollständig optimierten OLEDs werden mithilfe von Standardverfahren und des Messens von Elektrolumineszenzspektren, der externen Quanteneffizienz (in %) in Abhängigkeit von der Intensität, charakterisiert, unter Verwendung des durch die Photodiode detektierten Lichts und des Stroms berechnet. Die Lebensdauer der OLED-Vorrichtung wird von der Veränderung der Leuchtdichte während des Betriebs bei konstanter Stromdichte extrahiert. Der LT50-Wert entspricht der Zeit, bei der die gemessene Luminanz auf 50 % der anfänglichen Leuchtdichte gesunken ist, analog entspricht LT80 dem Zeitpunkt, bei dem die gemessene Leuchtdichte auf 80 % der anfänglichen Leuchtdichte gesunken ist, LT95 dem Zeitpunkt, bei dem die gemessene Leuchtdichte auf 95 % der anfänglichen Leuchtdichte gesunken ist, usw.

Beschleunigte Messungen der Lebensdauer werden durchgeführt (z. B. Anwenden von erhöhten Stromdichten). Beispielhaft werden LT80-Werte bei 500 cd/m$^2$ mithilfe der folgenden Gleichung bestimmt:

$$\mathrm{LT80}\left(500\,\frac{cd^2}{m^2}\right) = \mathrm{LT80}(L_0)\left(\frac{L_0}{500\,\frac{cd^2}{m^2}}\right)^{1.6}$$

wobei $L_0$ die anfängliche Leuchtdichte bei der angelegten Stromdichte kennzeichnet.

Der Wert entspricht dem Durchschnitt von mehreren Pixeln (typischerweise zwei bis acht), wobei die Standardabweichung zwischen diesen Pixeln gegeben ist. Die Figuren zeigen die Datenserien für einen OLED-Pixel.

Beispiel 1

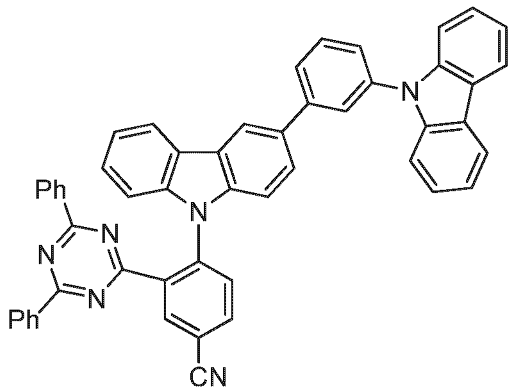

Beispiel 1 wurde gemäß

***AAVO*** (74 % Ausbeute), wobei 3-Bromcarbazol (CAS 1592-95-6) und

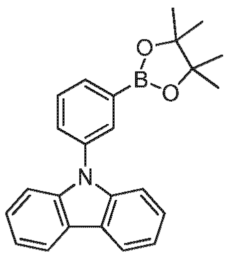

als E0 verwendet wurde,

***AAV1*** (83 % Ausbeute), wobei 2-Chlor-4,6-diphenyl-1,3,5-triazine (CAS 3842-55-5) als E0-2 und 2-Fluoro-5-Cyanophenylboronsäurepinakolester als **E0-1** verwendet wurde und

***AA V3*** (68% Ausbeute) synthetisiert.

MS (HPLC-MS), m/z (Retentionszeit): 740,24 (11,71 min).

Figur 1 stellt das Emissionsspektrum von Beispiel **1** dar (10 Gew.-% in PMMA). Das Emissionsmaximum liegt bei 483 nm. Die Photolumineszenquantenausbeute (PLQY) beträgt 82%, die Halbwertsbreite beträgt 0,43 eV und die Emissionsabklingzeit beträgt 10 µs.

**Beispiel 2**

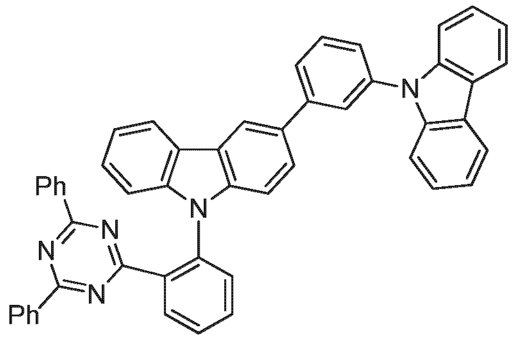

Beispiel **2** wurde gemäß ***AAVO*** (74 % Ausbeute), wobei 3-Bromcarbazol (CAS 1592-95-6) und

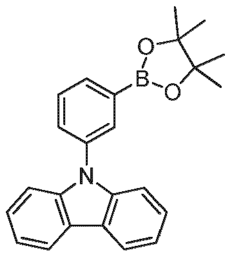

als E0 verwendet wurde,

***AA V2*** (99 % Ausbeute), wobei Benzonitril (CAS 100-47-0) als **E0-4** und 2-Fluorobenzoylchlord als E0-3 verwendet wurde und ***AA V3*** (50% Ausbeute) synthetisiert.

MS (HPLC-MS), m/z (Retentionszeit): 714,23 (12,75 min).

Figur 2 stellt das Emissionsspektrum von Beispiel **2** dar (10 Gew.-% in PMMA). Das Emissionsmaximum liegt bei 467 nm. Die Photolumineszenquantenausbeute (PLQY) beträgt 75%, die Halbwertsbreite beträgt 0,43 eV und die Emissionsabklingzeit beträgt 19 µs.

**Beispiel 3**

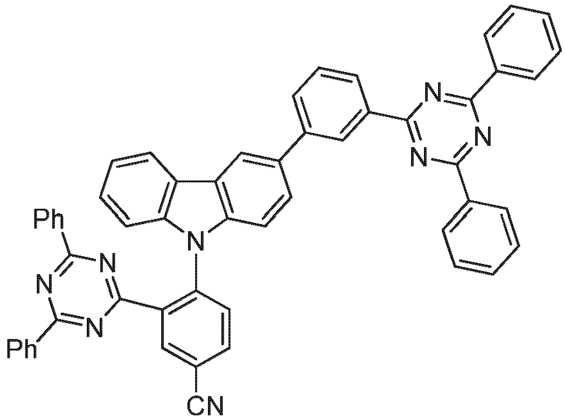

Beispiel **3** wurde gemäß

***AA VO*** (27 % Ausbeute), wobei 3-Bromcarbazol (CAS 1592-95-6) und

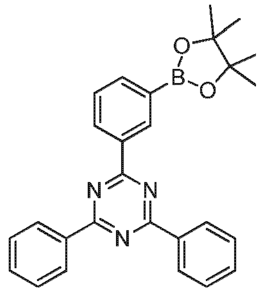

als **E0** verwendet wurde,

***AAV1*** (83 % Ausbeute), wobei 2-Chlor-4,6-diphenyl-1,3,5-triazine (CAS 3842-55-5) als **E0-2** und 2-Fluoro-5-Cyanophenylboronsäurepinakolester als **E0-1** verwendet wurde und ***AA V3*** (65% Ausbeute) synthetisiert.

MS (HPLC-MS), m/z (Retentionszeit): 806,24 (12,43 min).

Figur 3 stellt das Emissionsspektrum von Beispiel 3 dar (10 Gew.-% in PMMA). Das Emissionsmaximum liegt bei 490 nm. Die Photolumineszenquantenausbeute (PLQY) beträgt 74% und die Halbwertsbreite beträgt 0,44 eV.

**Beispiel 4**

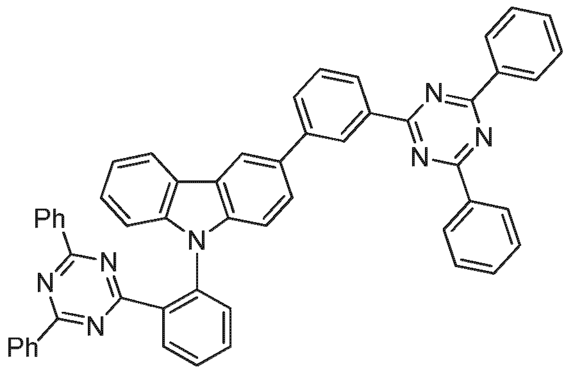

Beispiel 4 wurde gemäß

***AA VO*** (27 % Ausbeute), wobei 3-Bromcarbazol (CAS 1592-95-6) und

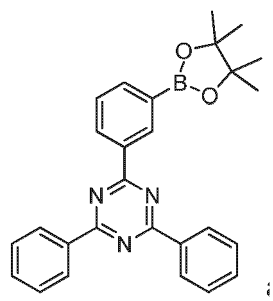

als **E0** verwendet wurde,

***AA V2*** (99 % Ausbeute), wobei Benzonitril (CAS 100-47-0) als E0-4 und 2-Fluorobenzoylchlord als E0-3 verwendet wurde und

***AA V3*** (74% Ausbeute) synthetisiert.

MS (HPLC-MS), m/z (Retentionszeit): 780,19 (17,13 min).

Figur 4 stellt das Emissionsspektrum von Beispiel 4 dar (10 Gew.-% in PMMA). Das Emissionsmaximum liegt bei 473 nm. Die Photolumineszenquantenausbeute (PLQY) beträgt 62%, die Halbwertsbreite beträgt 0,45 eV und die Emissionsabklingzeit beträgt 43 µs.

**Beispiel 5**

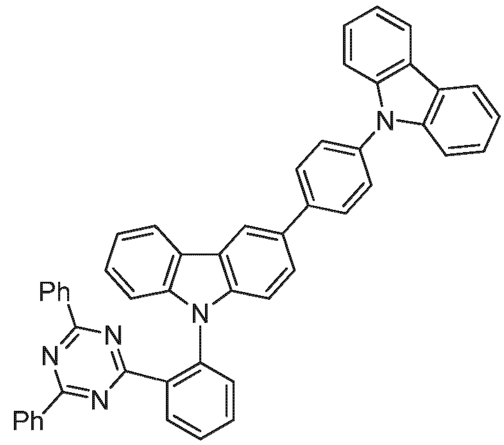

Beispiel 5 wurde gemäß

***AAV0*** (62% Ausbeute), wobei 3-Bromcarbazol (CAS 1592-95-6) und

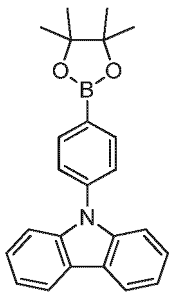

als **E0** verwendet wurde,
***AA V2*** (99 % Ausbeute), wobei Benzonitril (CAS 100-47-0) als **E0-4** und 2-Fluorobenzoylchlord als **E0-3** verwendet wurde und ***AA V3*** (31% Ausbeute) synthetisiert.

MS (HPLC-MS), m/z (Retentionszeit): 716,36 (15,17 min).
Figur 5 stellt das Emissionsspektrum von Beispiel 5 dar (10 Gew.-% in PMMA). Das Emissionsmaximum liegt bei 473 nm. Die Photolumineszenquantenausbeute (PLQY) beträgt 77%, die Halbwertsbreite beträgt 0,44 eV und die Emissionsabklingzeit beträgt 60 µs.

**Beispiel 6**

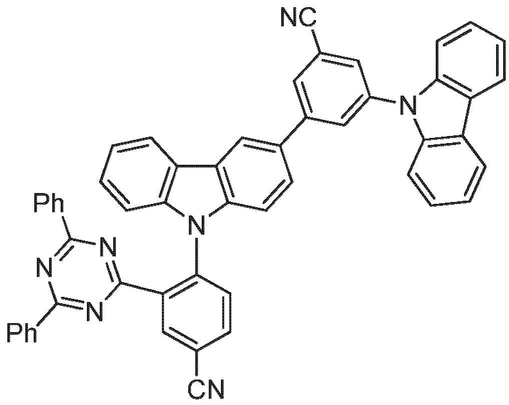

Beispiel 6 wurde gemäß

***AAV1*** (83 % Ausbeute), wobei 2-Chlor-4,6-diphenyl-1,3,5-triazine (CAS 3842-55-5) als **E0-2** und 2-Fluoro-5-Cyanophenylboronsäurepinakolester als **E0-1** verwendet wurde und analog zu ***AAV3*** (81 % Ausbeute), wobei 3-Bromocarbazol (CAS 1592-95-6) als D-H verwendet wurde. Das Produkt aus der Reaktion gemäß ***AA V3*:**

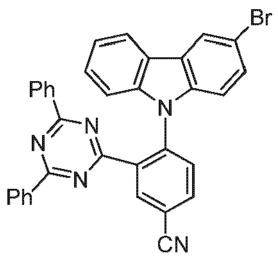

wird im Anschluss mit Bis(pinacolato)diboron wie folgt umgesetzt:

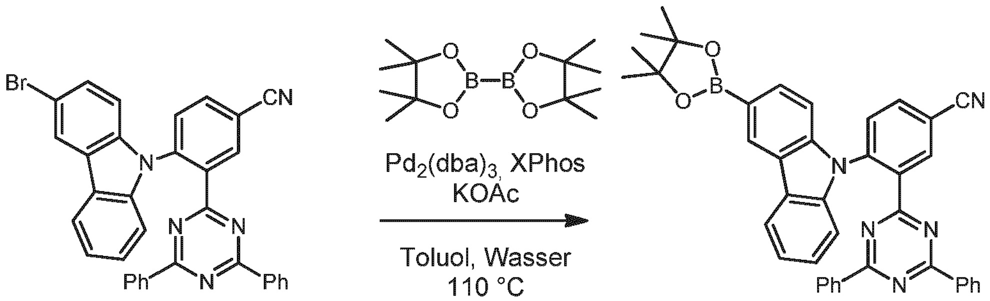


und anschließend mit

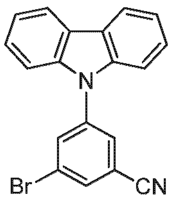

gemäß

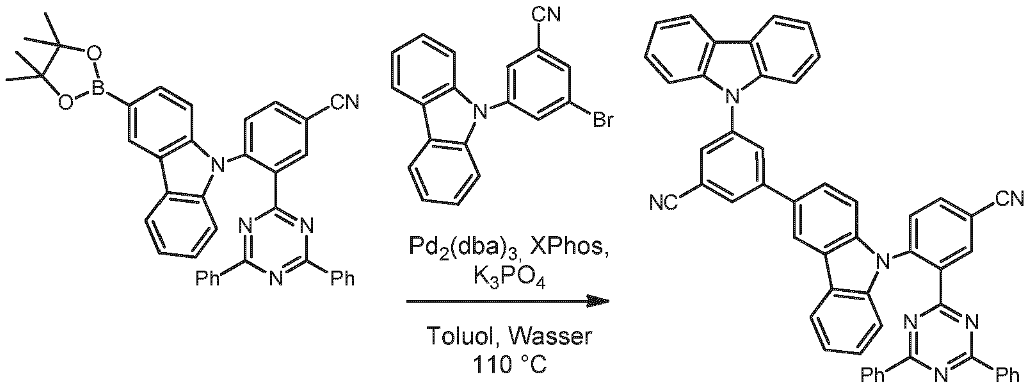


umgesetzt.

MS (HPLC-MS), m/z (Retentionszeit): 765,22 (11,07 min).

Figur 6 stellt das Emissionsspektrum von Beispiel 6 dar (10 Gew.-% in PMMA). Das Emissionsmaximum liegt bei 473 nm. Die Photolumineszenquantenausbeute (PLQY) beträgt 64%, die Halbwertsbreite beträgt 0,43 eV und die Emissionsabklingzeit beträgt 15 µs.

Beispiel 7

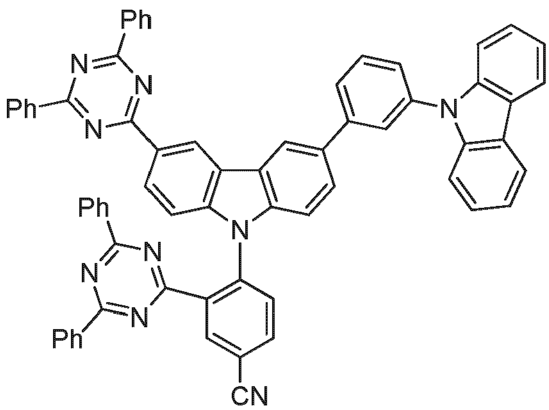

Beispiel 7 wurde gemäß ***AA V2*** (99 % Ausbeute), wobei Benzonitril (CAS 100-47-0) als E0-4 und 2-Fluorobenzoylchlord als **E0-3** verwendet wurde, analog zu ***AA V3*** (81% Ausbeute), wobei 3-Bromocarbazol (CAS 1592-95-6) als D-H verwendet wurde. Das Produkt aus der Reaktion gemäß

***AAV3:***

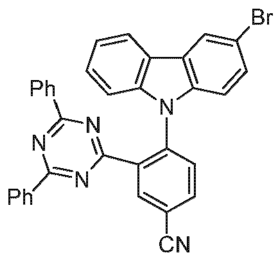

wurde im Anschluss mit Bis(pinacolato)diboron wie folgt umgesetzt:

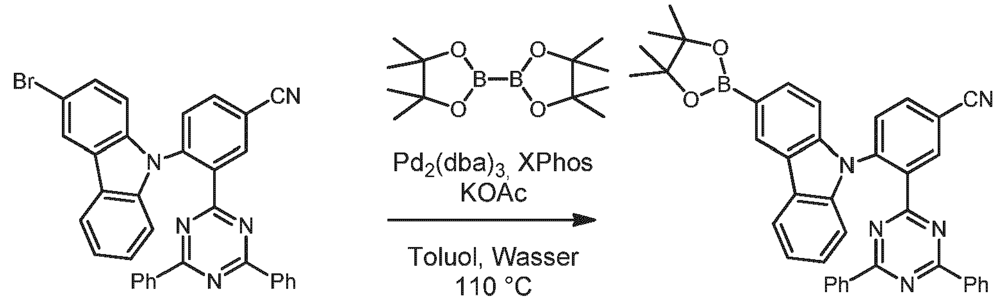


und anschließend mit 2-Chlor-4,6-diphenyl-1,3,5-triazine (CAS 3842-55-5) gemäß

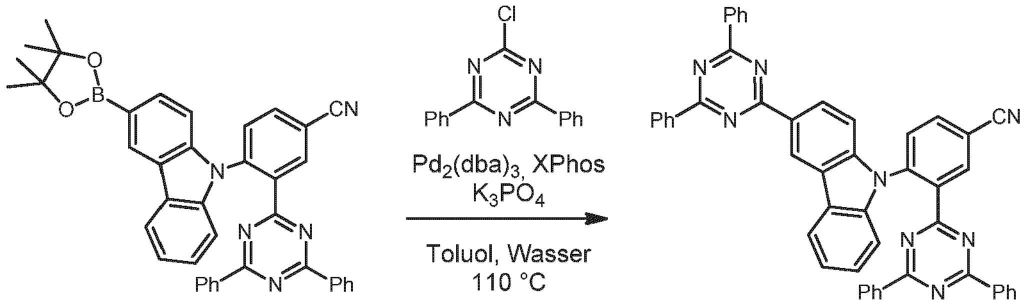


umgesetzt, welches im Anschluss mit N-Bromsuccinimid (CAS 128-08-5) gemäß

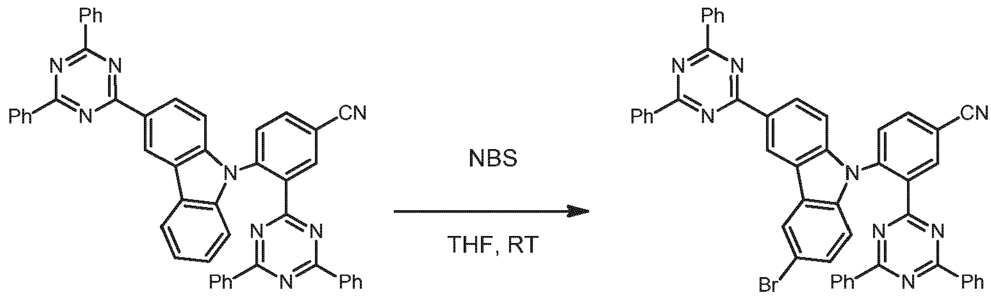


umgesetzt wurde (83 % Ausbeute). Das Produkt aus der Reaktion wurde gemäß

***AAV0*** (68 % Ausbeute), wobei

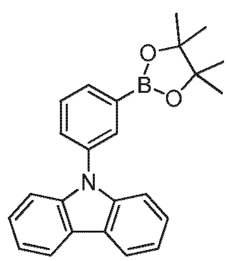

als E0 verwendet wurde, umgesetzt.

MS (HPLC-MS), m/z (Retentionszeit): 971,17 (15,43 min).

Figur 7 stellt das Emissionsspektrum von Beispiel 7 dar (10 Gew.-% in PMMA). Das Emissionsmaximum liegt bei 481 nm. Die Photolumineszenzquantenausbeute (PLQY) beträgt 69%, die Halbwertsbreite beträgt 0,43 eV und die Emissionsabklingzeit beträgt 12 μs.

**Beispiel 8**

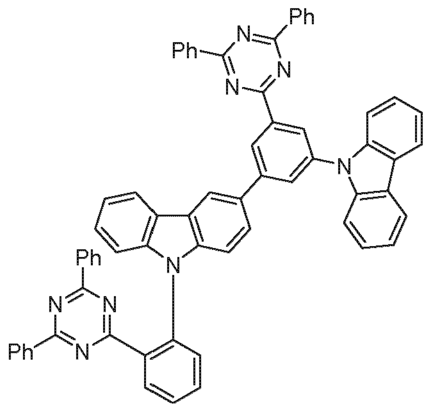

Das Emissionsmaximum von Beispiel 8 (10 Gew.-% in PMMA) liegt bei 466 nm.

**Beispiel** 9

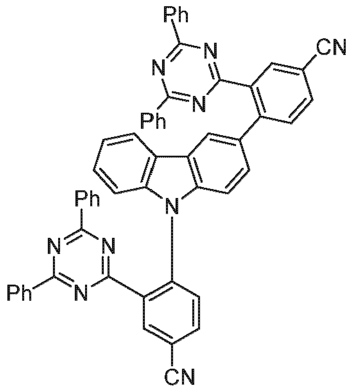

Das Emissionsmaximum von Beispiel 9 (10 Gew.-% in PMMA) liegt bei 480 nm.

**Beispiele D1**

Beispiel 1 wurde in den OLED-Bauteilen D1 mit folgendem Aufbau getestet (der Anteil des erfindungsgemäßen Moleküls an der Emissionsschicht ist in Massenprozent angegeben):

| **Schicht** | **Dicke** | **D1** |
|---|---|---|
| **10** | 100 nm | Al |
| **9** | 2 nm | Liq |
| **8** | 20 nm | NBPhen |
| **7** | 10 nm | **MAT1** |
| **6** | 50 nm | mCBP (70%) : **MAT1**(20%) : **1** (10%) |
| **5** | 10 nm | mCBP |

(fortgesetzt)

| Schicht | Dicke | D1 |
|---|---|---|
| **4** | 10 nm | TCTA |
| **3** | 40 nm | NPB |
| **2** | 5 nm | HAT-CN |
| **1** | 50 nm | ITO |
| **Substrat** | | Glas |

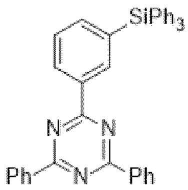

**MAT1**

Für das Bauteil D1 wurde eine externe Quanteneffizienz bei 1000 cd/$m^2$ von 19,2% ± 0,7 bestimmt. Das Emissionsmaximum liegt bei 487 nm, CIEx wurde mit 0,32 und die CIEy: 0,17 bei 5,9 V bestimmt.

Weitere Beispiele erfindungsgemäßer organischer Moleküle

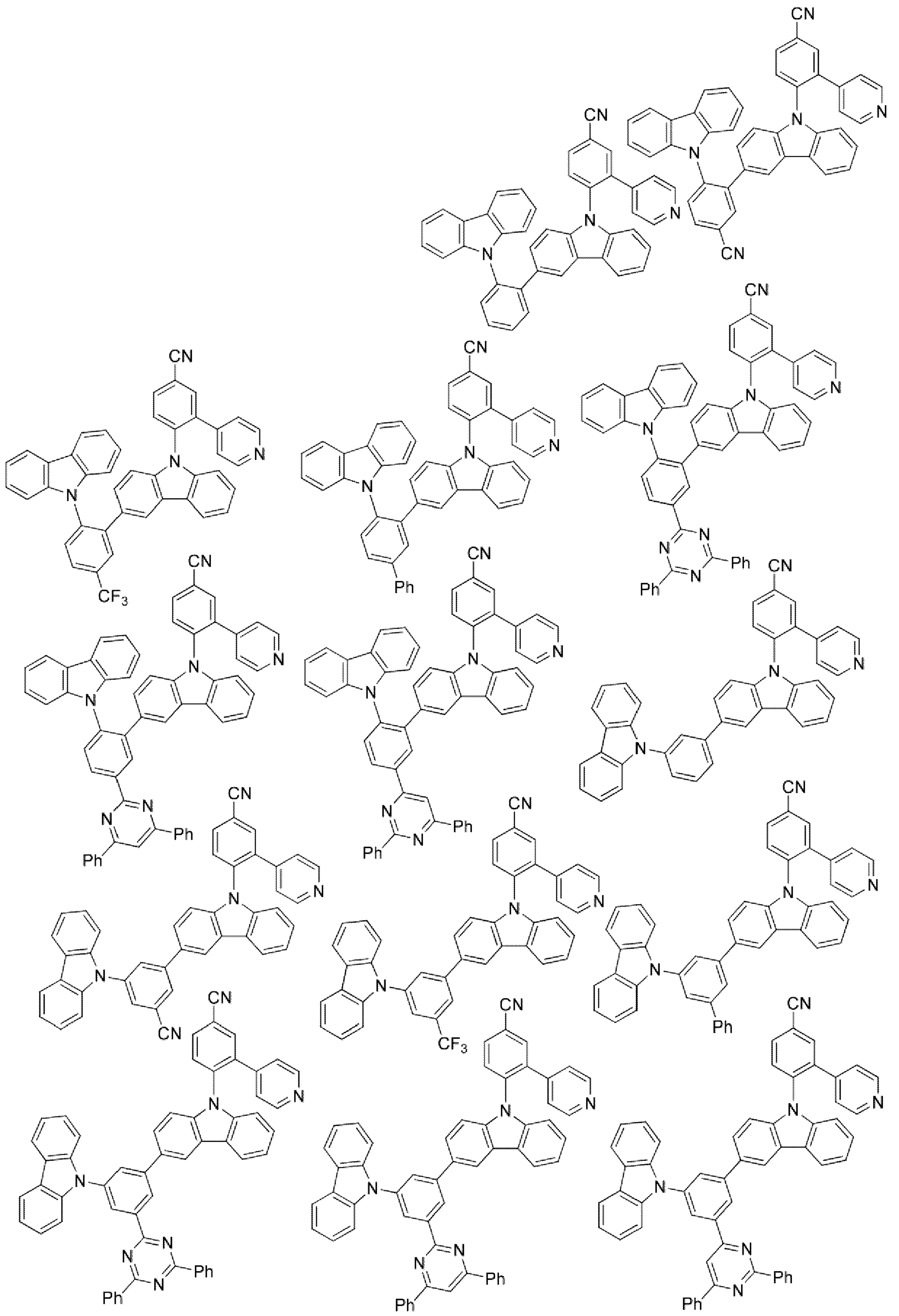

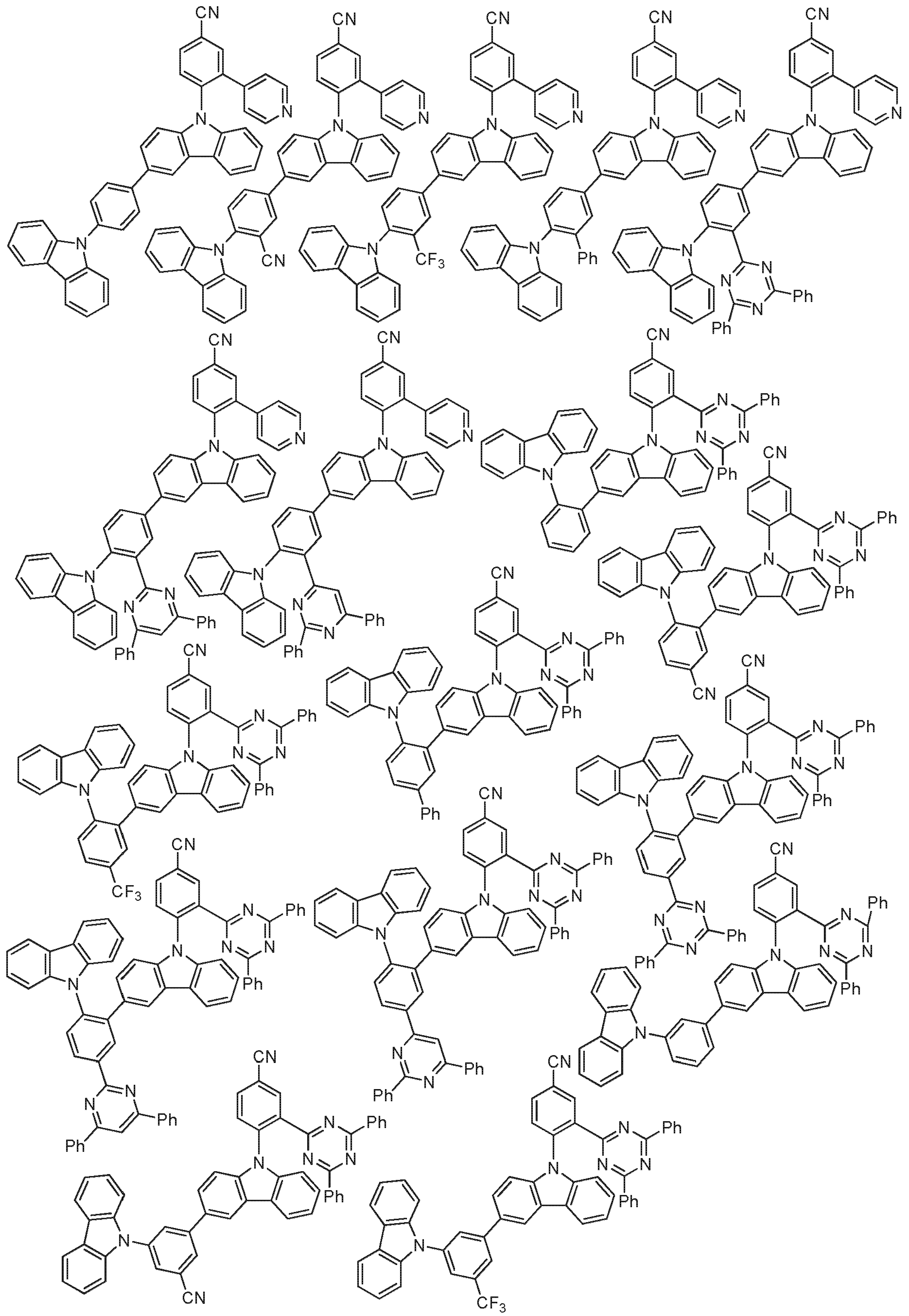

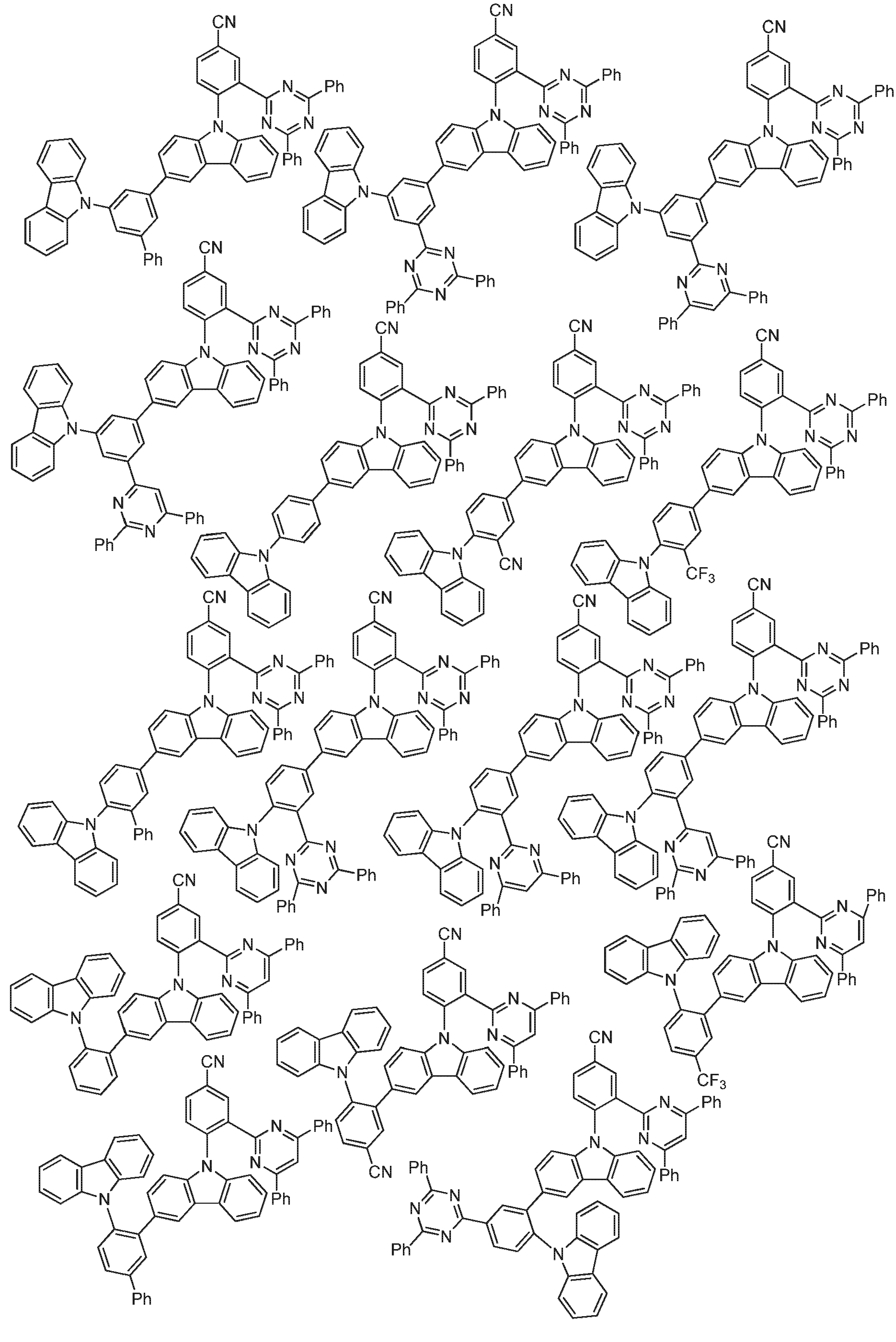

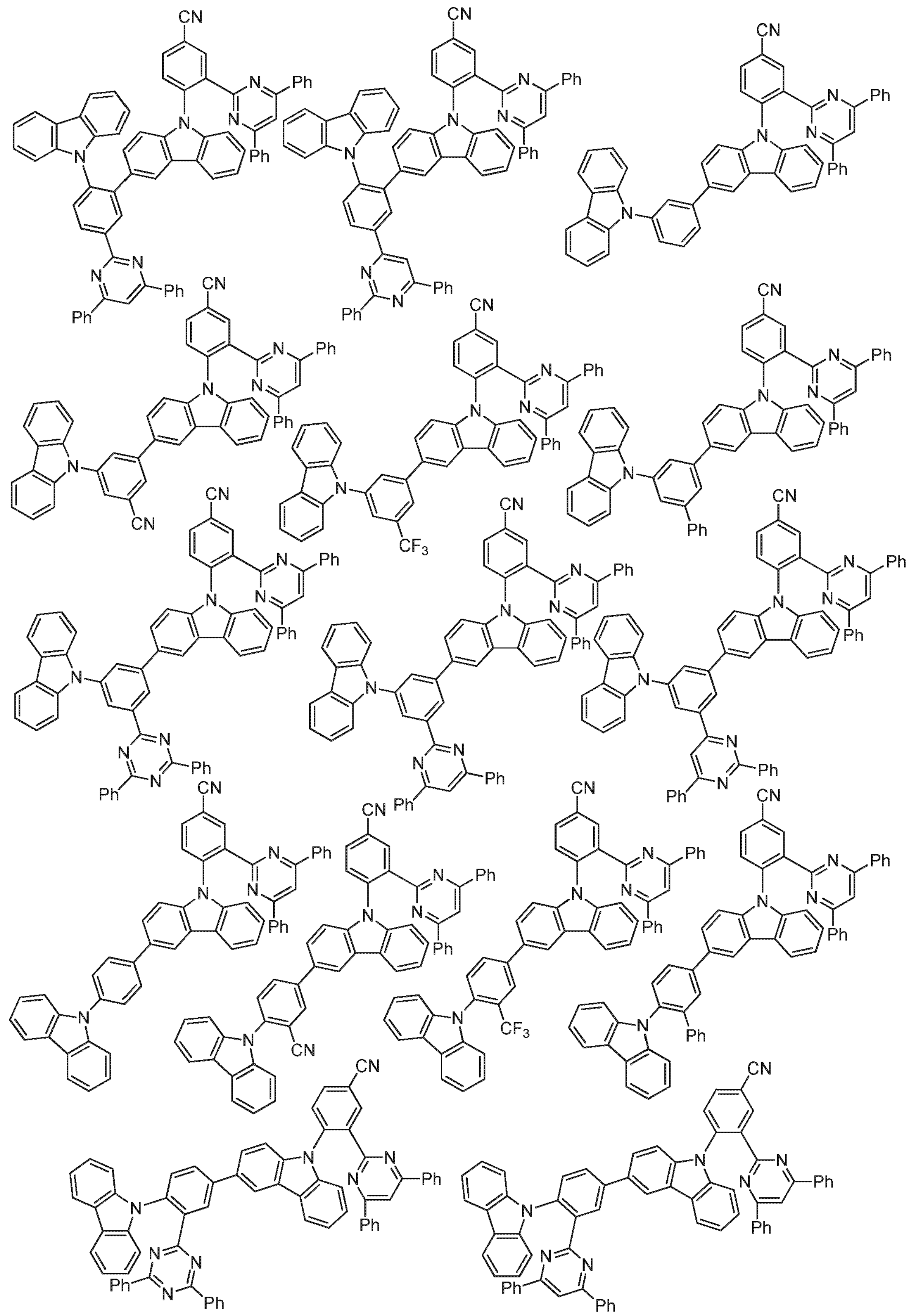

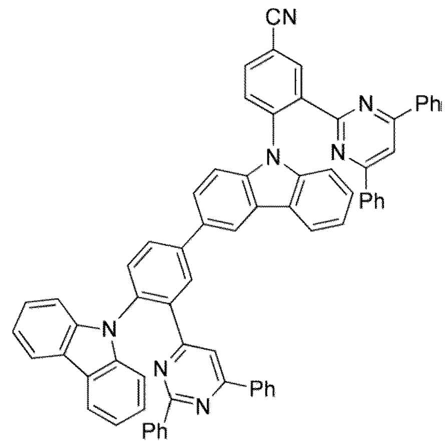

**Figuren**

Die Figuren zeigen:

Figur 1 Emissionsspektrum von Beispiel 1 (10 Gew.-%) in PMMA.
Figur 2 Emissionsspektrum von Beispiel 2 (10 Gew.-%) in PMMA.
Figur 3 Emissionsspektrum von Beispiel 3 (10 Gew.-%) in PMMA.
Figur 4 Emissionsspektrum von Beispiel 4 (10 Gew.-%) in PMMA.
Figur 5 Emissionsspektrum von Beispiel 5 (10 Gew.-%) in PMMA.
Figur 6 Emissionsspektrum von Beispiel 6 (10 Gew.-%) in PMMA.
Figur 7 Emissionsspektrum von Beispiel 7 (10 Gew.-%) in PMMA.

**Patentansprüche**

1. Organisches Molekül, ausgewählt aus der Gruppe bestehend aus Formel IIa-1, Formel IIa-2 und Formel IIa-3:

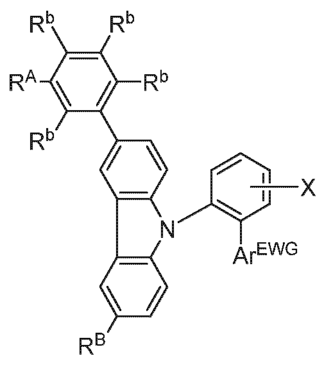

Formel IIa-1

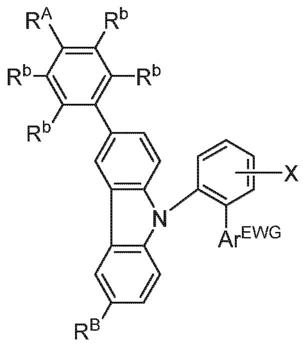

Formel IIa-2

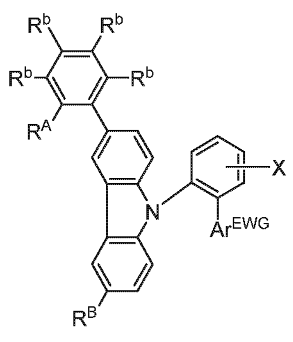

Formel IIa-3

wobei

X ausgewählt ist aus der Gruppe bestehend aus H, $SiMe_3$, $SiPh_3$, CN und $CF_3$;
$Ar^{EWG}$ ausgewählt ist aus der Gruppe bestehend aus den Formeln IIa bis IIc und IIi

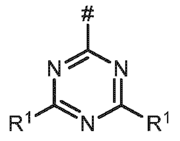

Formel IIa

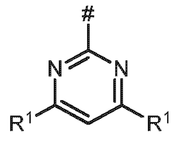

Formel IIb

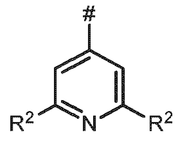

Formel IIc

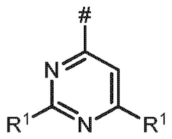

Formel IIi

wobei # die Bindungsstelle einer Einfachbindung darstellt, die $Ar^{EWG}$ mit dem Phenylring verbindet;
$R^1$ bei jedem Auftreten unabhängig voneinander ausgewählt ist aus der Gruppe bestehend aus Wasserstoff, Deuterium,
$C_1$-$C_5$-Alkyl,
wobei ein oder mehrere Wasserstoffatome optional substituiert sind durch Deuterium;
$C_2$-$C_8$-Alkenyl,
wobei ein oder mehrere Wasserstoffatome optional substituiert sind durch Deuterium;
$C_2$-$C_8$-Alkinyl,
wobei ein oder mehrere Wasserstoffatome optional substituiert sind durch Deuterium; und
$C_6$-$C_{18}$-Aryl,
das optional substituiert ist mit einem oder mehreren Substituenten $R^6$;
$R^2$ bei jedem Auftreten unabhängig voneinander ausgewählt ist aus der Gruppe bestehend aus Wasserstoff, Deuterium,
$C_1$-$C_5$-Alkyl,
wobei ein oder mehrere Wasserstoffatome optional substituiert sind durch Deuterium;
$C_2$-$C_8$-Alkenyl,
wobei ein oder mehrere Wasserstoffatome optional substituiert sind durch Deuterium;
$C_2$-$C_8$-Alkinyl,
wobei ein oder mehrere Wasserstoffatome optional substituiert sind durch Deuterium; und
$C_6$-$C_{18}$-Aryl,
das optional substituiert ist mit einem oder mehreren Substituenten $R^6$;
$R^d$ bei jedem Auftreten unabhängig voneinander ausgewählt ist aus der Gruppe bestehend aus Wasserstoff, Deuterium, $N(R^6)_2$, $OR^6$, $Si(R^6)_3$, $B(OR^6)_2$, $OSO_2R^6$, $CF_3$, CN, F, Br, I, $C_1$-$C_{40}$-Alkyl,

das optional substituiert ist mit einem oder mehreren Substituenten $R^6$ und
wobei eine oder mehrere nicht benachbarte $CH_2$-Gruppen optional substituiert sind durch $R^6C=CR^6$, C•C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_1$-$C_{40}$-Alkoxy,

das optional substituiert ist mit einem oder mehreren Substituenten $R^6$ und
wobei eine oder mehrere nicht benachbarte $CH_2$-Gruppen optional substituiert sind durch $R^6C=CR^6$, C. C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_1$-$C_{40}$-Thioalkoxy,

das optional substituiert ist mit einem oder mehreren Substituenten $R^6$ und
wobei eine oder mehrere nicht benachbarte $CH_2$-Gruppen optional substituiert sind durch $R^6C=CR^6$, C•C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_2$-$C_{40}$-Alkenyl,

das optional substituiert ist mit einem oder mehreren Substituenten $R^6$ und wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_2$-$C_{40}$-Alkinyl,

das optional substituiert ist mit einem oder mehreren Substituenten $R^6$ und wobei eine oder mehrere nicht benachbarte $CH_2$-Gruppen optional substituiert sind durch $R^6C=CR^6$, C· C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S oder $CONR^6$;

$C_6$-$C_{60}$-Aryl,

das optional substituiert ist mit einem oder mehreren Substituenten $R^6$; und $C_3$-$C_{57}$-Heteroaryl, das optional substituiert ist mit einem oder mehreren Substituenten $R^6$;

$R^6$ bei jedem Auftreten unabhängig voneinander ausgewählt ist aus der Gruppe bestehend aus Wasserstoff, Deuterium, OPh, $CF_3$, CN, F,
$C_1$-$C_5$-Alkyl,
wobei optional ein oder mehrere Wasserstoffatome unabhängig voneinander substituiert sind durch Deuterium, CN, $CF_3$ oder F;
$C_1$-$C_5$-Alkoxy,
wobei optional ein oder mehrere Wasserstoffatome unabhängig voneinander substituiert sind durch Deuterium, CN, $CF_3$ oder F;
$C_1$-$C_5$-Thioalkoxy,
wobei optional ein oder mehrere Wasserstoffatome unabhängig voneinander substituiert sind durch Deuterium, CN, $CF_3$ oder F;
$C_2$-$C_5$-Alkenyl,
wobei optional ein oder mehrere Wasserstoffatome unabhängig voneinander substituiert sind durch Deuterium, CN, $CF_3$ oder F;
$C_2$-$C_5$-Alkinyl,
wobei optional ein oder mehrere Wasserstoffatome unabhängig voneinander substituiert sind durch Deuterium, CN, $CF_3$ oder F;
$C_6$-$C_{18}$-Aryl,
das optional substituiert ist mit einem oder mehrere $C_1$-$C_5$-Alkyl-Substituenten;
$C_3$-$C_{17}$-Heteroaryl,
das optional substituiert ist mit einem oder mehrere $C_1$-$C_5$-Alkyl-Substituenten;
$N(C_6$-$C_{18}$-$Aryl)_2$;
$N(C_3$-$C_{17}$-$Heteroaryl)_2$,
und $N(C_3$-$C_{17}$-Heteroaryl$)(C_6$-$C_{18}$-aryl);
$R^A$ bei jedem Auftreten, unabhängig voneinander, ausgewählt ist aus einer chemischen Struktur der Formel IIA

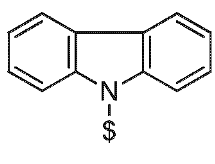

Formel IIA

oder einer chemischen Struktur der Formel IIB

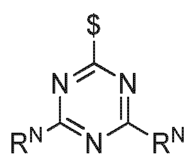

Formel IIB,

wobei $R^N$ ausgewählt ist aus der Gruppe bestehend aus Me, $^iPr$, $^tBu$, CN, $CF_3$ und Ph;
wobei $ die Bindungsstelle einer Einfachbindung darstellt;
wobei $R^B$ ausgewählt ist aus der Gruppe bestehend aus
H,
Ph,
CN,
$CF_3$,
aus einer chemischen Struktur der Formel IIA-2

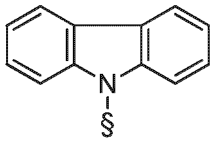

Formel IIA-2,

oder einer chemischen Struktur der Formel IIB-2

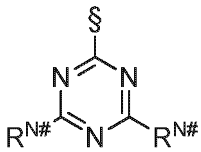

Formel IIB-2;

wobei $R^{N\#}$ ausgewählt ist aus der Gruppe bestehend aus Me, $^iPr$, $^tBu$, CN, $CF_3$ und Ph;
wobei § die Bindungsstelle einer Einfachbindung darstellt und
wobei $R^b$ bei jedem Auftreten unabhängig voneinander ausgewählt aus der Gruppe bestehend aus:

H,
Me,
$^iPr$,
$^tBu$,
CN,
$CF_3$,
Ph, das optional substituiert ist mit einem oder mehreren Substituenten unabhängig ausgewählt aus der Gruppe bestehend aus Me, $^iPr$, $^tBu$, CN, $CF_3$ und Ph,
Pyridinyl, das optional substituiert ist mit einem oder mehreren Substituenten unabhängig ausgewählt aus der Gruppe bestehend aus Me, $^iPr$, $^tBu$, CN, $CF_3$ und Ph,
Pyrimidinyl, das optional substituiert ist mit einem oder mehreren Substituenten unabhängig ausgewählt aus der Gruppe bestehend aus Me, $^iPr$, $^tBu$, CN, $CF_3$ und Ph,
Carbazolyl, das optional substituiert ist mit einem oder mehreren Substituenten unabhängig ausgewählt aus der Gruppe bestehend aus Me, $^iPr$, $^tBu$, CN, $CF_3$ und Ph,
Triazinyl, das optional substituiert ist mit einem oder mehreren Substituenten unabhängig ausgewählt aus der Gruppe bestehend aus Me, $^iPr$, $^tBu$, CN, $CF_3$ und Ph,
und $N(Ph)_2$.

**2.** Organisches Molekül nach Anspruch 1, wobei $R^1$ bei jedem Auftreten unabhängig voneinander Phenyl, das optional substituiert ist mit einem oder mehreren Substituenten $R^6$, ist und

$R^2$ bei jedem Auftreten unabhängig voneinander ausgewählt ist aus der Gruppe bestehend aus Wasserstoff,
CN,
$CF_3$ und
Phenyl, das optional substituiert ist mit einem oder mehreren Substituenten $R^6$.

**3.** Organisches Molekül nach Anspruch 1 oder 2, aufweisend eine Struktur, ausgewählt aus der nachfolgend darge-

stellten Gruppe:

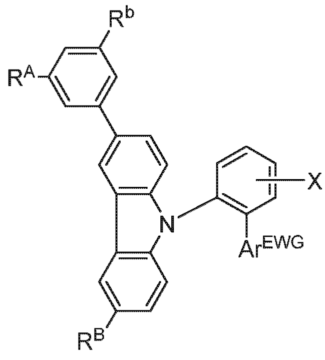

Formel IIc-1

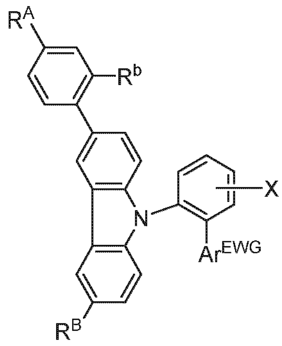

Formel IIc-2

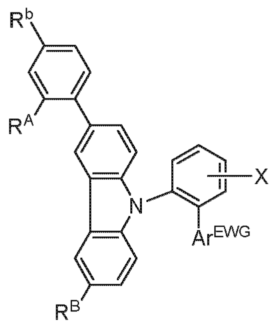

Formel IIc-3.

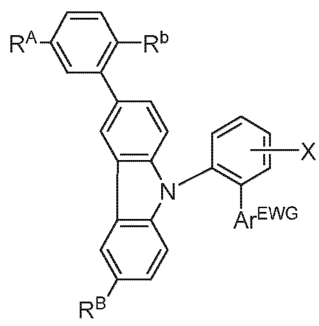

Formel IIc-4

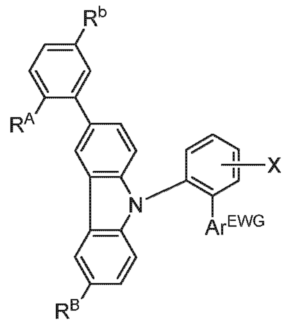

Formel IIc-5.

**4.** Organisches Molekül nach einem oder mehreren der Ansprüche 1 bis 3, wobei $R^b$ bei jedem Auftreten unabhängig ausgewählt ist aus der Gruppe bestehend aus H und CN.

**5.** Organisches Molekül nach einem oder mehreren der Ansprüche 1 bis 4, aufweisend oder bestehend aus einer Struktur, ausgewählt aus der nachfolgend dargestellten Gruppe:

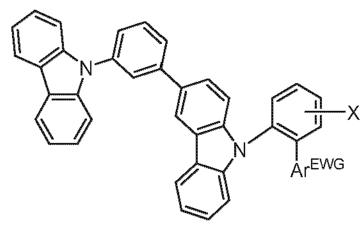

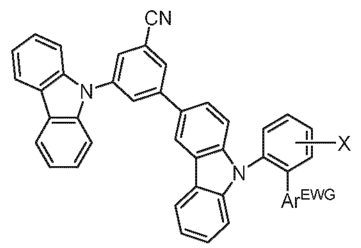

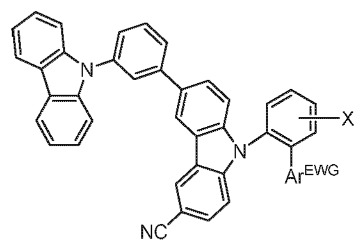

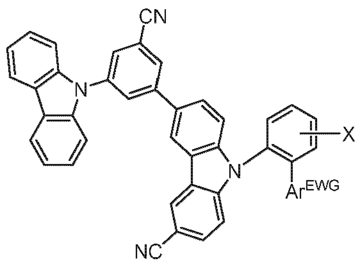

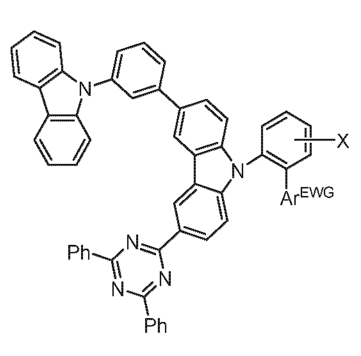

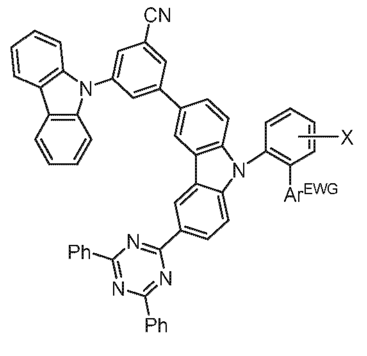

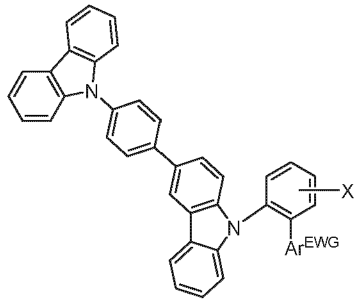
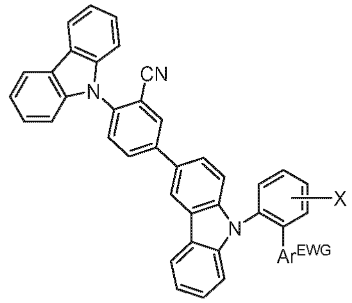
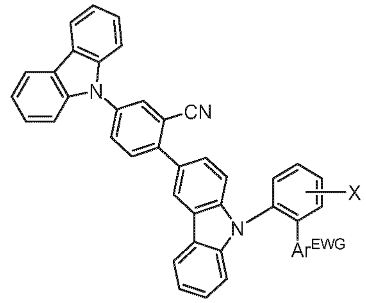

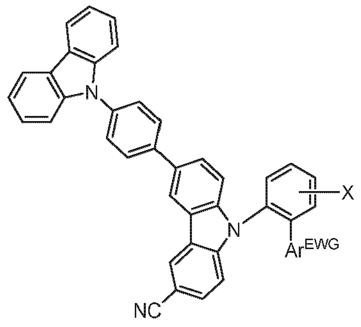
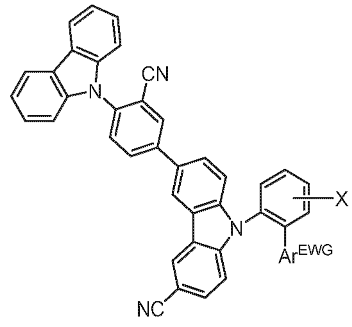
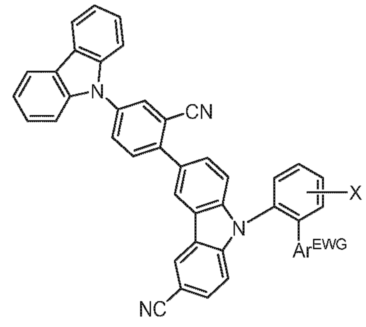

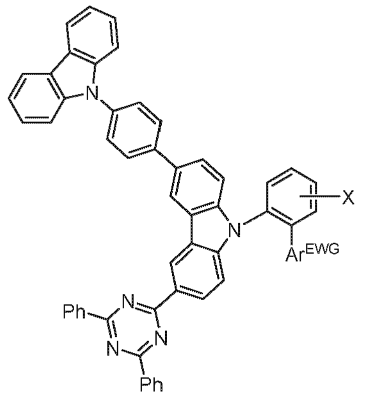
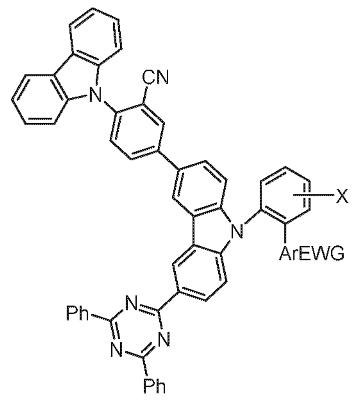
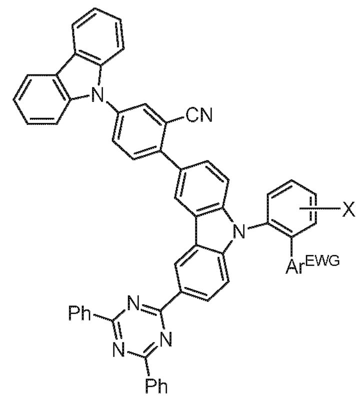

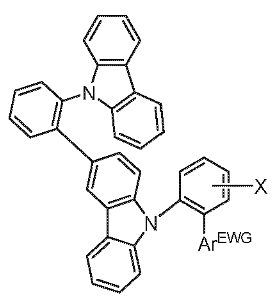
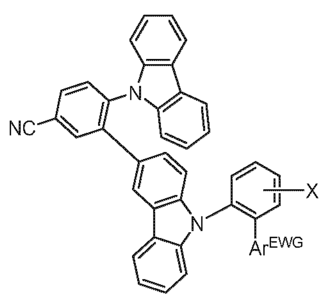
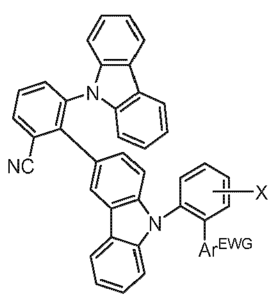

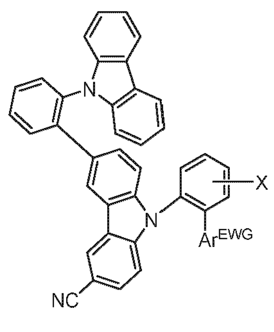
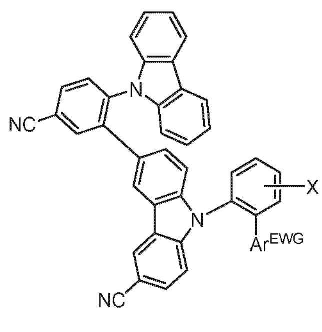
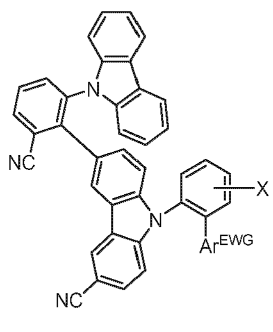

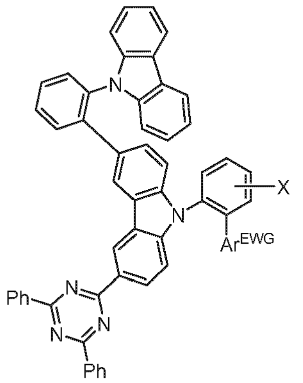
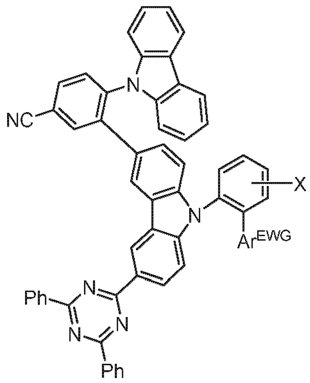
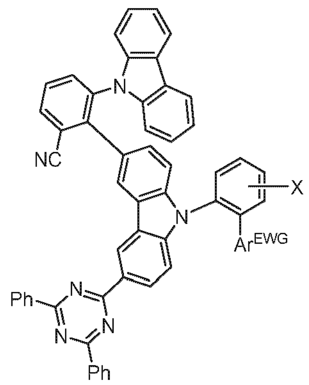

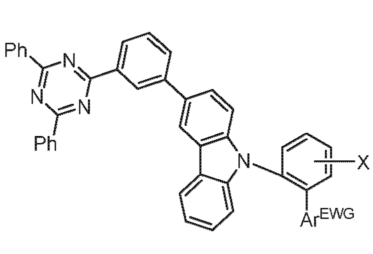
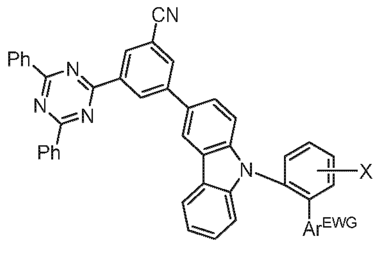
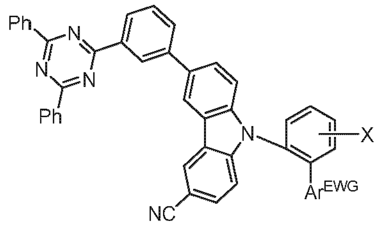

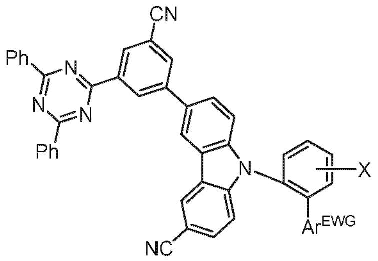
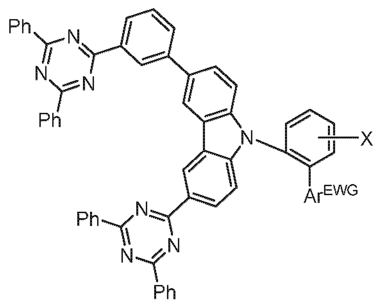
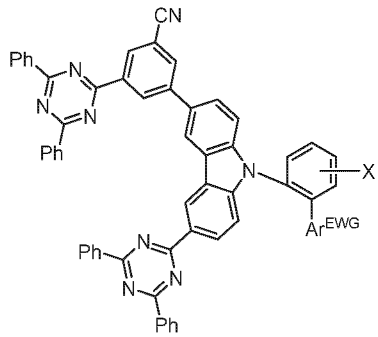

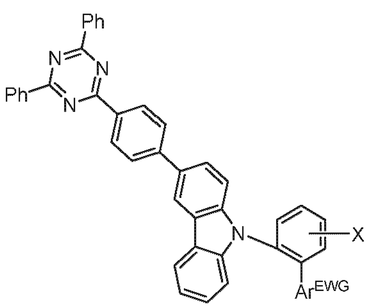
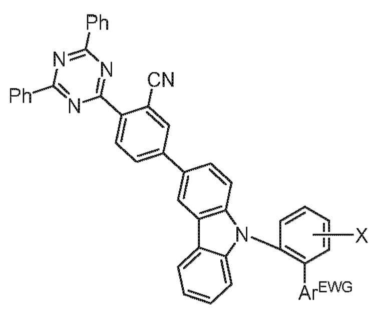
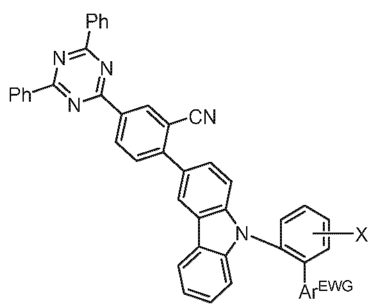

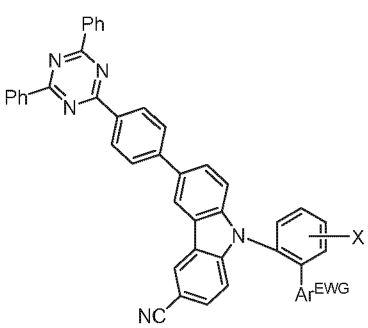
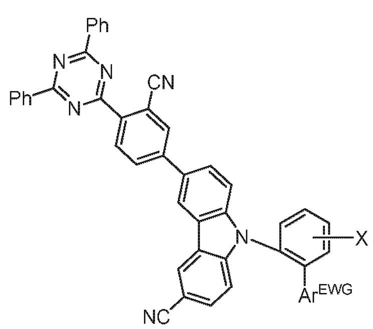
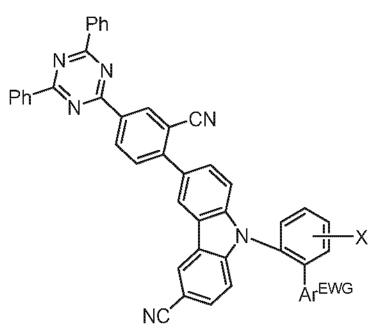

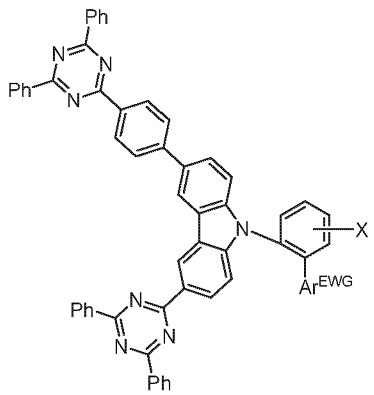
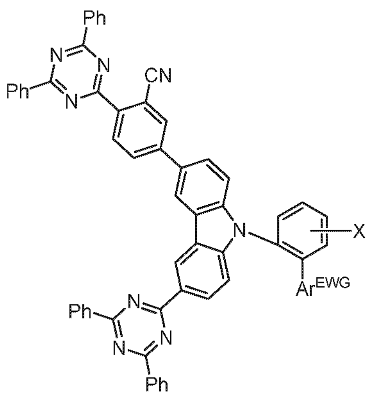
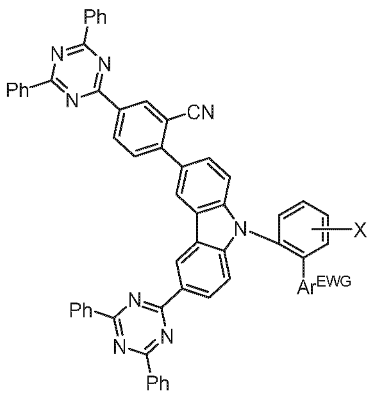

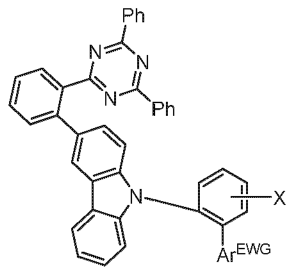
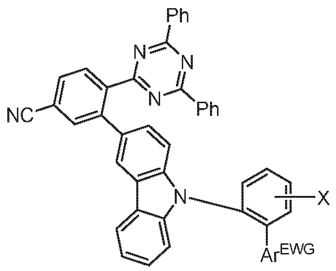
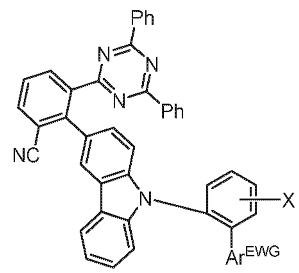

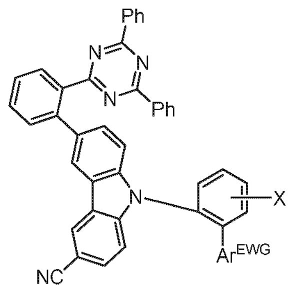
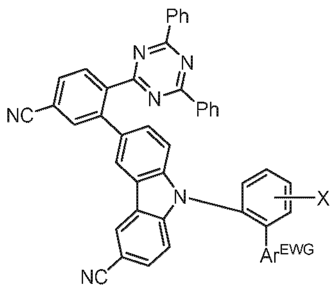
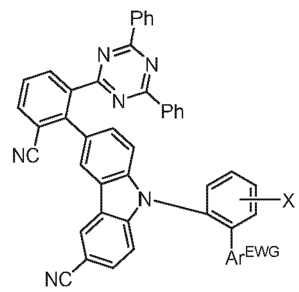

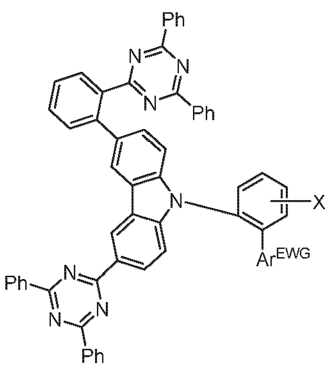
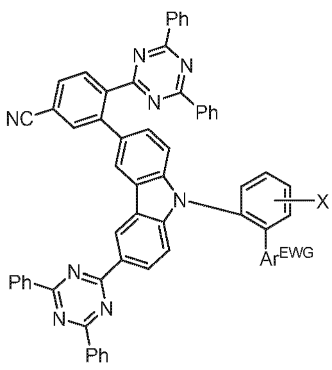
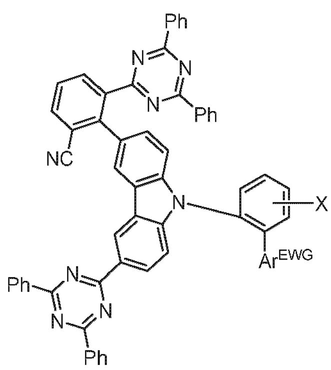

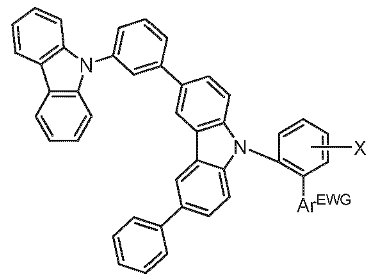

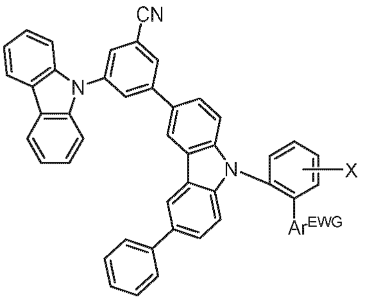

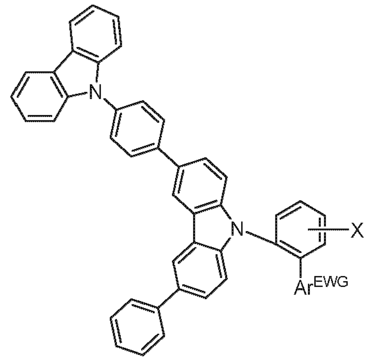

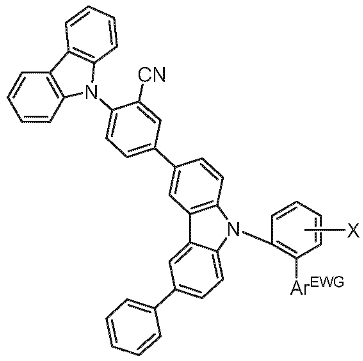

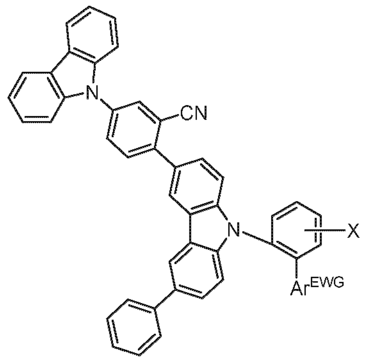

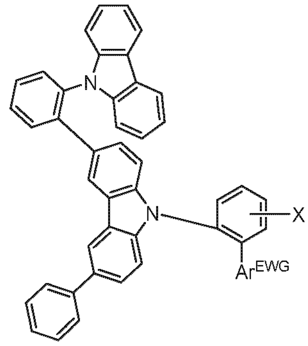

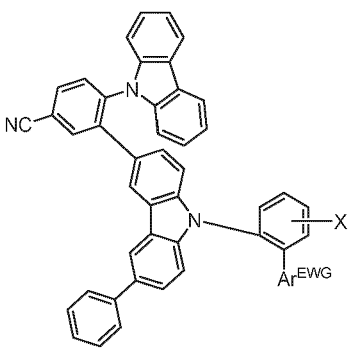

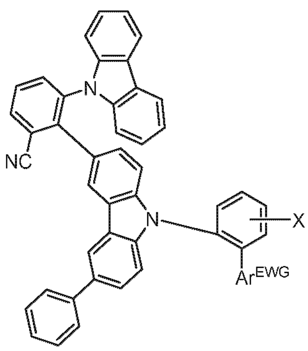

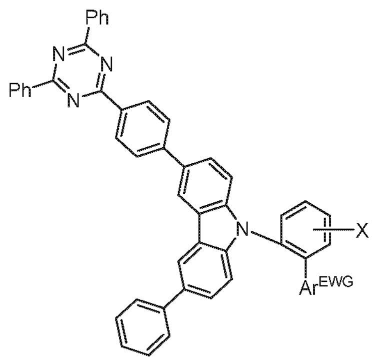

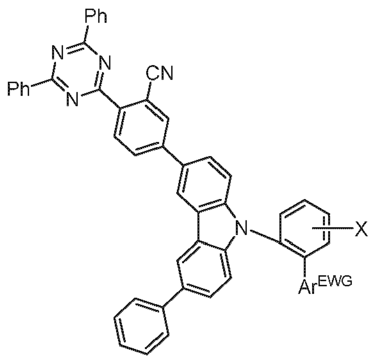

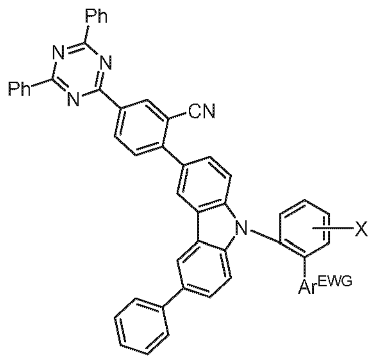

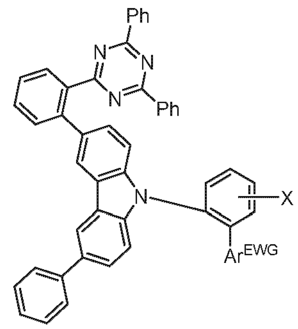

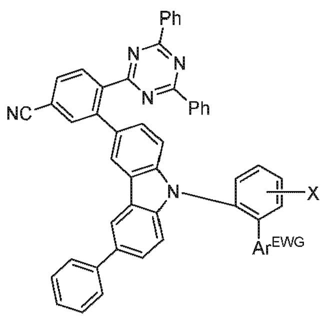
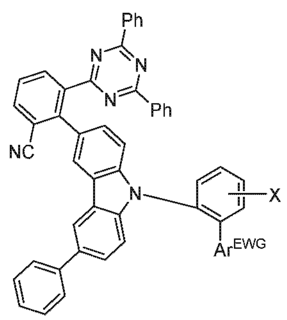
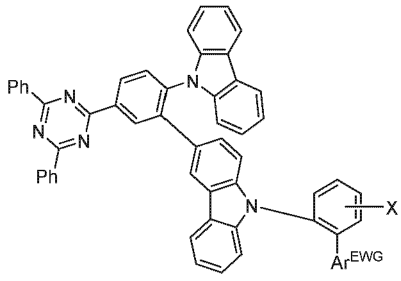

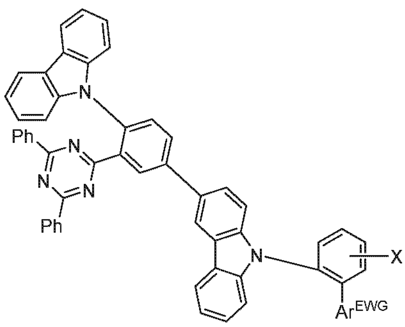
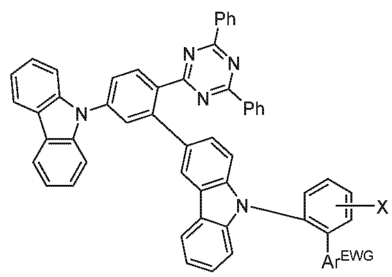
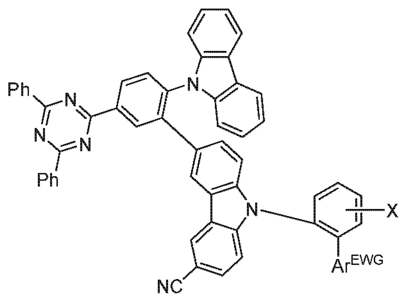

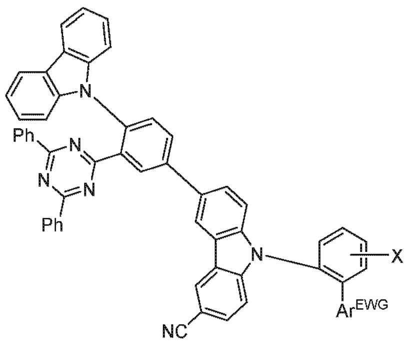
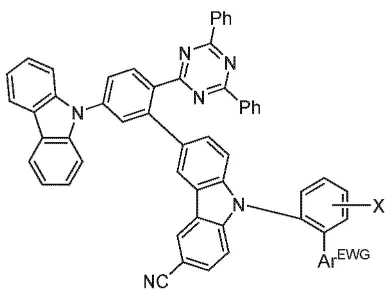
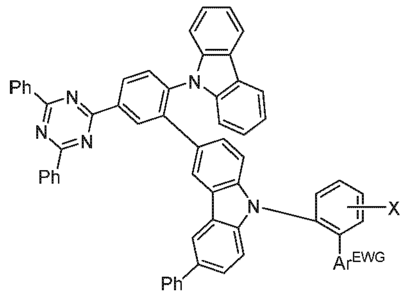

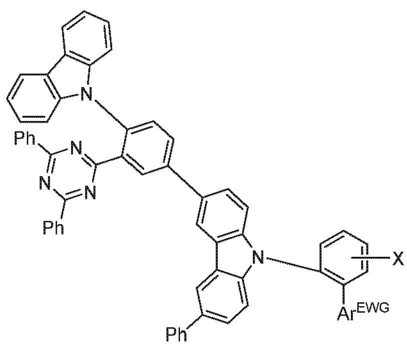
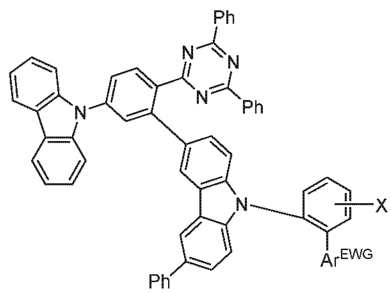

6. Verfahren zur Herstellung eines organischen Moleküls nach Anspruch 1 bis 5, aufweisend den Schritt der Bereitstellung eines 6-$R^B$-substituierten 3-Bromocarbazols als Reaktant.

7. Verwendung eines Moleküls nach einem oder mehrerer der Ansprüche 1 bis 5 als Lumineszenzemitter und/oder Transportmaterial und/oder Lochinjektionsmaterial und/oder Lochblockiermaterial in einer optoelektronischen Vorrichtung.

8. Verwendung nach Anspruch 7, wobei die optoelektronische Vorrichtung ausgewählt ist aus der Gruppe bestehend aus:

- organischen lichtemittierenden Dioden (OLEDs),
- lichtemittierenden elektrochemischen Zellen,
- OLED-Sensoren,
- organischen Dioden,
- organischen Solarzellen,
- organischen Transistoren,
- organischen Feldeffekttransistoren,
- organischen Lasern und
- Down-Konversionselementen.

**9.** Zusammensetzung, aufweisend:

(a) ein organisches Molekül nach einem oder mehreren der Ansprüche 1 bis 5, insbesondere in Form eines Emitters, und
(b) ein Emitter- und/oder Hostmaterial, das sich von dem organischen Molekül nach einem oder mehreren der Ansprüche 1 bis 5 unterscheidet, und
(c) optional einen Farbstoff und/oder ein Lösungsmittel.

**10.** Optoelektronische Vorrichtung, aufweisend ein organisches Molekül nach einem oder mehreren der Ansprüche 1 bis 5 oder eine Zusammensetzung nach Anspruch 9, insbesondere in Form einer Vorrichtung ausgewählt aus der Gruppe bestehend aus organischer lichtemittierender Diode (OLED), lichtemittierender elektrochemischer Zelle, OLED-Sensor, organischer Diode, organischer Solarzelle, organischem Transistor, organischem Feldeffekttransistor, organischem Laser und Down-Konversionselement.

**11.** Optoelektronische Vorrichtung nach Anspruch 10, aufweisend

- ein Substrat,
- eine Anode und
- eine Kathode, wobei die Anode oder die Kathode auf dem Substrat angeordnet sind, und
- eine lichtemittierende Schicht, die zwischen der Anode und der Kathode angeordnet ist und die ein organisches Molekül gemäß einem oder mehreren der Ansprüche 1 bis 5 oder eine Zusammensetzung nach Anspruch 9 aufweist.

**12.** Verfahren zur Herstellung einer optoelektronischen Vorrichtung, wobei ein organisches Molekül nach einem oder mehreren der Ansprüche 1 bis 5 oder eine Zusammensetzung nach Anspruch 9 verwendet wird.

**13.** Verfahren nach Anspruch 12, aufweisend das Verarbeiten des organischen Moleküls oder der organischen Zusammensetzung durch ein Vakuumverdampfungsverfahren oder aus einer Lösung.

## Claims

**1.** Organic molecule selected from the group consisting of formula IIa-1, formula IIa-2 and formula IIa-3:

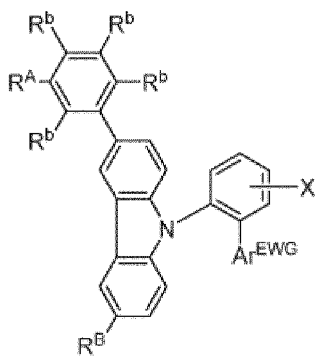

Formula IIa-1

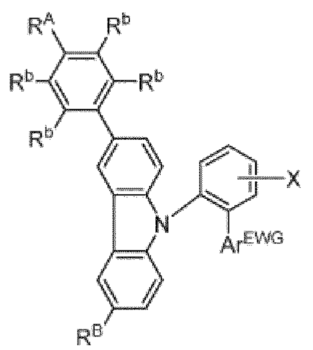

Formula IIa-2

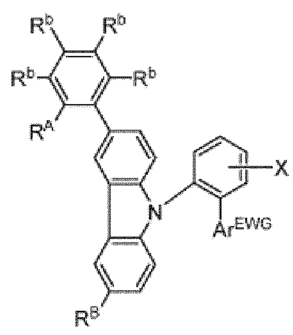

Formula IIa-3

wherein

X is selected from the group consisting of H, $SiMe_3$, $SiPh_3$, CN and $CF_3$;
$Ar^{EWG}$ is selected from the group consisting of formulae IIa to IIc and IIi

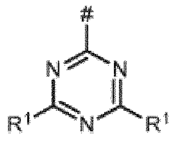

Formula IIa

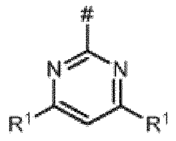

Formula IIb

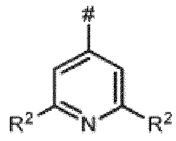

Formula IIc

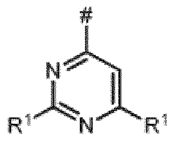

Formula IIi

wherein # represents the bonding site of a single bond connecting $Ar^{EWG}$ to the phenyl ring;
$R^1$ is selected independently from each other at each occurrence from the group consisting of
hydrogen,
deuterium,
$C_1$-$C_5$ alkyl,
wherein one or more hydrogen atoms are optionally substituted by deuterium; $C_2$-$C_8$ alkenyl,
wherein one or more hydrogen atoms are optionally substituted by deuterium; $C_2$-$C_8$ alkynyl,
wherein one or more hydrogen atoms are optionally substituted by deuterium;
and
$C_6$-$C_{18}$ aryl,
which is optionally substituted by one or more substituents $R^6$;
$R^2$ is selected independently from each other at each occurrence from the group consisting of
hydrogen,
deuterium,
$C_1$-$C_5$ alkyl,
wherein one or more hydrogen atoms are optionally substituted by deuterium; $C_2$-$C_8$ alkenyl,
wherein one or more hydrogen atoms are optionally substituted by deuterium; $C_2$-$C_8$ alkynyl,
wherein one or more hydrogen atoms are optionally substituted by deuterium; and
$C_6$-$C_{18}$ aryl,
which is optionally substituted with one or more substituents $R^6$;
$R^d$ is selected independently from each other at each occurrence from the group consisting of hydrogen, deuterium, $N(R^6)_2$, $OR^6$, $Si(R^6)_3$, $B(OR^6)_2$, $OSO_2R^6$, $CF_3$,
CN, F, Br, I,
$C_1$-$C_{40}$ alkyl,
which is optionally substituted by one or more substituents $R^6$ and wherein one or more non-adjacent $CH_2$ groups are optionally substituted by $R^6C$=$CR^6$, C·C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S or $CONR^6$;
$C_1$-$C_{40}$-alkoxy,
which is optionally substituted by one or more substituents $R^6$ and wherein one or more non-adjacent $CH_2$ groups are optionally substituted by $R^6C$=$CR^6$, C•C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S or $CONR^6$;
$C_1$-$C_{40}$-thioalkoxy,
which is optionally substituted by one or more substituents $R^6$ and wherein one or more non-adjacent $CH_2$ groups are optionally substituted by $R^6C$=$CR^6$, C·C, $Si(R^6)_2$, $Ge(R^6)_2$, $Sn(R^6)_2$, C=O, C=S, C=Se, C=$NR^6$, P(=O)($R^6$), SO, $SO_2$, $NR^6$, O, S or $CONR^6$;
$C_2$-$C_{40}$-alkenyl,
which is optionally substituted by one or more substituents $R^6$ and wherein one or more non-adjacent $CH_2$ groups are optionally substituted by $R^6C$=$CR^6$, C·C, $Si(R^6)_2$, $Ge(R^6)_2$, $Sn(R^6)_2$, C=O, C=S, C=Se, C=$NR^6$,

P(=O)($R^6$), SO, $SO_2$, $NR^6$, O, S or $CONR^6$;
$C_2$-$C_{40}$-alkynyl,
which is optionally substituted by one or more substituents $R^6$ and wherein one or more non-adjacent $CH_2$ groups are optionally substituted by $R^6C=CR^6$, C•C, $Si(R^6)_2$, $Ge(R^6)_2$, $Sn(R^6)_2$, C=O, C=S, C=Se, $C=NR^6$, P(=O)($R^6$), SO, $SO_2$, $NR^6$, O, S or $CONR^6$;
$C_6$-$C_{60}$ aryl,
which is optionally substituted by one or more substituents $R^6$; and
$C_3$-$C_{57}$ heteroaryl,
which is optionally substituted by one or more substituents $R^6$;
$R^6$ is selected independently from each other at each occurrence from the group consisting of hydrogen, deuterium, OPh, $CF_3$, CN, F,
$C_1$-$C_5$-alkyl,
wherein optionally one or more hydrogen atoms are substituted independently from each other by deuterium, CN, $CF_3$ or F;
$C_1$-$C_5$-alkoxy,
wherein optionally one or more hydrogen atoms are substituted independently from each other by deuterium, CN, $CF_3$ or F;
$C_1$-$C_5$-thioalkoxy,
wherein optionally one or more hydrogen atoms are substituted independently from each other by deuterium, CN, $CF_3$ or F;
$C_2$-$C_5$-alkenyl,
wherein optionally one or more hydrogen atoms are substituted independently from each other by deuterium, CN, $CF_3$ or F;
$C_2$-$C_5$-alkynyl,
wherein optionally one or more hydrogen atoms are substituted independently from each other by deuterium, CN, $CF_3$ or F;
$C_6$-$C_{18}$-aryl,
which is optionally substituted by one or more $C_1$-$C_5$-alkyl substituents; $C_3$-$C_{17}$-heteroaryl,
which is optionally substituted by one or more $C_1$-$C_5$-alkyl substituents; $N(C_6$-$C_{18}$-aryl$)_2$;
$N(C_3$-$C_{17}$-heteroaryl$)_2$,
and $N(C_3$-$C_{17}$-heteroaryl)($C_6$-$C_{18}$-aryl);
$R^A$ is selected independently from each other at each occurrence from a chemical structure of formula IIA

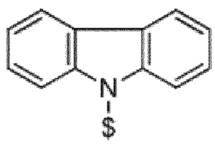

Formula IIA

or a chemical structure of formula IIB

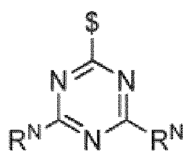

Formula IIB,

wherein $R^N$ is selected from the group consisting of Me, $^iPr$, $^tBu$, CN, $CF_3$ and Ph;
wherein $ represents the binding site of a single bond;
wherein $R^B$ is selected from the group consisting of
H,
Ph,
CN,
$CF_3$,

from a chemical structure of formula IIA-2

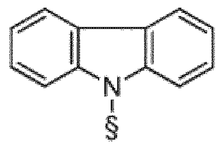

Formula IIA-2,

or a chemical structure of formula IIB-2

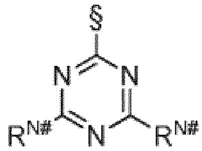

Formula IIB-2;

wherein $R^{N\#}$ is selected from the group consisting of Me, $^{i}Pr$, $^{t}Bu$, CN, $CF_3$ and Ph;
wherein § represents the binding site of a single bond; and
wherein $R^b$ is selected independently from each other at each occurrence from the group consisting of
H,
Me,
$^{i}Pr$,
$^{t}Bu$,
CN,
$CF_3$,
Ph, which is optionally substituted by one or more substituents independently selected from the group consisting of Me, $^{i}Pr$, $^{t}Bu$, CN, $CF_3$ and Ph,
pyridinyl, which is optionally substituted by one or more substituents independently selected from the group consisting of Me, $^{i}Pr$, $^{t}Bu$, CN, $CF_3$ and Ph,
pyrimidinyl, which is optionally substituted by one or more substituents independently selected from the group consisting of Me, $^{i}Pr$, $^{t}Bu$, CN, $CF_3$ and Ph,
carbazolyl, which is optionally substituted by one or more substituents independently selected from the group consisting of Me, $^{i}Pr$, $^{t}Bu$, CN, $CF_3$ and Ph,
triazinyl, which is optionally substituted by one or more substituents independently selected from the group consisting of Me, $^{i}Pr$, $^{t}Bu$, CN, $CF_3$ and Ph,
and $N(Ph)_2$.

**2.** Organic molecule according to claim 1, wherein $R^1$ is independently from each other at each occurrence phenyl which is optionally substituted by one or more substituents $R^6$, and $R^2$ is selected independently from each other at each occurrence from the group consisting of

hydrogen,
CN,
$CF_3$, and
phenyl which is optionally substituted by one or more substituents $R^6$.

**3.** Organic molecule according to claim 1 or 2, comprising a structure selected from the group shown below:

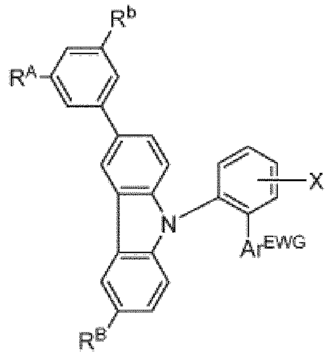

Formula IIc-1

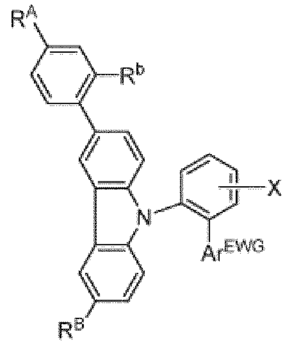

Formula IIc-2

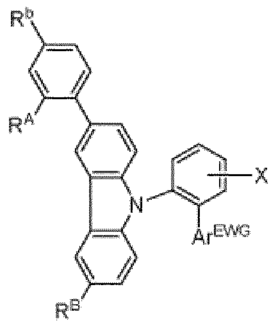

Formula IIc-3

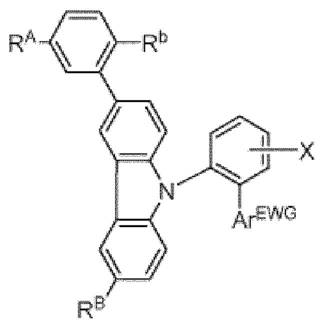

Formula IIc-4

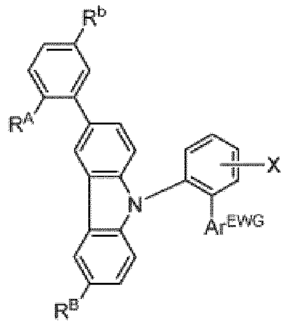

Formula IIc-5.

**4.** Organic molecule according to one or more of claims 1 to 3, wherein $R^b$ is independently selected at each occurrence from the group consisting of H and CN.

**5.** Organic molecule according to one or more of claims 1 to 4, comprising or consisting of a structure selected from the group shown below:

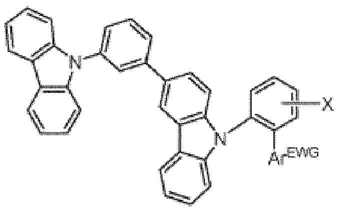

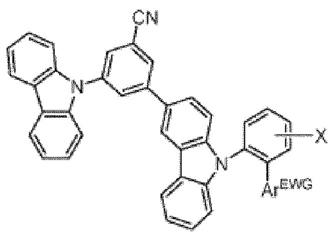

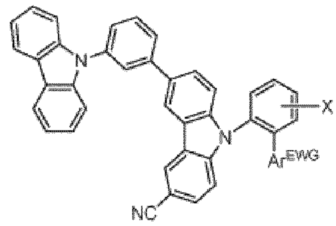

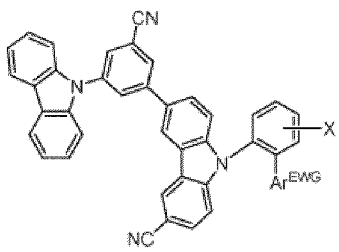

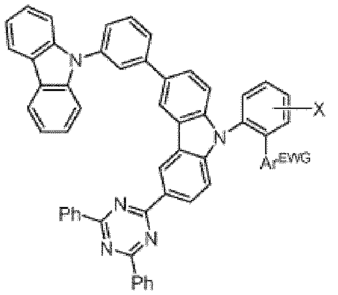

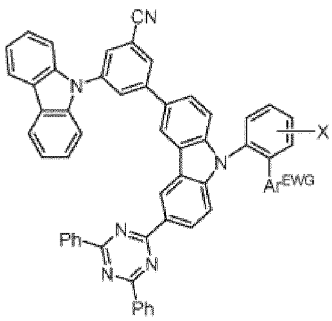

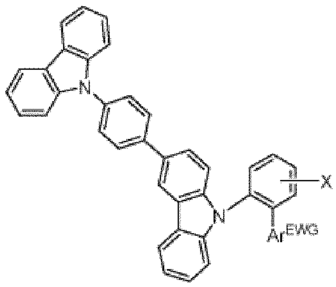
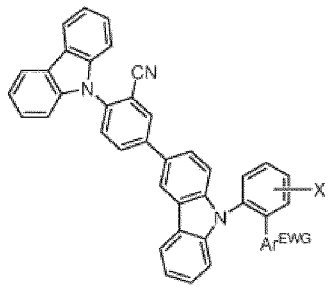
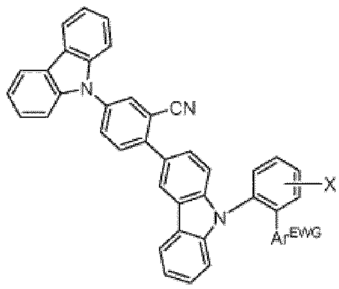

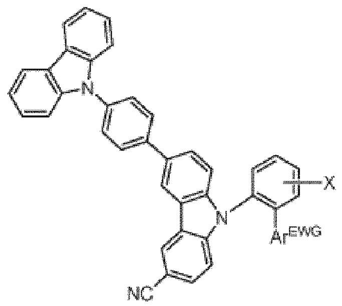
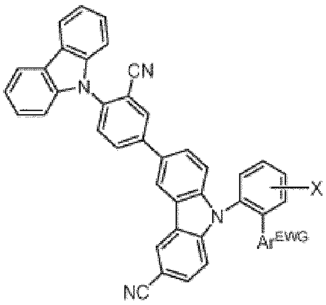
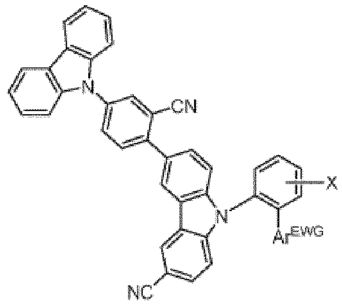

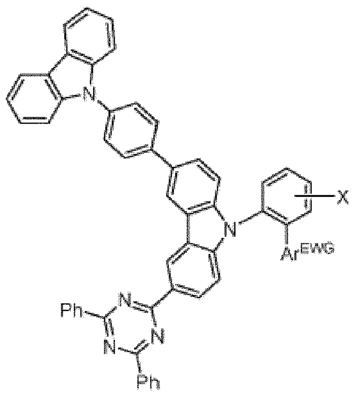
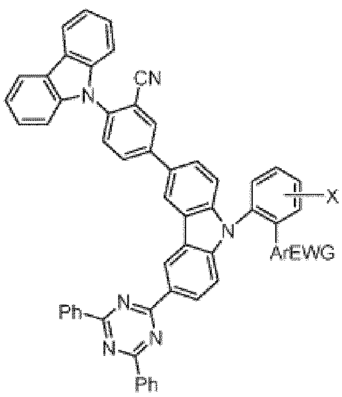
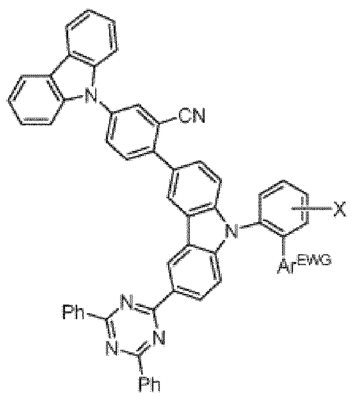

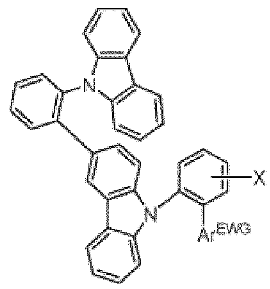
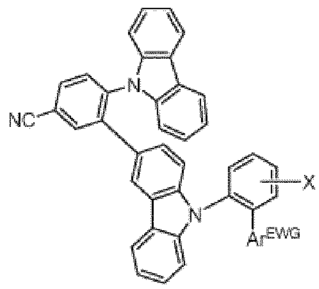
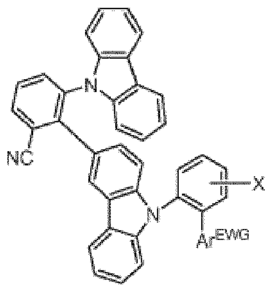

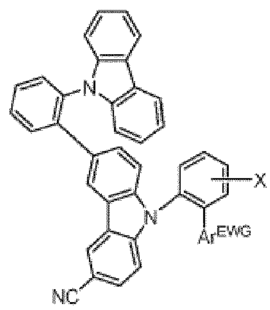
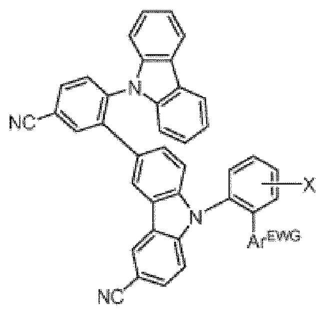
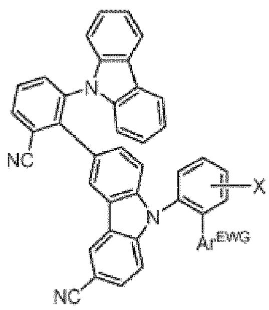

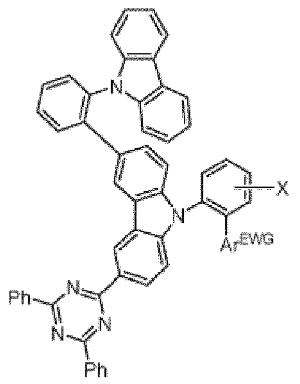
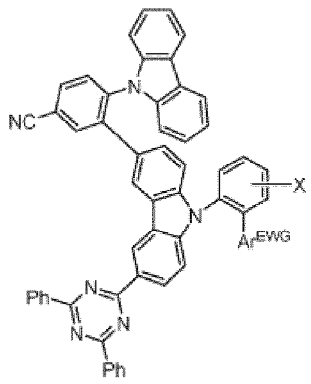
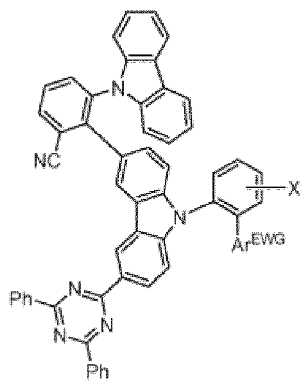

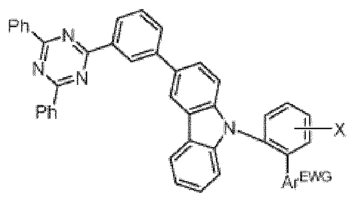
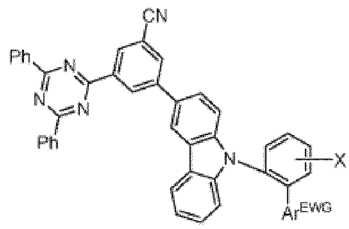
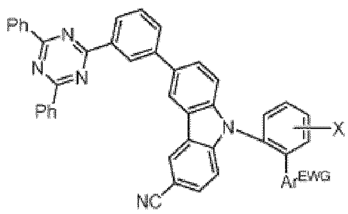

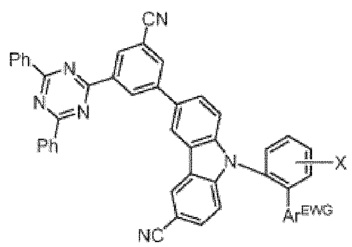
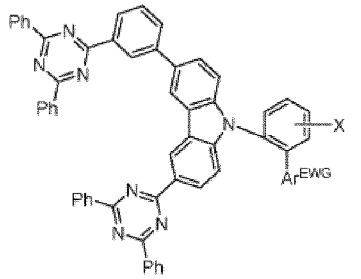
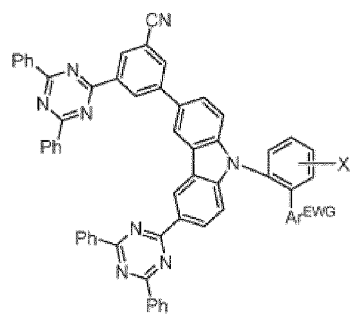

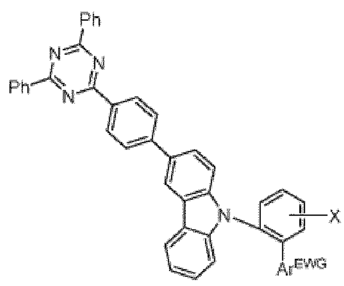
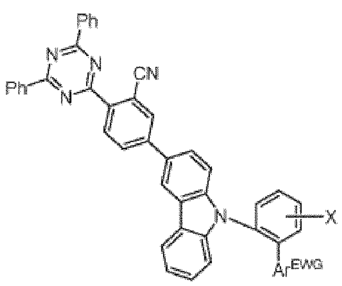
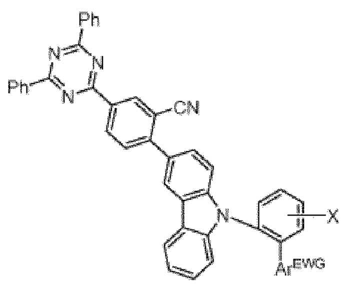

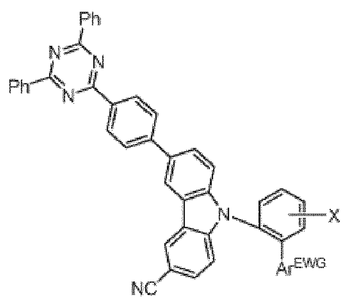
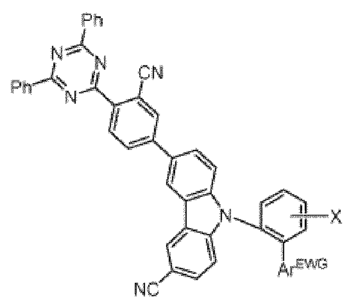
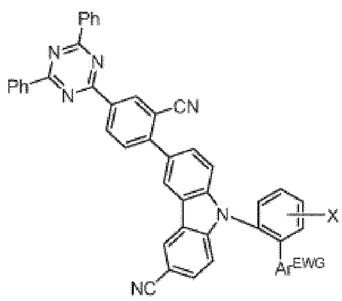

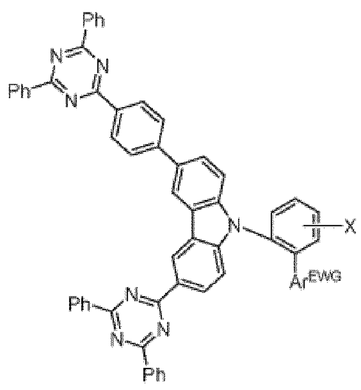
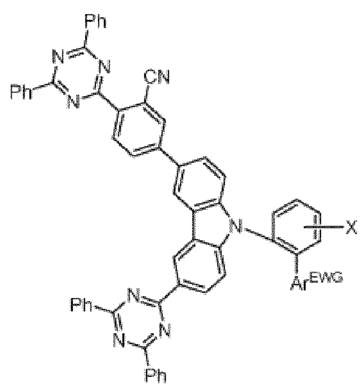
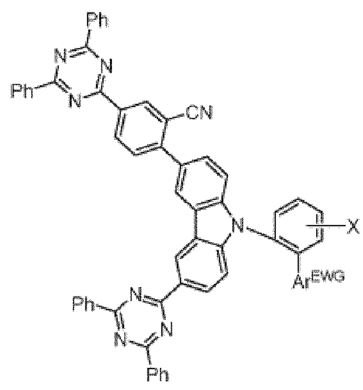

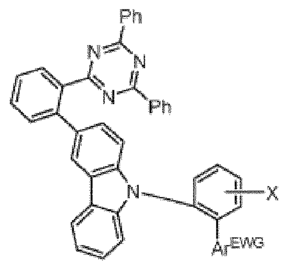
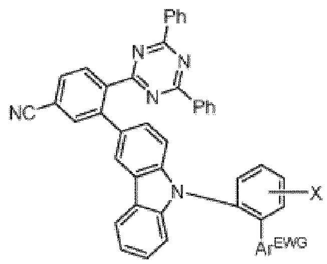
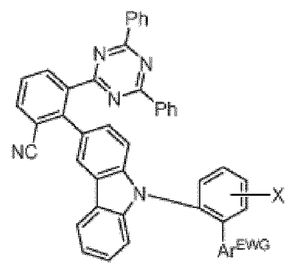

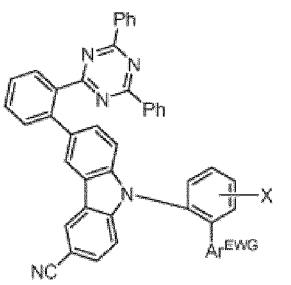
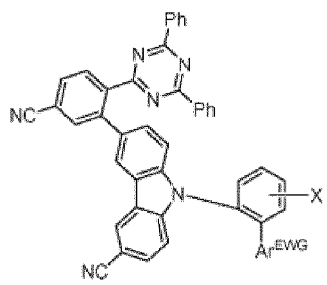
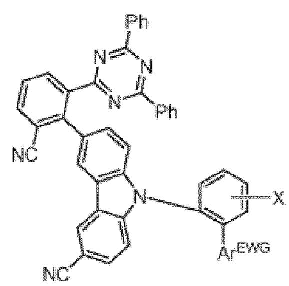

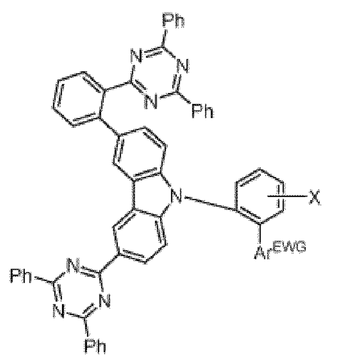
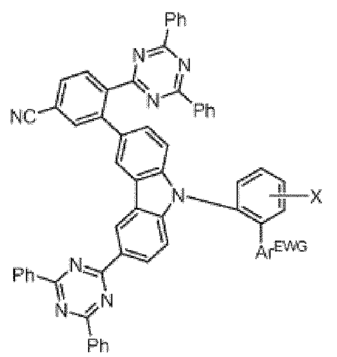
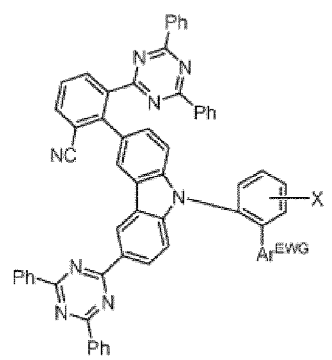

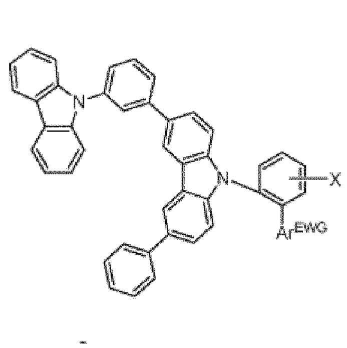
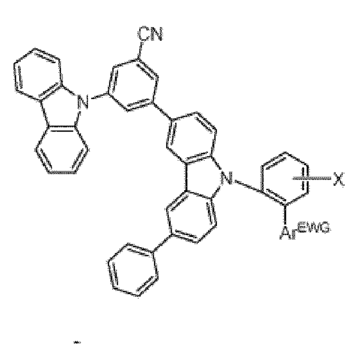
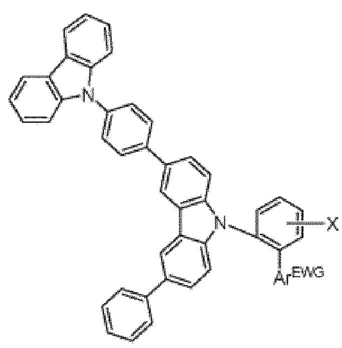

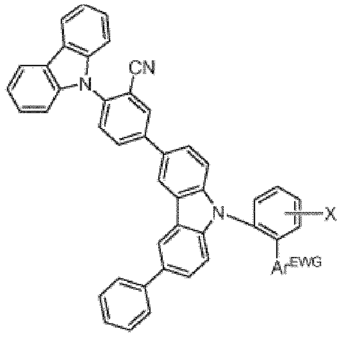

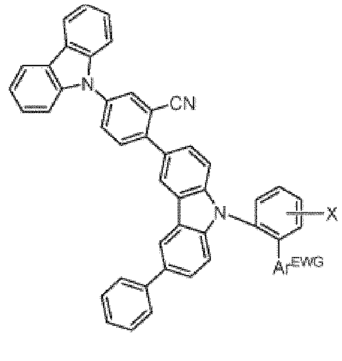

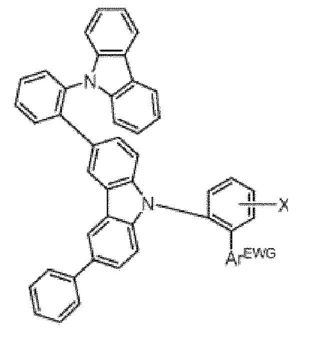

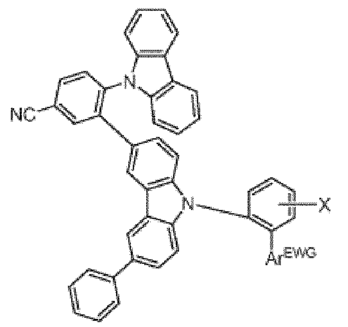

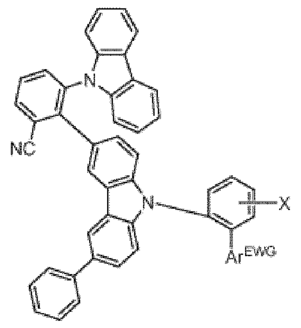

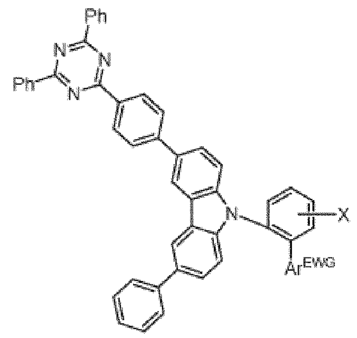

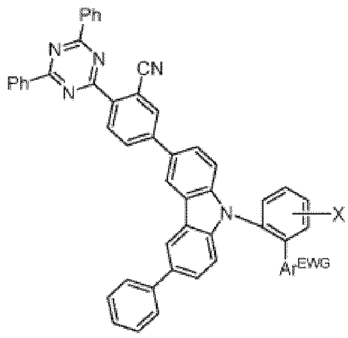

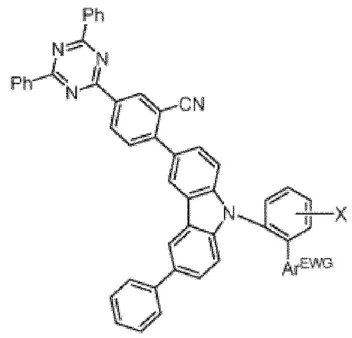

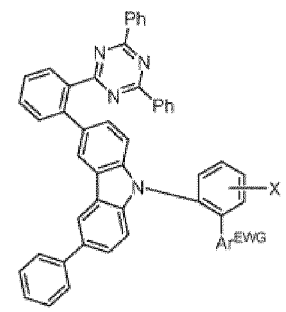

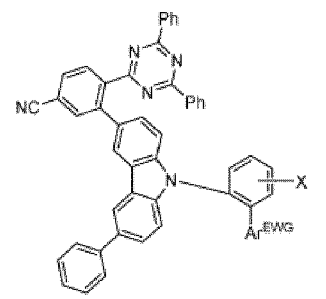

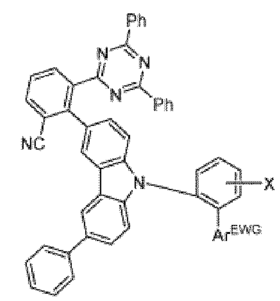

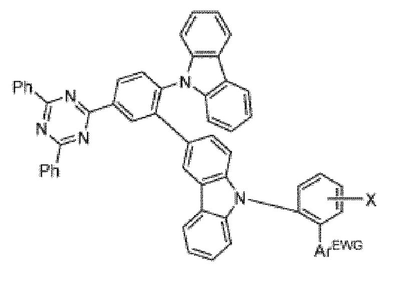

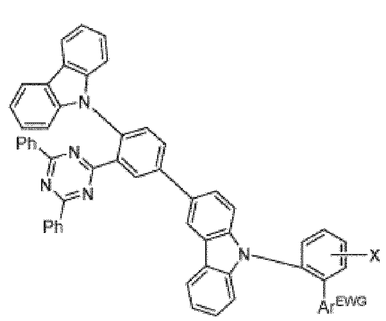

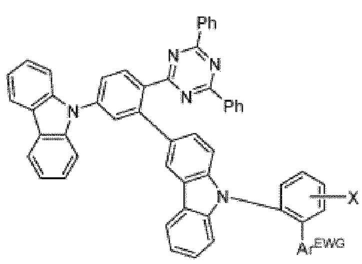

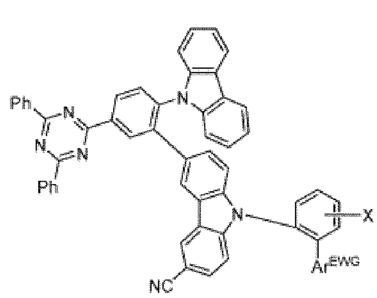

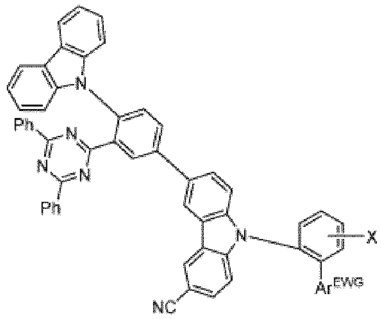
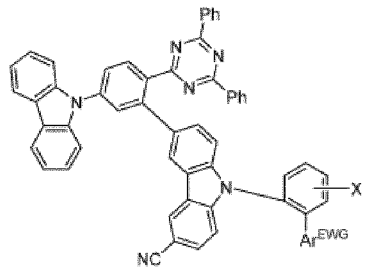
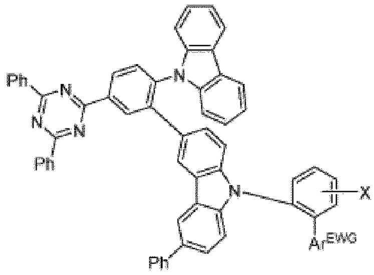

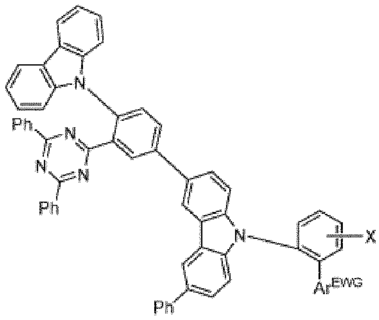
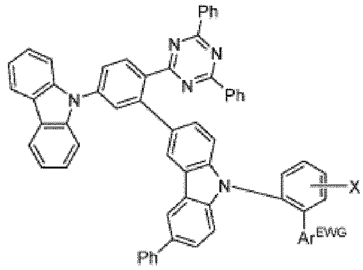

6. Method for producing an organic molecule according to claim 1 to 5, comprising the step of providing a 6-$R^B$-substituted-3-bromocarbazole as a reactant.

7. Use of a molecule according to one or more of claims 1 to 5 as a luminescence emitter and/or transport material and/or hole injection material and/or hole blocking material in an optoelectronic device.

8. Use according to claim 7, wherein the optoelectronic device is selected from the group consisting of:

- organic light emitting diodes (OLEDs),
- light emitting electrochemical cells,
- OLED sensors,
- organic diodes,
- organic solar cells,
- organic transistors,
- organic field effect transistors,
- organic lasers and
- down-conversion elements.

9. Composition, comprising:

(a) an organic molecule according to one or more of claims 1 to 5, in particular in the form of an emitter, and
(b) an emitter and/or host material which differs from the organic molecule according to one or more of claims 1 to 5, and
(c) optionally a dye and/or a solvent.

10. Optoelectronic device, comprising an organic molecule according to one or more of claims 1 to 5 or a composition according to claim 9, in particular in the form of a device selected from the group consisting of organic light emitting diode (OLED), light emitting electrochemical cell, OLED sensor, organic diode, organic solar cell, organic transistor, organic field effect transistor, organic laser and down-conversion element.

11. Optoelectronic device according to claim 10, comprising

- a substrate,
- an anode and

- a cathode, wherein the anode or the cathode are arranged on the substrate, and
- a light-emitting layer arranged between the anode and the cathode and comprising an organic molecule according to one or more of claims 1 to 5 or a composition according to claim 9.

**12.** Method for producing an optoelectronic device, wherein an organic molecule according to one or more of claims 1 to 5 or a composition according to claim 9 is used.

**13.** Method according to claim 12, comprising processing the organic molecule or the organic composition by a vacuum evaporation process or from a solution.

## Revendications

**1.** Molécule organique sélectionnée dans le groupe comprenant les formules IIa-1, IIa-2 et IIa-3 :

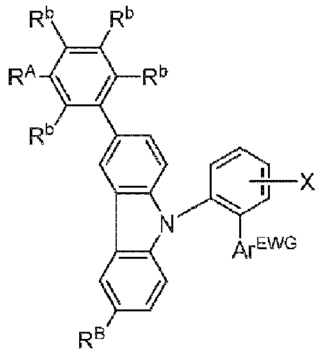
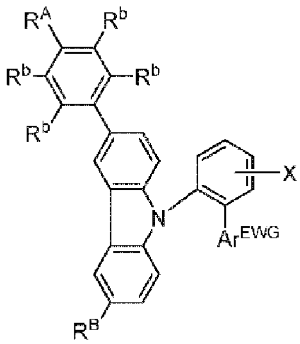
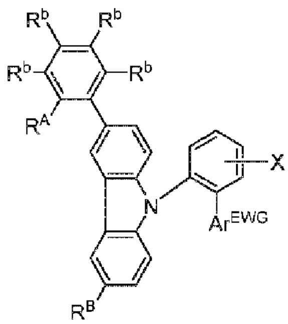

Formule IIa-1 Formule IIa-2 Formule IIa-3

dans laquelle

X est choisi dans le groupe comprenant H, $SiMe_3$, $SiPh_3$, CN et $CF_3$ ;
$Ar^{EWG}$ est choisi dans le groupe comprenant les formules IIa à IIc et IIi

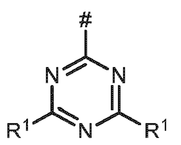
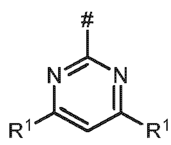
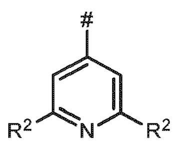

Formule IIa Formule IIb Formule IIc

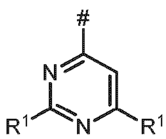

Formule IIi

où # représente le point de liaison d'une liaison simple reliant $Ar^{EWG}$ au cycle phényle ;
$R^1$ est choisi, indépendamment pour chaque occurrence, dans le groupe comprenant :

hydrogène,
deutérium,
alkyle $C_1$-$C_5$,
un ou plusieurs atomes d'hydrogène étant facultativement substitués avec du deutérium ;
alkényle $C_2$-$C_8$,

un ou plusieurs atomes d'hydrogène étant facultativement substitués avec du deutérium ;
alkinyle $C_2$-$C_8$,
un ou plusieurs atomes d'hydrogène étant facultativement substitués avec du deutérium ;
et aryle $C_6$-$C_{18}$,
avec substitution facultative par un ou plusieurs substituants $R^6$ ;
$R^2$ est choisi, indépendamment pour chaque occurrence, dans le groupe comprenant :
hydrogène,
deutérium,
alkyle $C_1$-$C_5$,
un ou plusieurs atomes d'hydrogène étant facultativement substitués avec du deutérium ;
alkényle $C_2$-$C_8$,
un ou plusieurs atomes d'hydrogène étant facultativement substitués avec du deutérium ;
alkinyle $C_2$-$C_8$,
un ou plusieurs atomes d'hydrogène étant facultativement substitués avec du deutérium ;
et aryle $C_6$-$C_{18}$,
avec substitution facultative par un ou plusieurs substituants $R^6$ ;
$R^d$ est choisi, indépendamment pour chaque occurrence, dans le groupe comprenant :

hydrogène, deutérium, $N(R^6)_2$, $OR^6$, $Si(R^6)_3$, $B(OR^6)_2$, $OSO_2R^6$, $CF_3$, CN, F, Br, I, alkyle $C_1$-$C_{40}$,
avec substitution facultative par un ou plusieurs substituants $R^6$ et
où un ou plusieurs groupes $CH_2$ non voisins sont substitués facultativement par $R^6C=CR^6$, C•C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alkoxy $C_1$-$C_{40}$,
avec substitution facultative par un ou plusieurs substituants $R^6$ et
où un ou plusieurs groupes $CH_2$ non voisins sont substitués facultativement par $R^6C=CR^6$, C.C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
thioalkoxy $C_1$-$C_{40}$,
avec substitution facultative par un ou plusieurs substituants $R^6$ et
où un ou plusieurs groupes $CH_2$ non voisins sont substitués facultativement par $R^6C=CR^6$, C.C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alkényle $C_2$-$C_{40}$,
avec substitution facultative par un ou plusieurs substituants $R^6$ et
où un ou plusieurs groupes $CH_2$ non voisins sont substitués facultativement par $R^6C=CR^6$, C·C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
alkinyle $C_2$-$C_{40}$,
avec substitution facultative par un ou plusieurs substituants $R^6$ et
où un ou plusieurs groupes $CH_2$ non voisins sont substitués facultativement par $R^6C=CR^6$, C·C, $Si(R^6)_2$, $Ge(R^6)_2$, $Sn(R^6)_2$, C=O, C=S, C=Se, $C=NR^6$, $P(=O)(R^6)$, SO, $SO_2$, $NR^6$, O, S ou $CONR^6$ ;
aryle $C_6$-$C_{60}$,
avec substitution facultative par un ou plusieurs substituants $R^6$ ;
et hétéroaryle $C_3$-$C_{57}$,
avec substitution facultative par un ou plusieurs substituants $R^6$ ;
$R^6$ est choisi, indépendamment pour chaque occurrence, dans le groupe comprenant :
hydrogène, deutérium, OPh, $CF_3$, CN, F,
alkyle $C_1$-$C_5$,
dans lequel, facultativement, un ou plusieurs atomes d'hydrogène sont substitués, indépendamment les uns des autres, par : deutérium, CN, $CF_3$ ou F ; alkoxy $C_1$-$C_5$,
dans lequel, facultativement, un ou plusieurs atomes d'hydrogène sont substitués, indépendamment les uns des autres, par : deutérium, CN, $CF_3$ ou F ; thioalkoxy $C_1$-$C_5$,
dans lequel, facultativement, un ou plusieurs atomes d'hydrogène sont substitués, indépendamment les uns des autres, par : deutérium, CN, $CF_3$ ou F ; alkényle $C_2$-$C_5$,
dans lequel, facultativement, un ou plusieurs atomes d'hydrogène sont substitués, indépendamment les uns des autres, par : deutérium, CN, $CF_3$ ou F ; alkinyle $C_2$-$C_5$,
dans lequel, facultativement, un ou plusieurs atomes d'hydrogène sont substitués, indépendamment les uns des autres, par : deutérium, CN, $CF_3$ ou F ; aryle $C_6$-$C_{18}$,
substitué facultativement avec un ou plusieurs substituants alkyle $C_1$-$C_5$ ; hétéroaryle $C_3$-$C_{17}$,
substitué facultativement avec un ou plusieurs substituants alkyle $C_1$-$C_5$ ; N(aryle $C_6$-$C_{18})_2$ ;
N(hétéroaryle $C_3$-$C_{17})_2$,

et N(hétéroaryle $C_3$-$C_{17}$-)(aryle $C_6$-$C_{18}$) ;
$R^A$ est choisi, indépendamment pour chaque occurrence, parmi une structure chimique de formule IIA

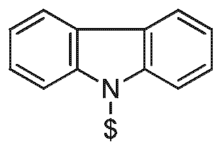

Formule IIA

ou une structure chimique de formule IIB

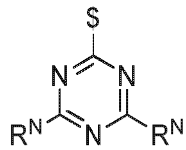

Formule IIB,

où $R^N$ est choisi dans le groupe comprenant Me, $^i$Pr, $^t$Bu, CN, $CF_3$ et Ph ;
où $ représente le point de liaison d'une liaison simple ;
où $R^B$ est choisi dans le groupe comprenant
H,
Ph,
CN,
$CF_3$,
d'une structure chimique de formule IIA-2

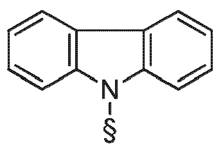

Formule IIA-2

ou d'une structure chimique de formule IIB-2

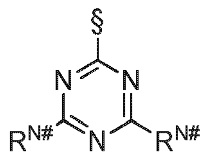

Formule IIB-2 ;

où $R^{N\#}$ est choisi dans le groupe comprenant Me, $^i$Pr, $^t$Bu, CN, $CF_3$ et Ph ;
où S représente le point de liaison d'une liaison simple et
où $R^b$ est choisi, indépendamment pour chaque occurrence, dans le groupe comprenant :

H,
Me,
$^i$Pr,
$^t$Bu,
CN,
$CF_3$,

Ph, avec substitution facultative par un ou plusieurs substituants choisis indépendamment dans le groupe comprenant Me, $^{i}Pr$, $^{t}Bu$, CN, $CF_3$ et Ph, pyridinyle, avec substitution facultative par un ou plusieurs substituants choisis indépendamment dans le groupe comprenant Me, $^{i}Pr$, $^{t}Bu$, CN, $CF_3$ et Ph.

pyrimidinyle, avec substitution facultative par un ou plusieurs substituants choisis indépendamment dans le groupe comprenant Me, $^{i}Pr$, $^{t}Bu$, CN, $CF_3$ et Ph,

carbazolyle, avec substitution facultative par un ou plusieurs substituants choisis indépendamment dans le groupe comprenant Me, $^{i}Pr$, $^{t}Bu$, CN, $CF_3$ et Ph, triazinyle, avec substitution facultative par un ou plusieurs substituants choisis indépendamment dans le groupe comprenant Me, $^{i}Pr$, $^{t}Bu$, CN, $CF_3$ et Ph, et $N(Ph)_2$.

2. Molécule organique selon la revendication 1, dans laquelle $R^1$ est, indépendamment pour chaque occurrence, un phényle, avec substitution facultative par un ou plusieurs substituants $R^6$, et $R^2$ est choisi, indépendamment pour chaque occurrence, dans le groupe comprenant :

   hydrogène,
   CN,
   $CF_3$ et
   phényle, avec substitution facultative par un ou plusieurs substituants $R^6$.

3. Molécule organique selon la revendication 1 ou 2, présentant une structure choisie dans le groupe représenté ci-après :

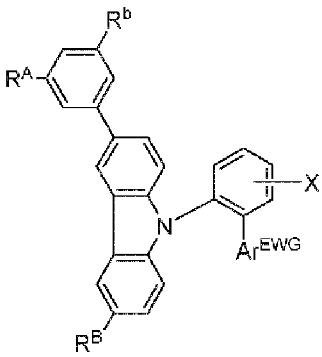
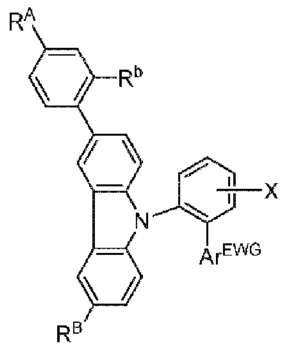
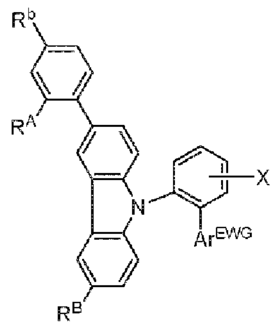

Formule IIc-1 Formule IIc-2 Formule IIc-3

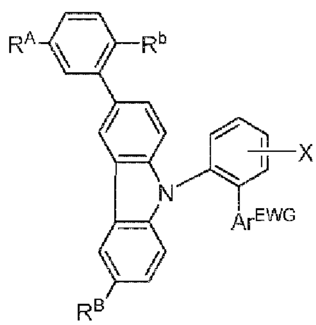
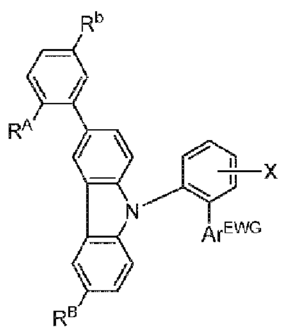

Formule IIc-4 Formule IIc-5.

4. Molécule organique selon une ou plusieurs des revendications 1 à 3, dans laquelle $R^b$ est choisi, indépendamment pour chaque occurrence, dans le groupe comprenant H et CN.

5. Molécule organique selon une ou plusieurs des revendications 1 à 4, comportant ou se composant d'une structure choisie dans le groupe représenté ci-après :

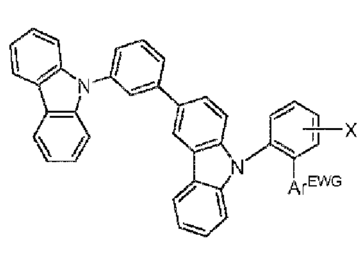
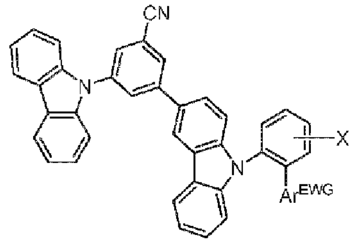
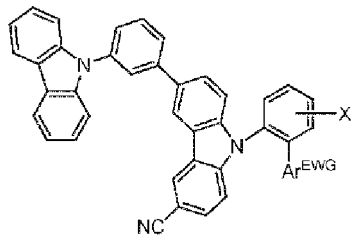

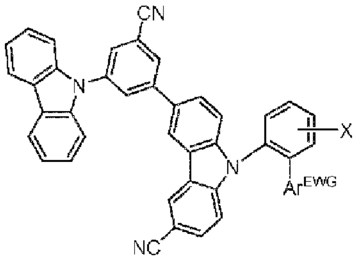
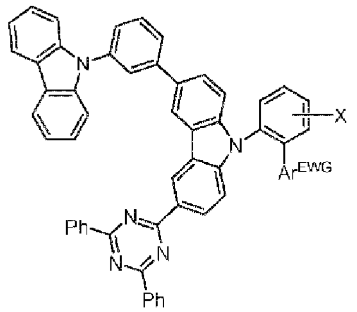
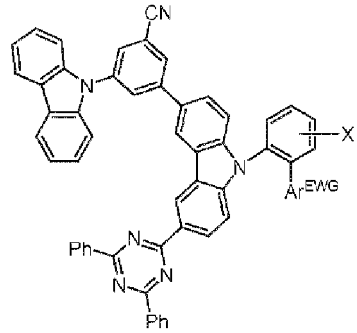

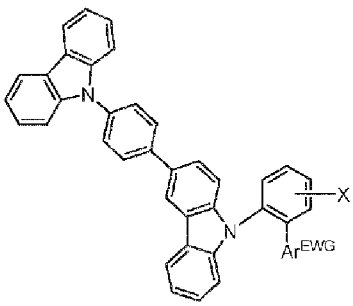
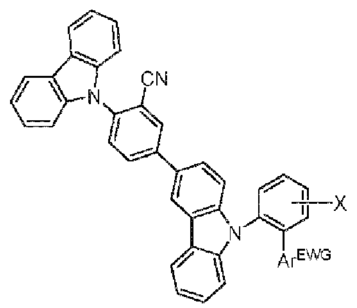
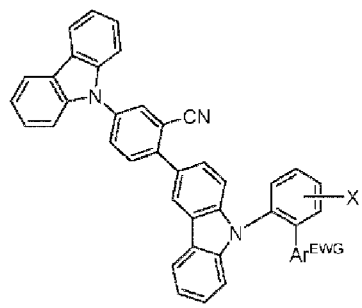

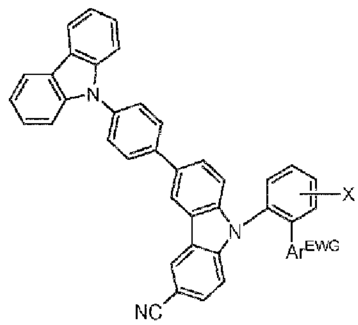
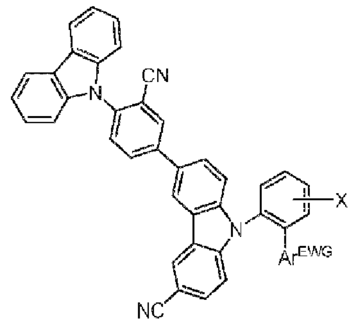
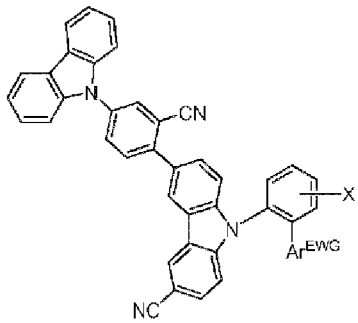

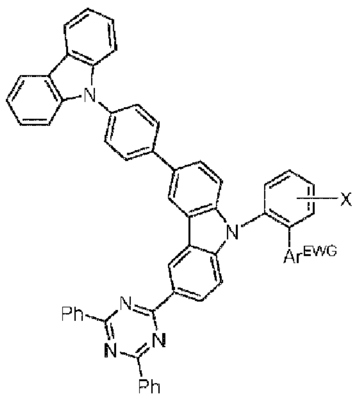
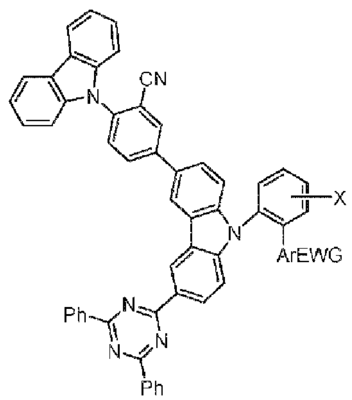
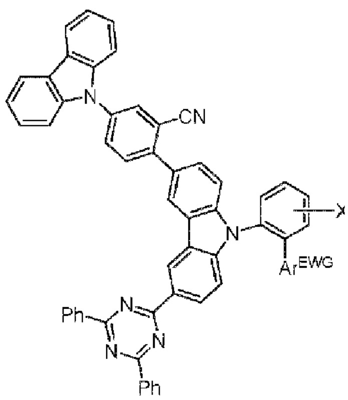

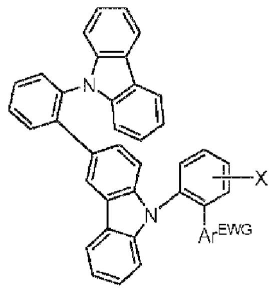
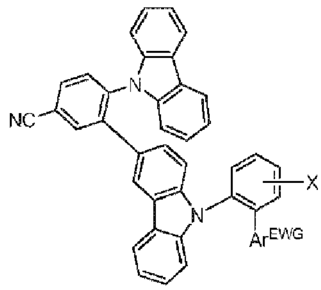
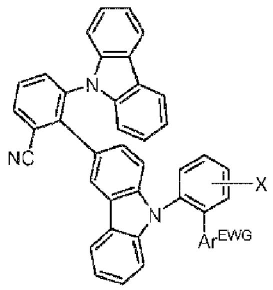

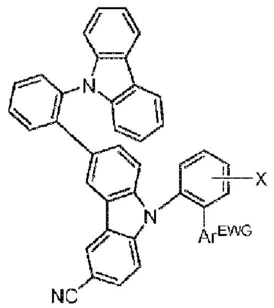
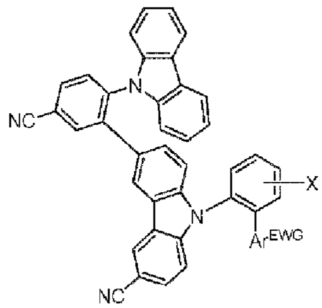
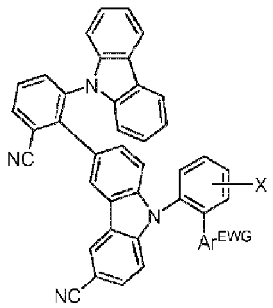

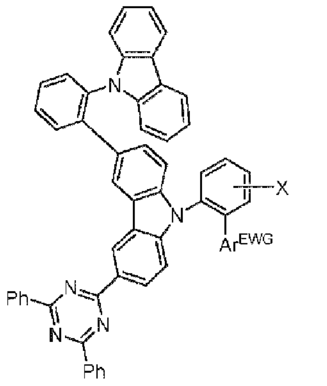
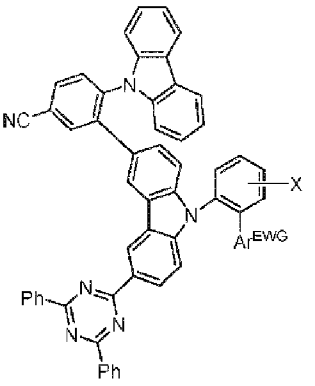
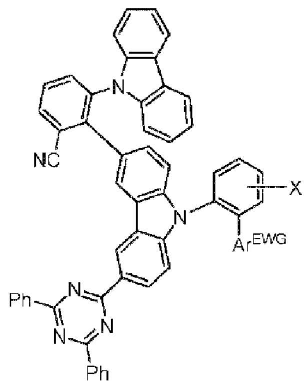

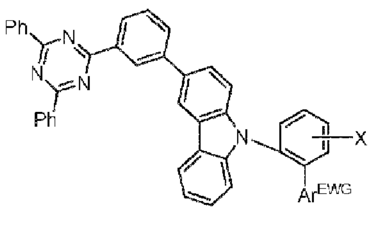
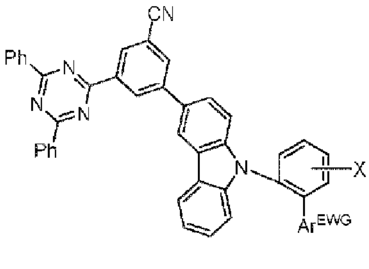
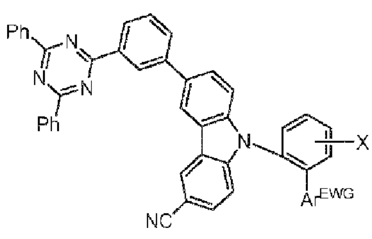

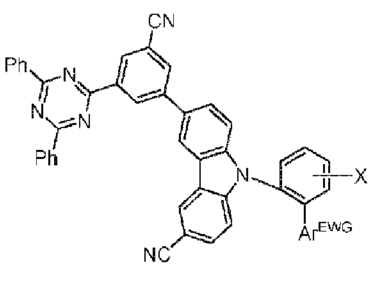
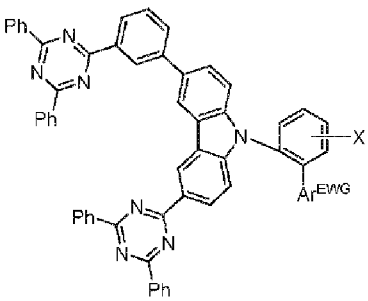
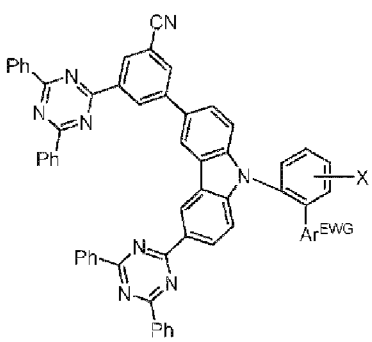

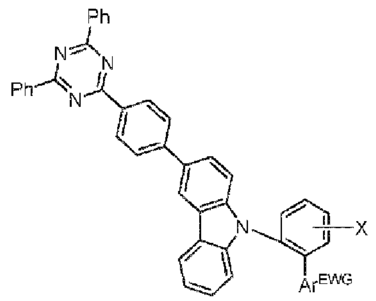

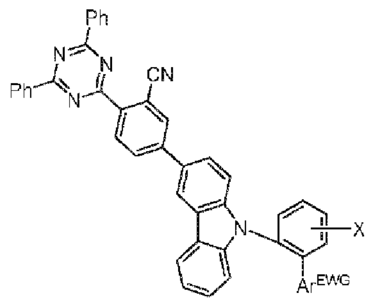

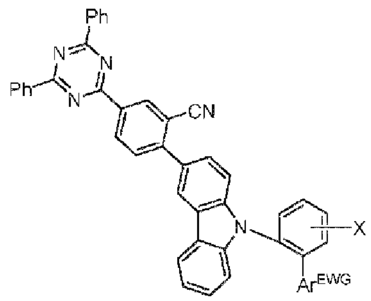

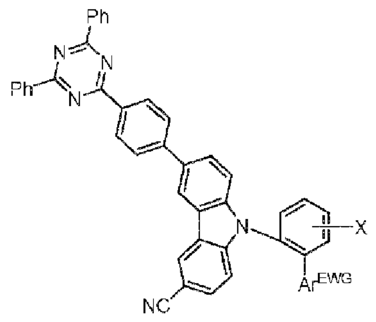

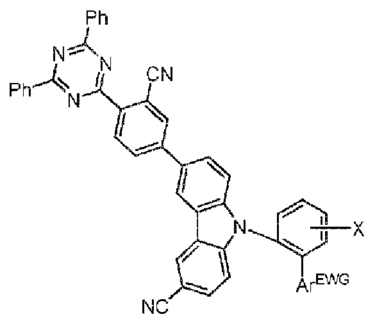

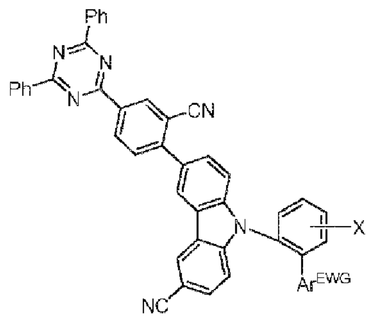

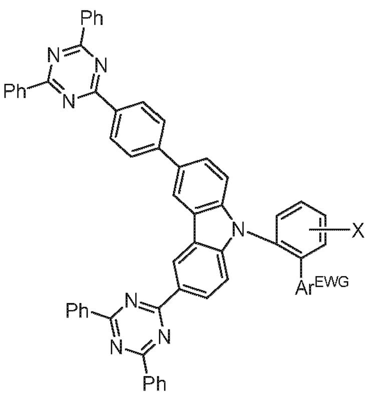

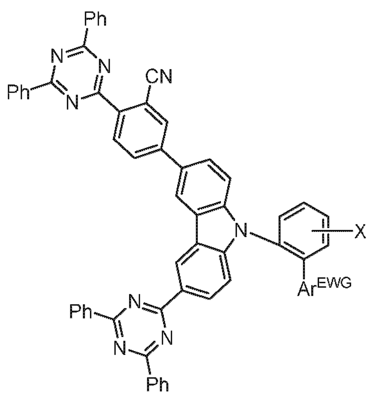

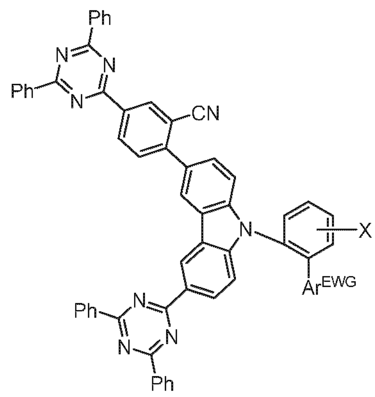

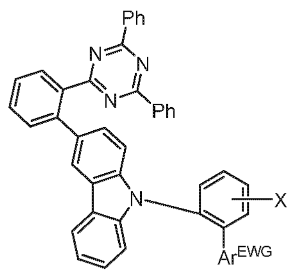

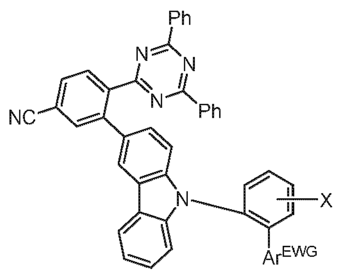

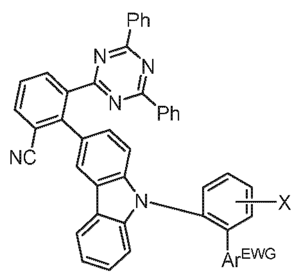

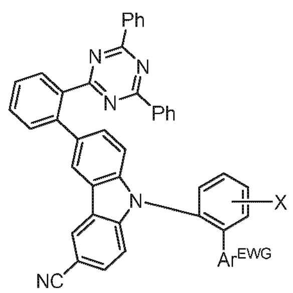

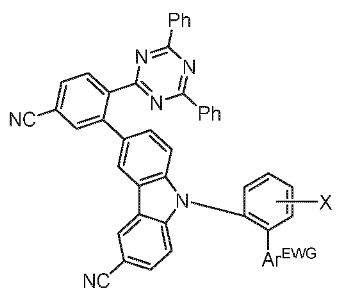

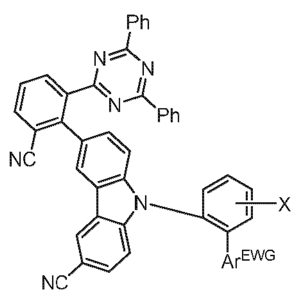

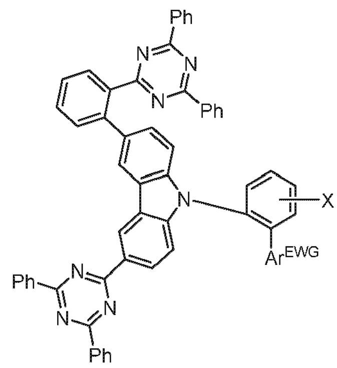
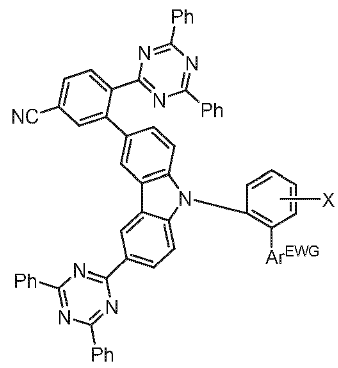
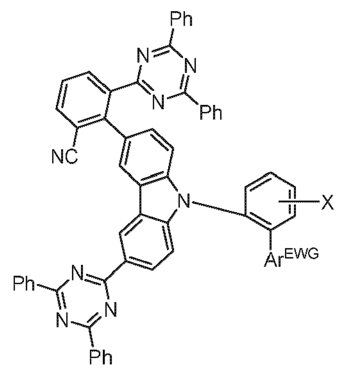

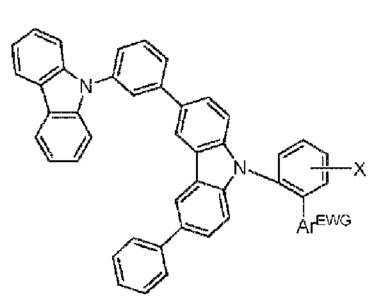
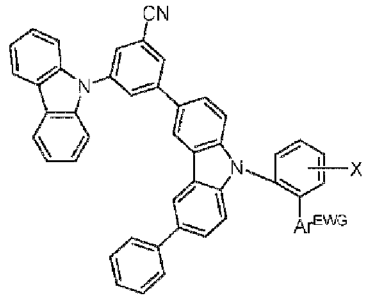
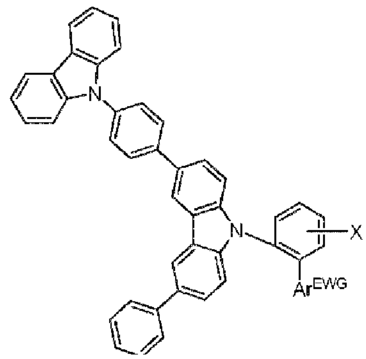

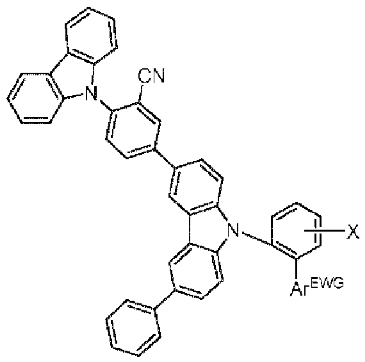
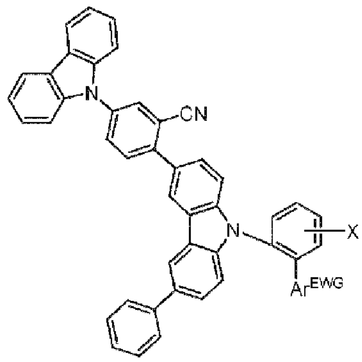
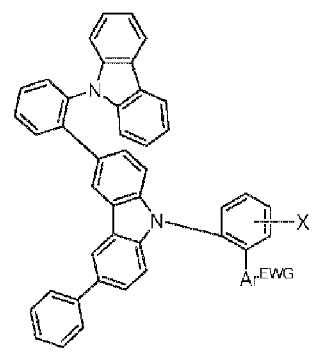

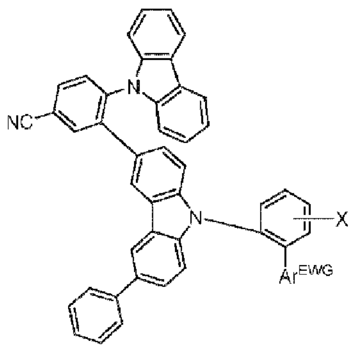
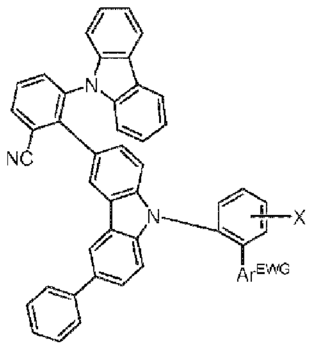
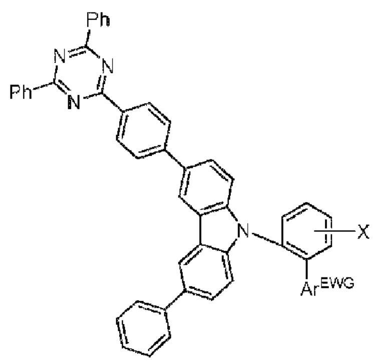

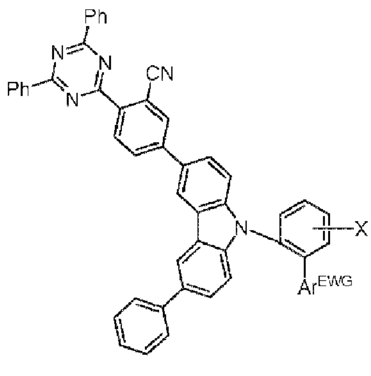

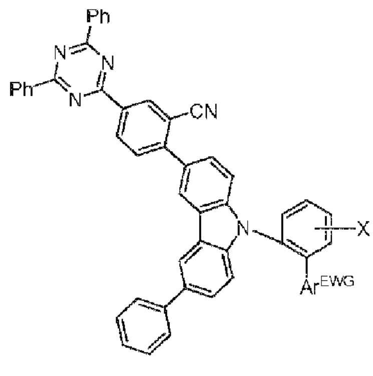

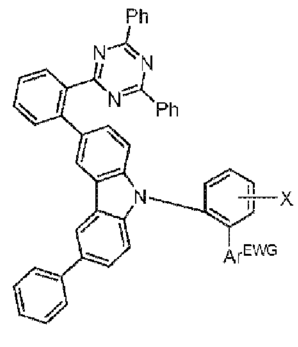

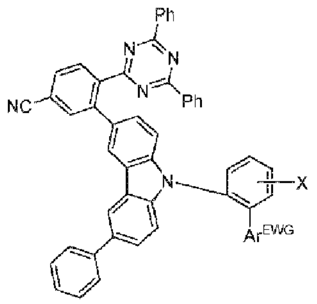

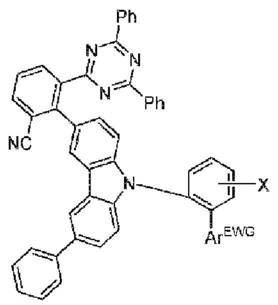

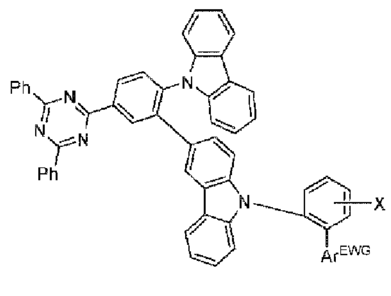

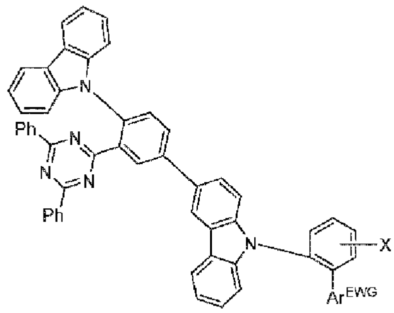

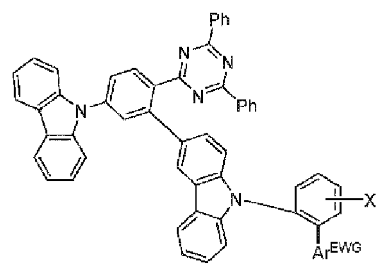

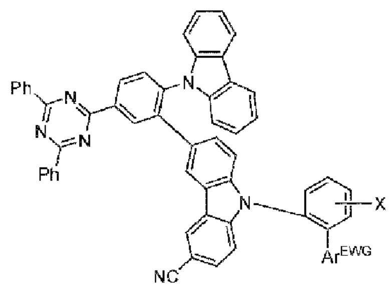

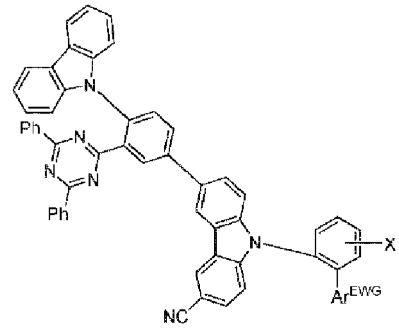

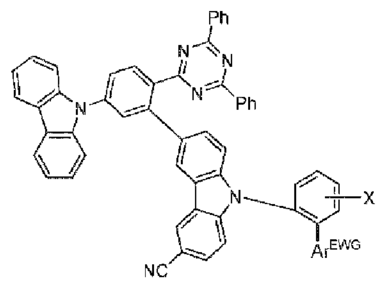

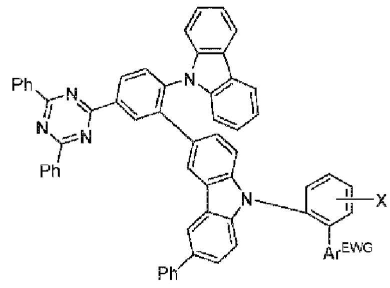

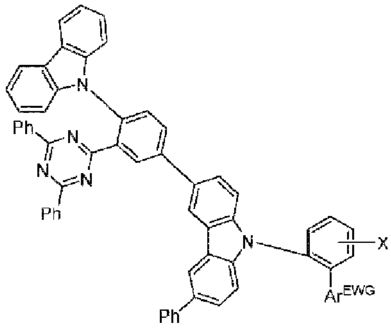 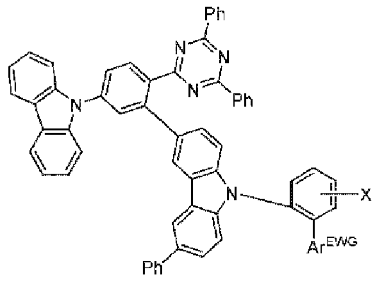

6. Procédé pour la fabrication d'une molécule organique selon les revendications 1 à 5, comprenant l'étape de préparation d'un 3-bromocarbazole substitué par 6-$R^B$ servant de réactif.

7. Utilisation d'une molécule organique selon les revendications 1 à 5 comme émetteur de luminescence et/ou matériau de transport et/ou matériau d'injection de trous et/ou matériau d'obturation de trous dans un dispositif optoélectronique.

8. Utilisation selon la revendication 7, dans laquelle le dispositif optoélectronique est choisi dans le groupe comprenant :

   • diodes électroluminescentes organiques (OLED),
   • cellules photoélectrochimiques,
   • capteurs à OLED,
   • diodes organiques,
   • cellules solaires organiques,
   • transistors organiques,
   • transistors à effet de champ organiques,
   • lasers organiques et
   • éléments convertisseurs-abaisseurs.

9. Composition comprenant :

   (a) une molécule organique selon une ou plusieurs des revendications 1 à 5, en particulier sous la forme d'un émetteur, et
   (b) un matériau émetteur et/ou hôte différent de la molécule organique selon une ou plusieurs des revendications 1 à 5, et
   (c) facultativement, un colorant et/ou un solvant.

10. Dispositif optoélectronique comprenant une molécule organique selon une ou plusieurs des revendications 1 à 5 ou une composition selon la revendication 9, en particulier sous la forme d'un dispositif choisi parmi le groupe comprenant un diode électroluminescente organique (OLED), une cellule photoélectrochimique, un capteur à OLED, une diode organique, une cellule solaire organique, un transistor organique, un transistor à effet de champ organique, un laser organique et un élément convertisseurs-abaisseur.

11. Dispositif optoélectronique selon la revendication 10, comportant

   - un substrat,
   - une anode et
   - une cathode, l'anode ou la cathode étant disposée sur le substrat, et
   - une couche luminescente qui est disposée entre l'anode et la cathode et qui contient une molécule organique selon une ou plusieurs des revendications 1 à 5 ou une composition selon la revendication 9.

12. Procédé de fabrication d'un dispositif optoélectronique, dans lequel une molécule organique selon une ou plusieurs des revendications 1 à 5 ou une composition selon la revendication 9 est utilisée.

13. Procédé selon la revendication 12, comprenant le traitement de la molécule organique ou de la composition organique par un procédé d'évaporation sous vide ou à partir d'une solution.

Figur 1

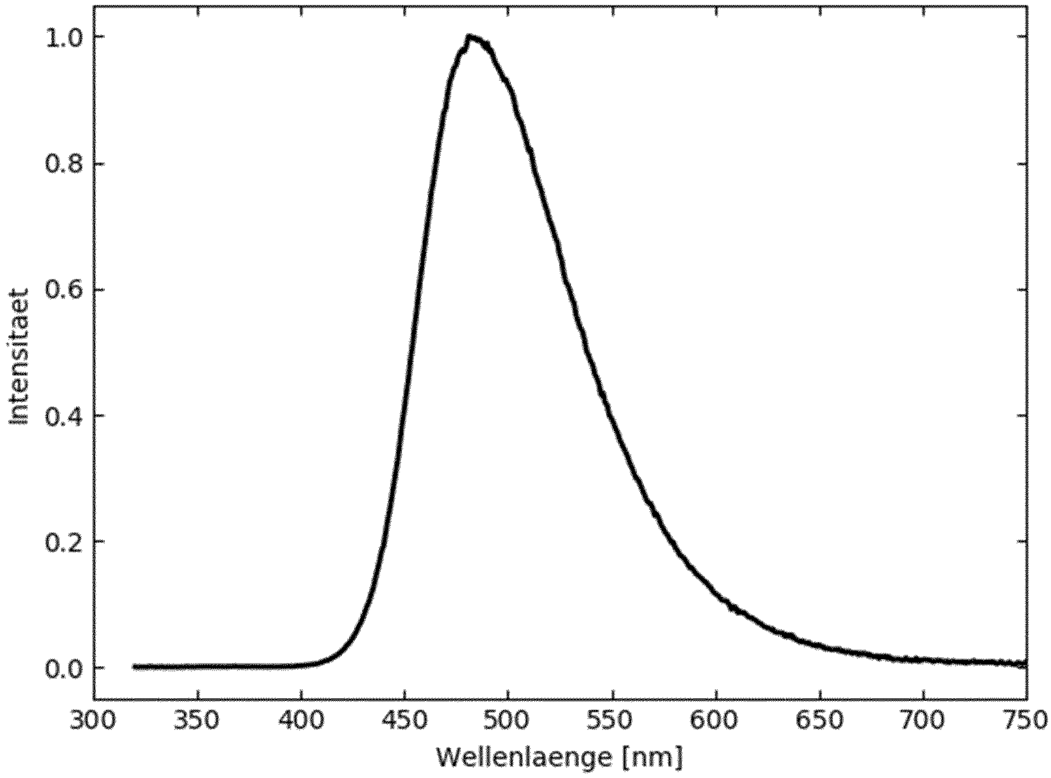


Figur 2

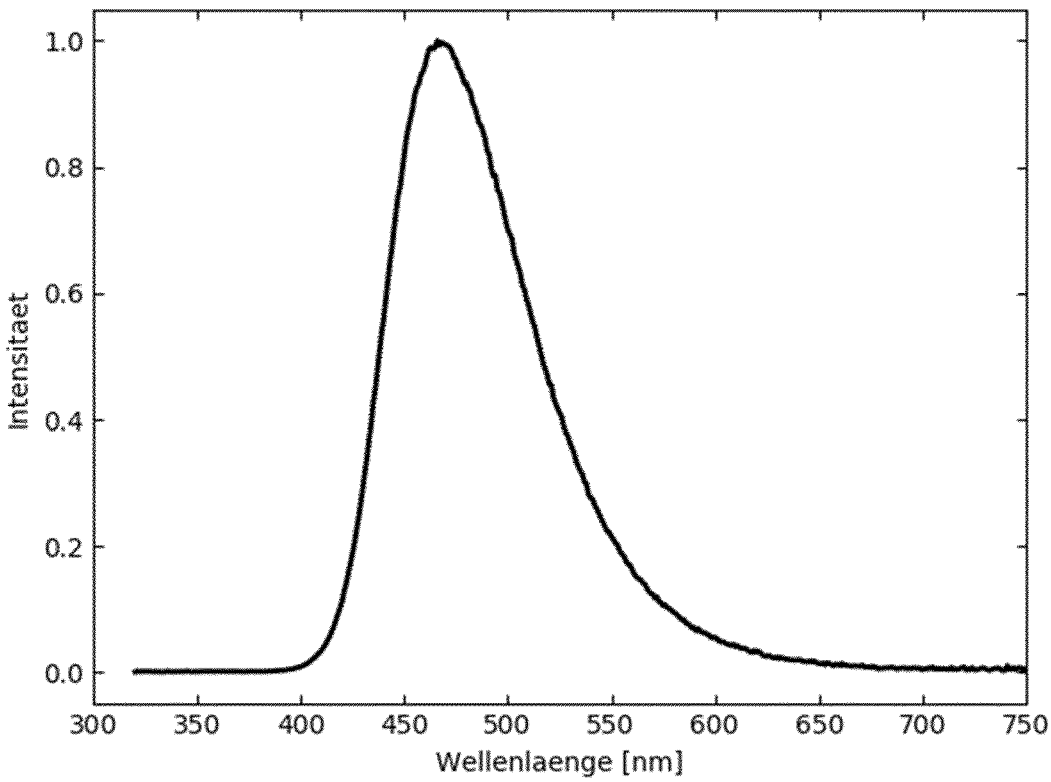

Figur 3

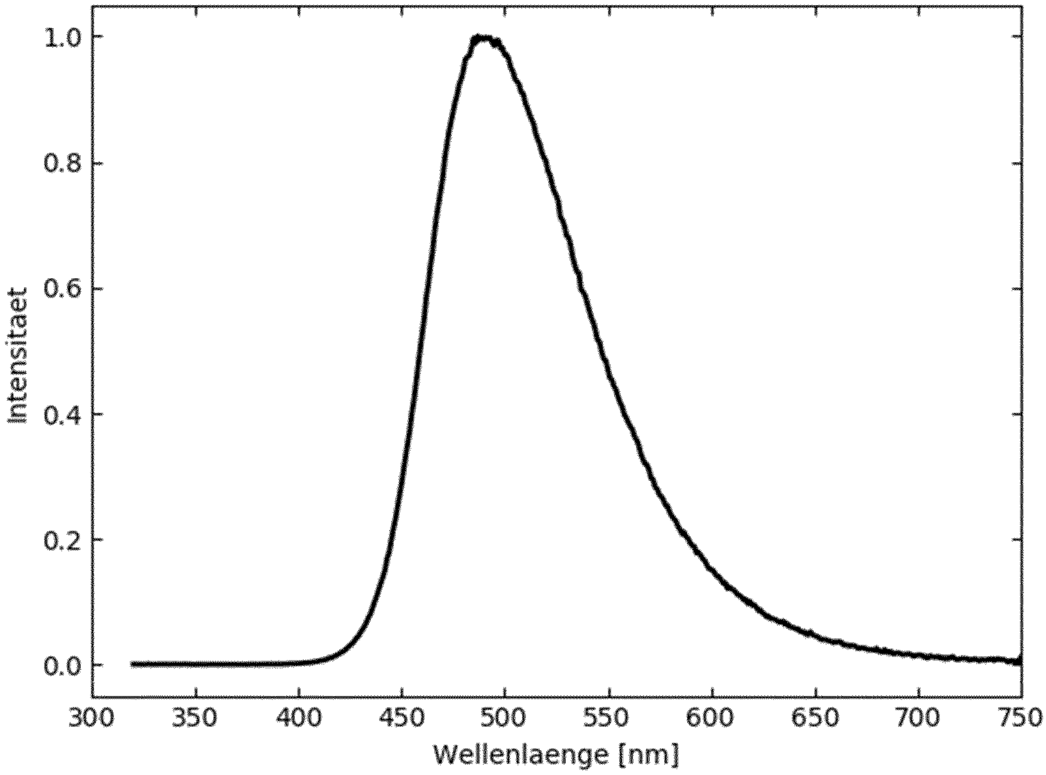


Figur 4

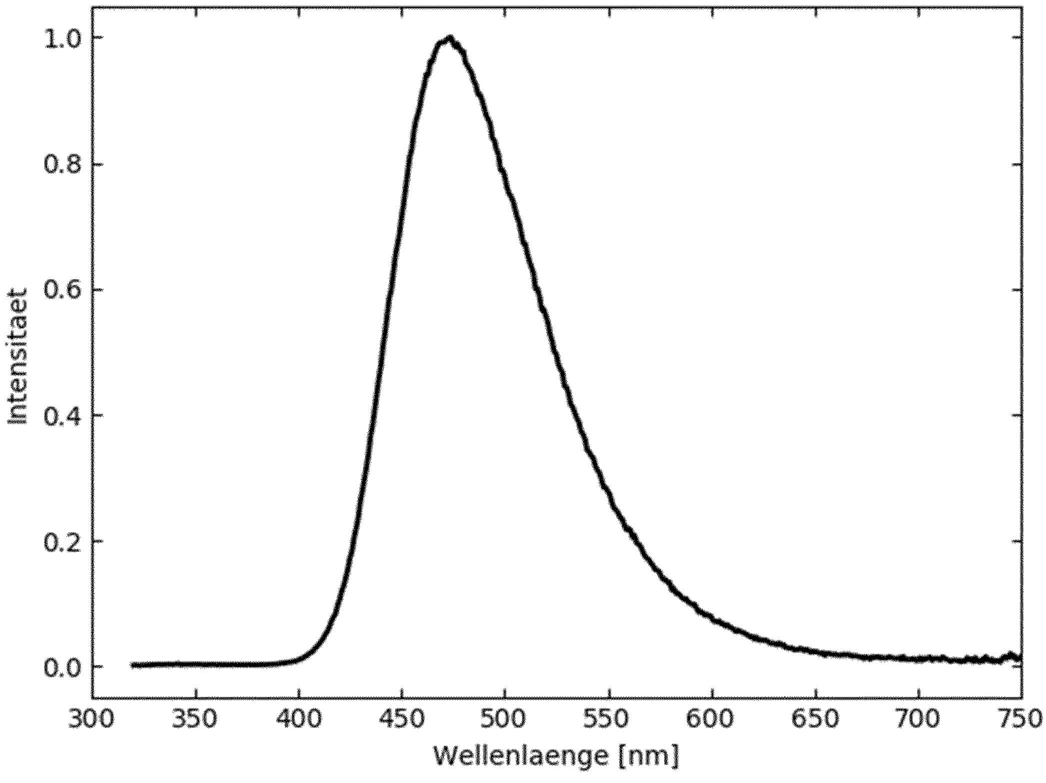

Figur 5

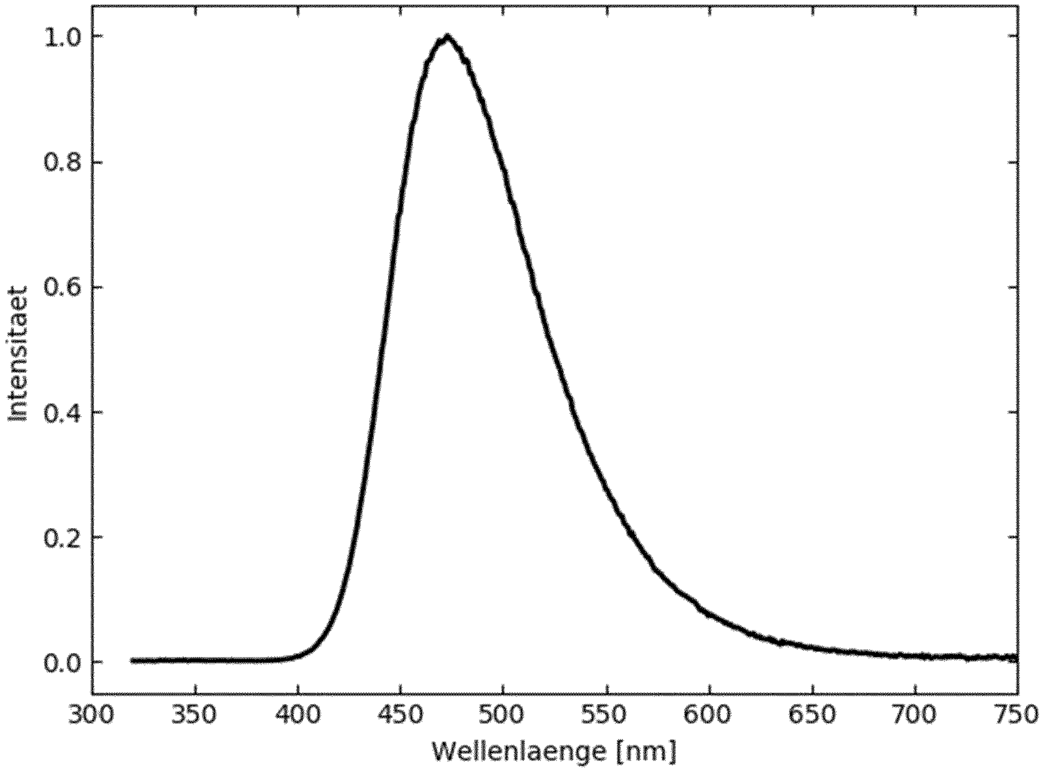


Figur 6

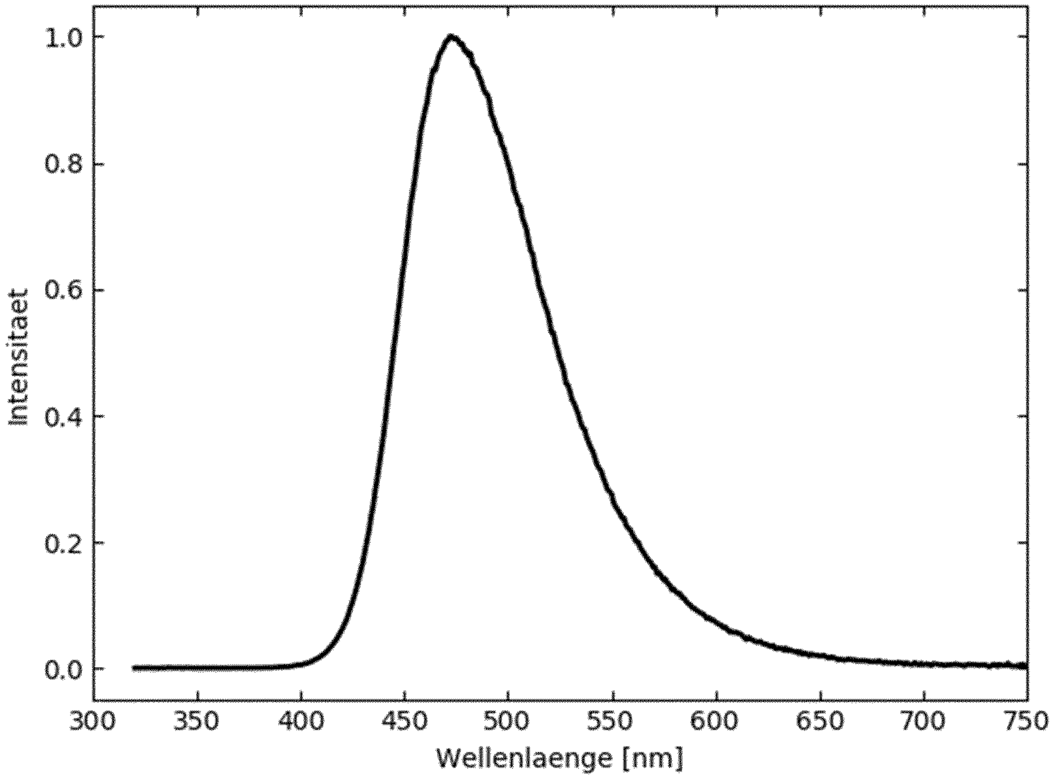

Figur 7

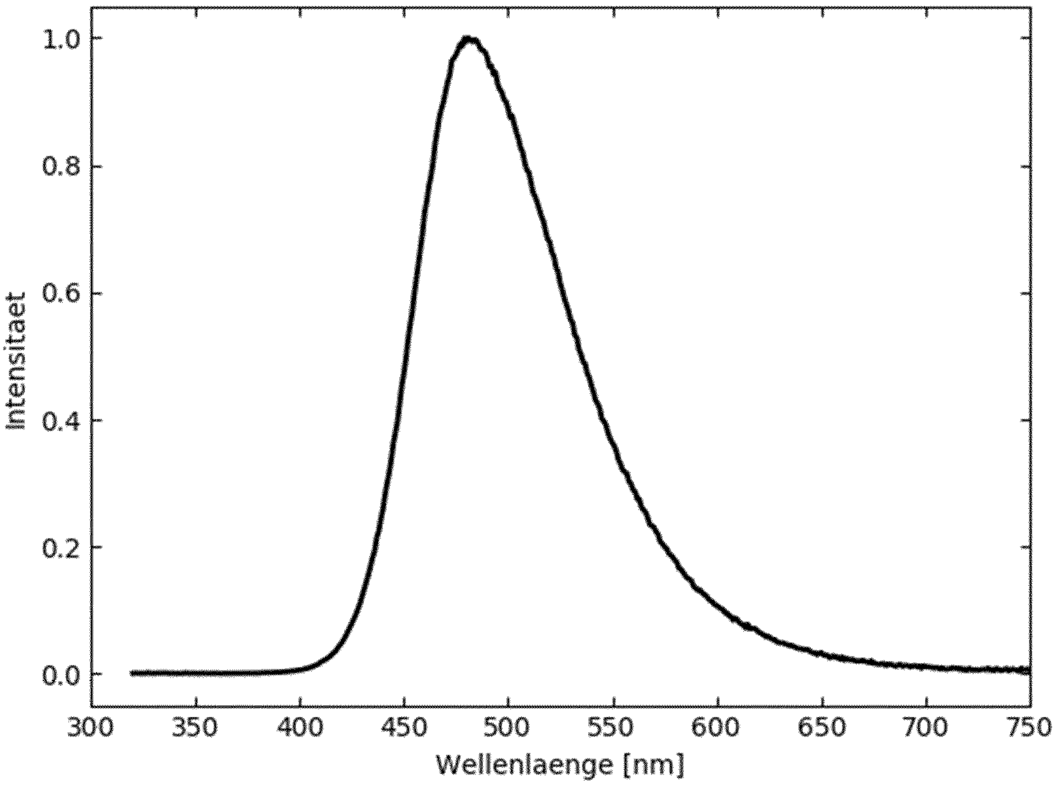
1.0
0.8
0.6
0.4
0.2
0.0
Intensitaet
300
350
400
450
500
550
600
650
700
750
Wellenlaenge [nm]

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2013100540 A1
- EP 3109247 A1
- WO 2016013875 A1
- WO 2015142036 A1
- WO 2013165192 A1

### In der Beschreibung aufgeführte Nicht-Patentliteratur

- **OKINAKA et al.** Invited Paper: New Fluorescent Blue Host Materials for Achieving Low Voltage in OLEDs. *SID Symposium Digest of Technical Papers,* 2015, vol. 22 (1), 46
- *CHEMICAL ABSTRACTS,* 73183-34-3
- *CHEMICAL ABSTRACTS,* 1592-95-6
- *CHEMICAL ABSTRACTS,* 3842-55-5
- *CHEMICAL ABSTRACTS,* 100-47-0
- *CHEMICAL ABSTRACTS,* 128-08-5